(19) Europäisches Patentamt
European Patent Office
Office européen des brevets

(11) EP 1 894 924 A1

(12) EUROPEAN PATENT APPLICATION

(43) Date of publication:
05.03.2008 Bulletin 2008/10

(21) Application number: 06018023.9

(22) Date of filing: 29.08.2006

(51) Int Cl.:
*C07D 401/06* (2006.01) *C07D 401/12* (2006.01)
*C07D 413/12* (2006.01) *C07D 413/14* (2006.01)
*A61K 31/4427* (2006.01) *A61K 31/501* (2006.01)
*A61K 31/506* (2006.01) *A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Phenex Pharmaceuticals AG**
**67056 Ludwigshafen (DE)**

(72) Inventors:
- **Kremoser, Claus**
**69121 Heidelberg (DE)**
- **Deuschle, Ulrich**
**67549 Worms (DE)**
- **Abel, Ulrich**
**69126 Heidelberg (DE)**
- **Schulz, Andreas**
**69118 Heidelberg (DE)**

(74) Representative: **Grünecker, Kinkeldey, Stockmair & Schwanhäusser Anwaltssozietät**
**Maximilianstrasse 58**
**80538 München (DE)**

(54) **Heterocyclic FXR binding compounds**

(57) The present invention relates to compounds which bind to the NR1H4 receptor (FXR) and act as agonists or partial agonists of the NR1H4 receptor (FXR). The invention further relates to the use of the compounds for the preparation of a medicament for the treatment of diseases and/or conditions through binding of said nuclear receptor by said compounds, and to a process for the synthesis of said compounds.

EP 1 894 924 A1

## Description

The present invention relates to compounds which bind to the NR1H4 receptor (FXR) and act as agonists or partial agonists of the NR1H4 receptor (FXR). The invention further relates to the use of the compounds for the preparation of a medicament for the treatment of diseases and/or conditions through binding of said nuclear receptor by said compounds, and to a process for the synthesis of said compounds.

Multicellular organisms are dependent on advanced mechanisms of information transfer between cells and body compartments. The information that is transmitted can be highly complex and can result in the alteration of genetic programs involved in cellular differentiation, proliferation, or reproduction. The signals, or hormones, are often low molecular weight molecules, such as peptides, fatty acid, or cholesterol derivatives.

Many of these signals produce their effects by ultimately changing the transcription of specific genes. One well-studied group of proteins that mediate a cell's response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known. Orphan receptors may be indicative of unknown signalling pathways in the cell or may be nuclear receptors that function without ligand activation. The activation of transcription by some of these orphan receptors may occur in the absence of an exogenous ligand and/or through signal transduction pathways originating from the cell surface (D. Mangelsdorf et al. "The nuclear receptor superfamily: the second decade", Cell 1995, 83(6), 835-839; R Evans "The nuclear receptor superfamily: a rosetta stone for physiology" Mol. Endocrinol. 2005, 19(6), 1429-1438).

In general, three functional domains have been defined in NRs. An amino terminal domain is believed to have some regulatory function. A DNA-binding domain hereinafter referred to as "DBD" usually comprises two zinc finger elements and recognizes a specific Hormone Responsive Element hereinafter referred to as "HRE" within the promoters of responsive genes. Specific amino acid residues in the "DBD" have been shown to confer DNA sequence binding specificity (M. Schena "Mammalian glucocorticoid receptor derivatives enhance transcription in yeast", Science 1988, 241(4868), 965-967). A ligand-binding-domain hereinafter referred to as "LBD" is at the carboxy-terminal region of known NRs.

In the absence of hormone, the LBD appears to interfere with the interaction of the DBD with its HRE. Hormone binding seems to result in a conformational change in the NR and thus opens this interference (A. Brzozowski et al. "Molecular basis of agonism and antagonism in the oestrogen receptor" Nature 1997, 389(6652), 753-758). A NR without the LBD constitutively activates transcription but at a low level.

Coactivators or transcriptional activators are proposed to bridge between sequence specific transcription factors, the basal transcription machinery and in addition to influence the chromatin structure of a target cell. Several proteins like SRC-1, ACTR, and Grip1 interact with NRs in a ligand enhanced manner (D. Heery et al. "A signature motif in transcriptional co-activators mediates binding to nuclear receptors" Nature 1997, 387(6634), 733-6.; T. Heinzel et al. "A complex containing N-CoR, mSin3 and histone deacetylase mediates transcriptional repression" Nature 1997, 387(6628), 16-17; K. Nettles, G. Greene "Ligand control of coregulator recruitment to nuclear receptors" Annu. Rev. Physiol. 2005, 67, 309-33).

Nuclear receptor modulators like steroid hormones affect the growth and function of specific cells by binding to intracellular receptors and forming nuclear receptor-ligand complexes. Nuclear receptor-hormone complexes then interact with a hormone response element (HRE) in the control region of specific genes and alter specific gene expression (A. Aranda, A. Pascual "Nuclear hormone receptors and gene expression" Physiol. Rev. 2001, 81(3), 1269-1304).

The Farnesoid X Receptor alpha (hereinafter also often referred to as NR1H4 when referring to the human receptor) is a prototypical type 2 nuclear receptor which activates genes upon binding to promoter region of target genes in a heterodimeric fashion with Retinoid X Receptor (B. Forman et al. "Identification of a nuclear receptor that is activated by farnesol metabolites" Cell 1995, 81(5), 687-693). The relevant physiological ligands of NR1H4 are bile acids (D. Parks et al. "Bile acids: natural ligands for an orphan nuclear receptor" Science 1999, 284(5418), 1365-1368; M. Makishima et al. "Identification of a nuclear receptor for bile acids" Science 1999, 284(5418), 1362-1365). The most potent one is chenodeoxycholic acid (CDCA), which regulates the expression of several genes that participate in bile acid homeostasis. Farnesol and derivatives, together called farnesoids, are originally described to activate the rat orthologue at high concentration but they do not activate the human or mouse receptor. FXR is expressed in the liver, small intestine, colon, ovary, adrenal gland and kidney. Beyond controlling intracellular gene expression, FXR seems to be also involved in paracrine and endocrine signaling (J. Holt et al. "Definition of a novel growth factor-dependent signal cascade for the suppression of bile acid biosynthesis" Genes Dev. 2003, 17(13), 1581-91; T. Inagaki et al. "Fibroblast growth factor 15 functions as an enterohepatic signal to regulate bile acid homeostasis" Cell Metab. 2005, 2(4), 217-225).

There is one publication which proposes a direct impact of FXR activation on the survival of infectious organisms such as bacteria or protozoic parasites via the upregulation of the lysosomal fate/survival factor Taco-2 in macrophages (P. Anandet al. "Downregulation of TACO gene transcription restricts mycobacterial entry/survival within human macrophages" FEMS Microbiol. Lett. 2005, 250(1), 137-144). This might pave the way for further studies that assess the suitability of FXR to act as drug target for the treatment of intracellular bacterial or parasitic infections such as Tuberculosis, Lepra, Leishmaniosis or Trypanosomiasis, e.g. Chagas Disease.

Small molecule compounds which act as FXR modulators have been disclosed in the following publications: WO 2004/048349, WO 2003/015771 and WO 2000/037077. Further small molecule FXR modulators have been recently reviewed (R. C. Buijsman et al. "Non-Steroidal Steroid Receptor Modulators" Curr. Med. Chem. 2005, 12, 1017-1075).

Many of the failures of drug candidates in development programs are attributed to their undesirable pharmacokinetic properties, such as too long or too short $t_{1/2}$, poor absorption, and extensive first-pass metabolism. In a survey, it was reported that of 319 new drug candidates investigated in humans, 77 (40%) of the 198 candidates were withdrawn due to serious pharmacokinetic problems (R. Prentis et al. "Pharmaceutical innovation by seven UK-owned pharmaceutical companies (1964-1985)" Br. J. Clin. Pharmacol. 1988, 25, 387-396). This high failure rate illustrates the importance of pharmacokinetics in drug discovery and development. To ensure the success of a drug's development, it is essential that a drug candidate has good bioavailability and a desirable $t_{1/2}$. Therefore, an accurate estimate of the pharmacokinetic data and a good understanding of the factors that affect the pharmacokinetics will guide drug design (J. Lin, A. Lu "Role of pharmacokinetics and Metabolism in Drug Discovery and Development" Pharmacol. Rev. 1997, 49(4), 404-449). Chemically modifiable factors that influence drug absorption and disposition are discussed as follows.

Some relevant physicochemical and ADME parameters include but are not limited to:

aqueous solubility, logD, PAMPA permeability, Caco-2 permeability, plasma protein binding, microsomal stability and hepatocyte stability.

Poor aqueous solubility can limit the absorption of compounds from the gastrointestinal (GI) tract, resulting in reduced oral bioavailability. It may also necessitate novel formulation strategies and hence increase cost and delays. Moreover, compound solubility can affect other *in vitro* assays. Poor aqueous solubility is an undesired characteristic and it is the largest physicochemical problem hindering oral drug activity (C. A. Lipinski "Drug-like properties and the causes of poor solubility and poor permeability", J. Pharmacol. Toxicol. Methods 2000, 44, 235-249).

Lipophilicity is a key determinant of the pharmacokinetic behaviour of drugs. It can influence distribution into tissues, absorption and the binding characteristics of a drug, as well as being an important factor in determining the solubility of a compound. LogD (distribution coefficient) is used as a measure of lipophilicity. One of the most common methods for determining this parameter is by measuring the partition of a compound between an organic solvent (typically octanol) and aqueous buffer. An optimal range for lipophilicity tends to be if the compound has a logD value between 0 and 3. Typically, these compounds have a good balance between solubility and permeability and this range tends to be optimal for oral absorption and cell membrane permeation. Hydrophilic compounds (logD <0) typically are highly soluble but exhibit low permeability across the gastrointestinal tract or blood brain barrier. Highly lipophilic compounds (logD > 5) exhibit problems with metabolic instability, high plasma protein binding and low solubility which leads to variable and poor oral absorption (L. Di, E. Kerns "Profiling drug-like properties in discovery research" Curr. Opin. Chem. Biol. 2003, 7, 402-408).

Drug permeability through cell monolayers or artificial membranes correlates well with intestinal permeability and oral bioavailability. Drugs with low membrane permeability, i.e. low lipophilicity, are generally absorbed slowly from solution in the stomach and small intestine. Knowing the rate and extent of absorption across the intestinal tract is critical if a drug is to be orally delivered. Drug permeability cannot be accurately predicted by physicochemical factors alone because there are many drug transport pathways. A generally accepted human cell-based model, human colon adenocarcinoma cell line (Caco-2), helps to predict intestinal permeability (A.M. Marino et al. "Validation of the 96-well Caco-2 cell culture model for high-throughput permeability and assessment of discovery compounds", Int. J. Pharmaceutics 2005, 297; 235-241). This assay is commonly employed during early discovery, especially in lead optimisation. A newer in vitro model, known as the parallel artificial membrane permeability assay (PAMPA) ranks compounds on their passive diffusion rates alone. PAMPA is increasingly used as the first-line permeability screen during lead profiling (F. Wohnsland, B. Faller "High-throughput Permeability pH Profile and High-throughput Alkane/Water Log P With Artificial Membranes", J. Med. Chem. 2001, 44, 923-930).

Plasma protein binding (PPB) can significantly affect the therapeutic action of a drug. It determines the extent and duration of action because only unbound drug is thought to be available for passive diffusion to extravascular space or tissue sites where therapeutic effects occur. The level of PPB is important for predicting the pharmacokinetic profile of a drug and determining appropriate oral dosing. *In vivo* dose levels can be estimated from the determined fraction of unbound drug (fu); an increase in dose may be necessary if a drug is highly bound to plasma (Y. Kwon "Handbook of essential pharmacokinetics, pharmacodynamics and drug metabolism for industrial scientists" Springer Verlag 2001).

In vitro models to predict compound metabolism have become accepted adjuncts to animal testing. Early drug metabolism models help predict the metabolic stability of a compound and there are several approaches to doing this. The enzyme sources in these studies are rat or human derived systems that consist of liver microsomes and hepatocytes. Microsomes

contain the full complement of phase I oxidative enzymes but do not have an intact cell membrane. Moreover, microsomes require the addition of a co-factor to the incubation. Hepatocytes are more representative of the *in vitro* situation because they contain a cell membrane and do not require additional cofactors. Hepatocytes contain enzymes for both phase I (oxidation, reduction and/or hydrolysis of test compound) and phase II (conjugation of test compounds or metabolites from phase I) metabolism. The microsomal stability screen is often used as a primary screen early in the drug discovery process. The hepatocyte stability assay is used as a secondary screen for the more favourable compounds discovered from the primary screen (T. Iwatsubo et al. "Prediction of in vivo drug metabolism in the human liver from in vitro metabolism data", Pharm. Ther. 1997, 73, 147-171).

In summary, favourable physicochemical and *in vitro* ADME parameters are prerequisite for a favourable pharmacokinetic (PK) profile of a drug. Obtaining early stage PK data evaluation of new chemical entities is a prerequisite for successful animal pharmacology and toxicology studies. Quantitative measures of drug exposure are key components needed for the sound interpretation of preclinical efficacy studies. PK data can also help in the design or species selection of preclinical toxicology studies. Pharmacokinetic studies are part of the regulatory drug development requirements and have also started to become an integral part of the early drug discovery process.

It is the object of the present invention to provide novel compounds that are agonists or partial agonists of FXR exhibiting physicochemical, *in vitro* and/or *in vivo* ADME (absorption, distribution, metabolism and excretion) properties superior to known agonists of FXR and/or superior pharmacokinetics *in vivo.* Physicochemical and ADME properties affect drug pharmacokinetics and can be assessed by *in vitro* methods.

Unexpectedly, we found that FXR modulating compounds described herein show improved physicochemical and/or ADME parameters *in vitro* resulting in advanced pharmacokinetic properties, i.e. a superior bioavailability and a favourable half life *in vivo* in comparison to the compounds disclosed in the prior art.

As a result, the present invention relates to compounds according to the general formula (I) which bind to the NR1H4 receptor (FXR) and act as agonists or partial agonists of the NR1H4 receptor (FXR). The invention further relates to the use of said compounds for the preparation of medicaments for the treatment of diseases and/or conditions through binding of said nuclear receptor by said compounds. The invention further also describes a method for the synthesis of said compounds. The compounds of the present invention show improved physicochemical and/or ADME parameters *in vitro* finally resulting in advanced pharmacokinetic properties *in vivo.*

The compounds of the present invention are defined by formula (I):

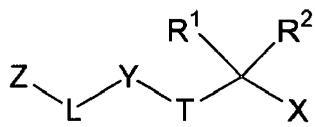

(I)

including enantiomers, diastereomers, tautomers, solvates and pharmaceutically acceptable salts thereof,
wherein

$R^1$ and $R^2$ are independently from each other selected from hydrogen, fluorine, cyano, nitro, azido, $NR^5R^6$, $OR^5$, $SR^5$, $C_1$-$C_6$ alkyl, $C_2$-$C_6$ alkenyl, $C_2$-$C_6$ alkynyl, $C_3$-$C_6$ cycloalkyl; or $R^1$ and $R^2$ are together =O or =S; or $R^1$ and $R^2$ may together form a 3-6-membered carbocyclic or heterocyclic ring which each can be unsaturated or saturated, wherein each alkyl, alkenyl, alkynyl, cycloalkyl group, carbocyclic or heterocyclic ring is unsubstituted or substituted with one to five substituents $R^{11}$; $R^5$ and $R^6$ are independently from each other selected from hydrogen, $C_1$-$C_6$-alkyl and $C_3$-$C_6$-cycloalkyl; or $R^5$ and $R^6$ together may form a 3-6-membered saturated heterocyclic ring, wherein the alkyl, cycloalkyl and heterocyclic group is unsubstituted or substituted with one to five substituents $R^{11}$;

X is

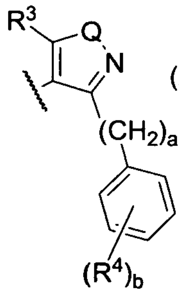 (X$^1$) or 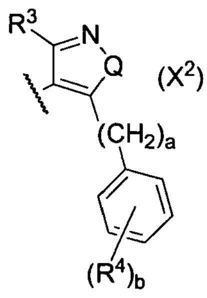 (X$^2$) or 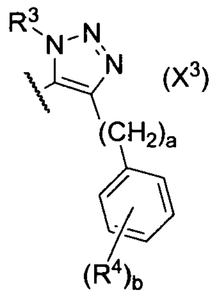 (X$^3$) or 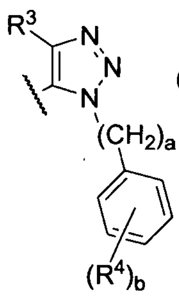 (X$^4$)

in each X$^1$, X$^2$, X$^4$

$R^3$ is hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, -$NR^{19}R^{20}$, $NR^{19}S(O)_mR^{20}$, $NR^{19}C(O)OR^{20}$, $NR^{19}C(O)R^{20}$, $NR^{19}C(O)NR^{19}R^{20}$, $OR^{19}$, $OC(O)R^{19}$, $S(O)_iR^{19}$, $SO_2NR^{19}C(O)R^{20}$, $S(O)_mNR^{19}R^{20}$, $C(O)R^{19}$, $C(O)OR^{20}$, $C(O)NR^{19}R^{20}$, $C(NR^{19})NR^{19}R^{20}$, wherein each alkyl, alkenyl, alkynyl, cycloalkyl heterocyclyl, aryl or heteroaryl is unsubstituted or substituted with one to five substituents $R^{11}$;

in each X$^3$

$R^3$ is hydrogen, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $SO_2R^{19}$, $C(O)R^{19}$, $C(O)OR^{19}$, $C(O)NR^{19}R^{20}$, wherein each alkyl, alkenyl, alkynyl, cycloalkyl, heterocyclyl, aryl or heteroaryl is unsubstituted or substituted with one to five substituents $R^{11}$;

$R^{19}$ and $R^{20}$ are independently from each other selected from hydrogen, $C_1$-$C_6$-alkyl, $C_2$-$C_6$-alkenyl, $C_2$-$C_6$ alkynyl, $C_3$-$C_6$-cycloalky, or $R^{19}$ and $R^{20}$ together may form a 3-7-membered heterocyclic or heteroaromatic ring, and wherein the $C_1$-$C_6$-alkyl, $C_2$-$C_6$-alkenyl, $C_3$-$C_6$-cycloalkyl, heterocyclyl and heteroaryl groups are unsubstituted or substituted with one to five substituents $R^{11}$;

$R^4$ is independently selected from hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{15}R^{16}$, $NR^{15}SO_2R^{16}$, $NR^{15}C(O)OR^{16}$, $NR^{15}C(O)R^{16}$, $NR^{15}C(O)NR^{15}R^{16}$, $NR^{15}C(NCN)NR^{15}R^{16}$, $OR^{15}$, $OC(O)R^{15}$, $S(O)_iR^{15}$, $SO_2NR^{15}C(O)R^{16}$, $S(O)_mNR^{15}R^{16}$, $SC(O)R^{15}$, $C(O)R^{15}$, $C(O)OR^{15}$, $C(O)NR^{15}R^{16}$, $C(O)NHOR^{15}$, $C(O)SR^{15}$, $C(NR^{15})NR^{15}R^{16}$, wherein each alkyl, alkenyl, alkynyl, cycloalkyl, heterocyclyl, aryl or heteroaryl is unsubstituted or substituted with one to five substituents $R^{11}$;

and further two substituents $R^4$ can be taken together with the atom to which they attach to form a 4-7 membered carbocyclic, aryl, heteroaryl or heterocyclic ring, each of which is substituted or unsubstituted with one to five substituents $R^{11}$;

$R^{15}$ and $R^{16}$ are independently from each other selected from hydrogen, $C_1$-$C_6$-alkyl, $C_2$-$C_6$-alkenyl, $C_2$-$C_6$ alkynyl, $C_3$-$C_6$-cycloalkyl; or $R^{15}$ and $R^{16}$ together may form a 3-7-membered heterocyclic or heteroaromatic ring, and wherein the $C_1$-$C_6$-alkyl, $C_2$-$C_6$-alkenyl, $C_3$-$C_6$-cycloalkyl, heterocyclyl and heteroaryl groups are unsubstituted or substituted with one to five substituents $R^{11}$;

$R^{11}$ is independently selected from hydrogen, halogen, cyano, nitro, azido, =O, =S, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{12}R^{13}$, $NR^{12}S(O)_mR^{13}$, $NR^{12}C(O)OR^{13}$, $NR^{12}C(O)R^{13}$, $NR^{12}C(O)NR^{12}R^{13}$, $NR^{12}C(NCN)NR^{12}R^{13}$, =$NOR^{12}$, -$OR^{12}$, $OC(O)R^{12}$, $S(O)_iR^{12}$, $SO_2NR^{12}C(O)R^{13}$, $S(O)_mNR^{12}R^{13}$, $SC(O)R^{12}$, $C(O)R^{12}$, $C(O)OR^{12}$, $C(O)SR^{12}$, $C(O)NR^{12}R^{13}$, $C(O)NOR^{12}$, and $C(NR^{12})NR^{12}R^{13}$;

$R^{12}$ and $R^{13}$ are independently from each other selected from hydrogen, $C_1$-$C_6$ alkyl or $C_3$-$C_6$ cycloalkyl, wherein each alkyl or cycloalkyl may be unsubstituted or substituted with one to five fluorines and/or one or two substituents selected from OH, $OCH_3$, $OCH_2F$, $OCHF_2$, $OCF_3$, =O, $SCF_3$, $NH_2$, $NHCH_3$ and $N(CH_3)_2$; or $R^{12}$ and $R^{13}$ can be taken together with the atom to which they are attached to form a 4 to 6 membered carbocyclic, heteroaryl or heterocyclic ring, each of which may be unsubstituted or substituted with one to five fluorines and/or one or two substituents selected from OH, $OCH_3$, -$OCH_2F$, $OCHF_2$, $OCF_3$, =O, $SCF_3$, $NH_2$, $NHCH_3$ and $N(CH_3)_2$;

Q is O or $NR^7$;

$R^7$ is hydrogen, $C_1$-$C_3$-alkyl, or $C_3$-$C_5$ cycloalkyl, wherein each alkyl or cycloalkyl is unsubstituted or substituted with 1-5 fluorine atoms;

T is -O-, -S-, -$N(R^{14})$-, $CH_2$ or $CF_2$;

$R^{14}$ is hydrogen, $C_1$-$C_3$-alkyl, or $C_3$-$C_5$ cycloalkyl, wherein each alkyl or cycloalkyl is unsubstituted or substituted with 1-5 fluorine atoms;

Y is selected from $Y^1$ to $Y^6$

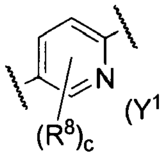 (Y¹)  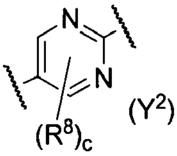 (Y²)  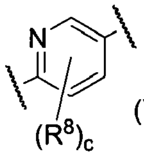 (Y³)

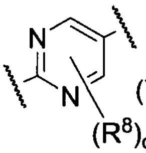 (Y⁴)  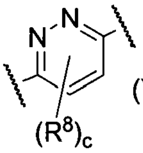 (Y⁵)  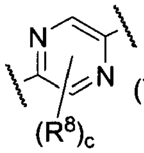 (Y⁶)

$R^8$ is independently selected from hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{12}$ $R^{13}$, $NR^{12}S(O)_mR^{13}$, $NR^{12}C(O)OR^{13}$, $NR^{12}C(O)R^{13}$, $NR^{12}C(O)NR^{12}R^{13}$, $OR^{12}$, $OC(O)R^{12}$, $S(O)_iR^{12}$, $SO_2NR^{12}C(O)R^{13}$, $S(O)_mNR^{12}R^{13}$, $C(O)R^{12}$, $C(O)OR^{12}$, $C(O)NR^{12}R^{13}$, and $C(NR^{12})NR^{12}R^{13}$, wherein each alkyl, alkenyl, alkynyl, cycloalkyl, heterocyclyl, aryl or heteroaryl is unsubstituted or substituted with one to five substituents $R^{11}$;

L is a bond, $-C(O)N(R^{10})-$, $-S(O)_mN(R^{10})-$, $-G-N(R^{10})-$, $-N(R^{10})C(O)-$, $-N(R^{10})S(O)_m-$, $-N(R^{10})-G-$, -G-S-, -G-O-, -S-G-, or O-G; or L is

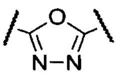 , 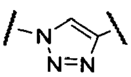 or 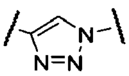

$R^{10}$ is hydrogen, $C_1$-$C_3$-alkyl, or $C_3$-$C_5$ cycloalkyl, wherein each alkyl or cycloalkyl is unsubstituted or substituted with 1-5 fluorine atoms;

G is methylene or ethylene which is unsubstituted or substituted with 1-5 fluorine atoms;

Z is phenyl-A-$R^9$, pyridyl-A-$R^9$, pyrimidyl-A-$R^9$ or pyridazyl-A-$R^9$, wherein phenyl, pyridyl, pyrimidyl or pyridazyl is unsubstituted or substituted with one, two or three groups selected from halogen, $C_1$-$C_4$ alkyl, $C_3$-$C_5$ cycloalkyl, $C_2$-$C_4$ alkenyl, $C_2$-$C_4$ alkynyl, cyano, OH, $OCH_3$, $OCH_2F$, $OCHF_2$, $OCF_3$, $SCF_3$, $NH_2$, $NHCH_3$ and $N(CH_3)_2$;

A is a bond, $CH_2$, $CHCH_3$, $C(CH_3)_2$ or $CF_2$;

$R^9$ is hydrogen, $COOR^{17}$, $CONR^{17}R^{18}$, $C(O)NHSO_2R^{17}$, $SO_2NHC(O)R^{17}$, $S(O)_mR^{17}$, $C(NR^{17})NR^{17}R^{18}$, or tetrazole which is connected to A via the C-atom;

$R^{17}$ and $R^{18}$ are independently from each other selected from hydrogen, $C_1$-$C_6$-alkyl, $C_2$-$C_6$-alkenyl, $C_2$-$C_6$ alkynyl, and $C_3$-$C_6$-cycloalkyl; or $R^{17}$ and $R^{18}$ together may form a 3-7-membered heterocyclic or heteroaromatic ring, wherein the alkyl, alkenyl, cycloalkyl, heterocyclyl and heteroaryl groups are unsubstituted or substituted with one to five substituents $R^{11}$;

a is 0 or 1;

b is 1, 2, or 3;

c is 1 or 2;

i is 0, 1, or 2; and

m is 1 or 2.

Preferably, $R^1$ and $R^2$ are independently from each other selected from hydrogen, fluorine and $C_{1-6}$ alkyl wherein the alkyl group is unsubstituted or substituted with one to five substituents $R^{11}$; or $R^1$ and $R^2$ are together =O or =S. More preferably, $R^1$ and $R^2$ are independently from each other selected from hydrogen and methyl.

It is preferred that in each $X^1$, $X^2$ and $X^4$ $R^3$ is hydrogen, $C_1$-$C_6$ alkyl, $NR^{19}R^{20}$ or $C_3$-$C_6$ cycloalkyl, wherein each alkyl or cycloalkyl is unsubstituted or substituted with one to five substituents $R^{11}$, preferably one, two or three substituents $R^{11}$, and that in each $X^3$ $R^3$ is hydrogen, $C_{1-6}$ alkyl or $C_3$-$C_6$ cycloalkyl, wherein each alkyl or cycloalkyl is unsubstituted or substituted with one to five substituents $R^{11}$, preferably one, two or three substituents $R^{11}$.

It is further preferred that $R^{19}$ and $R^{20}$ are independently from each other selected from hydrogen, $C_1$-$C_6$ alkyl

and $C_3$-$C_6$ cycloalkyl. In an alternative embodiment, $R^{19}$ and $R^{20}$ preferably form together a 3-7-membered heterocyclic or heteroaromatic ring. The alkyl, cycloalkyl, heterocyclic or heteroaromatic groups are unsubstituted or substituted with one to five substituents $R^{11}$, preferably one, two or three substituents $R^{11}$.
In a preferred embodiment, Q is O or NH.
In each of $X^1$ to $X^4$, $R^4$ is preferably selected from hydrogen, halogen, $C_{1-6}$ alkyl, O-$C_1$-$C_6$ alkyl, and CN, wherein each alkyl group is unsubstituted or substituted by one to five substituents $R^{11}$, preferably one, two or three substituents $R^{11}$. More preferably, $R^4$ is selected from hydrogen, halogen and $C_{1-6}$ alkyl wherein each alkyl group is unsubstituted or substituted by one, two or three substituents $R^{11}$.
The index b preferably is 1 or 2; most preferably b is 2.
The radical $R^4$ may be located on any position of the phenyl ring. Preferably, $R^4$ is located on the 2- and/or 4- and/or 6-position of the phenyl ring. Most preferably, $R^4$ is located on the 2- and 6-position of the phenyl ring.
In a preferred embodiment T is O, $CH_2$ or $NR^{14}$ wherein $R^{14}$ is as defined above.
Y is preferably selected from $Y^1$, $Y^2$ and $Y^3$ wherein $R^8$ and c are defined as above.
Preferably, $R^8$ is independently selected from hydrogen, halogen, $C_1$-$C_6$ alkyl, $OR^{12}$, $NR^{12}R^{13}$, C(O)$R^{12}$ and C(O)O$R^{12}$ wherein each alkyl is unsubstituted or substituted by one to five substituents $R^{11}$, preferably one, two or three substituents $R^{11}$ and wherein $R^{12}$ and $R^{13}$ are defined as above. More preferably, $R^{12}$ and $R^{13}$ are independently selected from hydrogen and $C_1$-$C_6$ alkyl. In a further preferred embodiment $R^8$ is independently selected from hydrogen, halogen, $C_1$-$C_6$-alkyl, or O-$C_1$-$C_3$-alkyl,
wherein each alkyl group is unsubstituted or substituted with one to five substituents $R^{11}$, preferably one, two or three substituents $R^{11}$.
L is preferably a bond, -C(O)N($R^{10}$)-, -S(O)$_i$N($R^{10}$)-, -G-N($R^{10}$)-, or -N($R^{10}$)-G, wherein $R^{10}$ is hydrogen or $C_1$-$C_6$-alkyl and i is 2.
It is preferred that Z is phenyl-A-$R^9$, wherein phenyl is unsubstituted or substituted with one to three groups selected from halogen, cyano, $C_{1-4}$ alkyl, OH, $OCH_3$, $OCH_2F$, $OCHF_2$, $OCF_3$, $SCF_3$, $NH_2$, $NHCH_3$ and $N(CH_3)_2$.

Preferred compounds of formula (I) are those compounds in which one or more of the residues contained therein have the meanings given above. It is understood, that the claimed compounds cover any compound obtained by combining any of the definitions disclosed within this description for the various substituents. With respect to all compounds of formula (I), the present invention also includes all tautomeric and stereoisomeric forms, solvates and mixtures thereof in all ratios, and their pharmaceutically acceptable salts.
In the above and the following, the employed terms have independently the meaning as described below:

Aryl is an aromatic mono- or polycyclic moiety with preferably 6 to 20 carbon atoms which is preferably selected from phenyl, biphenyl, naphthyl, tetrahydronaphthyl, fluorenyl, indenyl and phenanthrenyl, more preferably phenyl and naphthyl.

Heteroaryl is a monocyclic or polycyclic aromatic moiety having 5 to 20 carbon atoms with at least one ring containing a heteroatom selected from O, N and/or S, or heteroaryl is an aromatic ring containing at least one heteroatom selected from O, N and/or S and 1 to 6 carbon atoms. Preferably, heteroaryl contains 1 to 4, more preferably 1, 2 or 3 heteroatoms selected from O and/or N and is preferably selected from pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl and furopyridinyl. Spiro moieties are also included within the scope of this definition. Preferred heteroaryl includes pyridinyl, imidazolyl, pyrimidinyl, pyrazolyl, triazolyl, pyrazinyl, tetrazolyl, isoxazolyl, oxazolyl, isothiazolyl, oxadiazolyl and triazolyl.

Heterocyclyl is a 3 to 10-membered saturated or unsaturated ring containing at least one heteroatom selected from O, N and/or S and 1 to 6 carbon atoms. Preferably, heterocyclyl contains 1 to 4, more preferably 1, 2 or 3 heteroatoms selected from O and/or N. Heterocyclyl includes mono- and bicyclic ringsystems and is preferably selected from pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, azetidin-2-one-1-yl, pyrrolidin-2-one-1-yl, piperid-2-one-1-yl, azepan-2-one-1-yl, 3-azabicyco[3.1.0] hexanyl, 3-azabicyclo[4.1.0]heptanyl, azabicyclo[2.2.2]hexanyl, 3H-indolyl and quinolizinyl. Spiromoieties are also included within the scope of this definition.

$C_1$-$C_6$ Alkyl is a saturated hydrocarbon moiety, namely straight chain or branched alkyl having 1 to 6 carbon atoms,

preferably 1 to 4 carbon atoms, such as methyl, ethyl, n-propyl, isopropyl, n-butyl, isobutyl, tert-butyl, n-pentyl, isopentyl, neopentyl or hexyl.

$C_3$-$C_6$ Cycloalkyl is an alkyl ring having 3 to 6, such as cyclopropyl, cyclobutyl, cyclopentyl or cyclohexyl.

Carbocyclyl is a monocyclic or polycyclic ring system of 3 to 20 carbon atoms which may be saturated or unsaturated. Thus, the term "carbocyclyl" includes cycloalkyls as defined above as well as partially unsaturated carbocyclic groups such as cyclopentene, cyclopentadiene or cyclohexene.

$C_2$-$C_6$ Alkenyl is an unsaturated hydrocarbon moiety with one or more double bonds, preferably one double bond, namely straight chain or branched alkenyl having 2 to 6 carbon atoms, such as vinyl, allyl, methallyl, buten-2-yl, buten-3-yl, penten-2-yl, penten-3-yl, penten-4-yl, 3-methyl-but-3-enyl, 2-methyl-but-3-enyl, 1-methyl-but-3-enyl or hexenyl.

$C_2$-$C_6$ Alkynyl is an unsaturated hydrocarbon moiety with one or more triple bonds, preferably one triple bond, namely straight chain or branched alkynyl having 2 to 6 carbon atoms, such as ethynyl, propynyl, butyn-2-yl, butyn-3-yl, pentyn-2-yl, pentyn-3-yl, pentyn-4-yl, 2-methyl-but-3-ynyl, 1-methyl-but-3-ynyl or hexynyl.

Halo or halogen is a halogen atom selected from F, Cl, Br and I, preferably F, Cl and Br.

Preferred embodiments of the compounds according to the present invention are shown below.

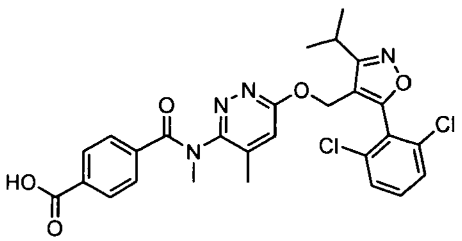
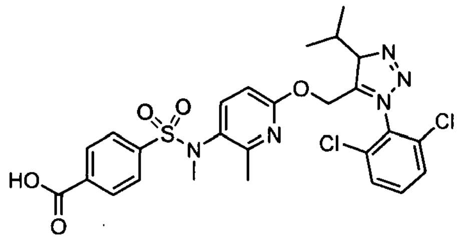
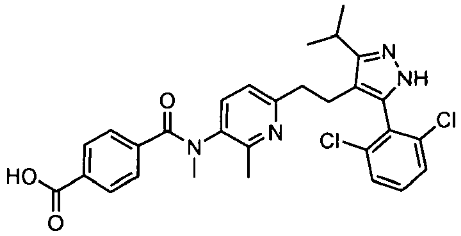
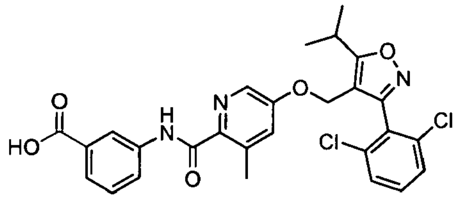
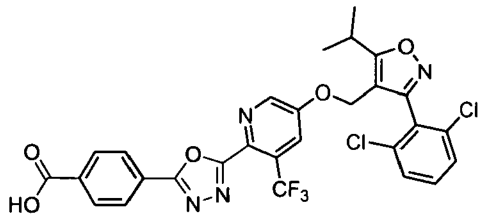
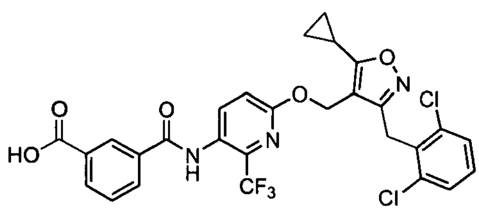
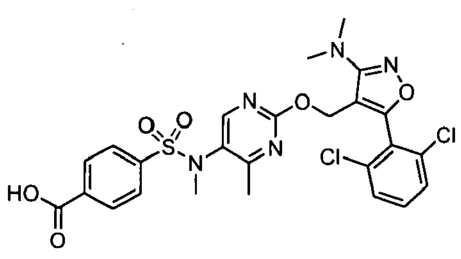
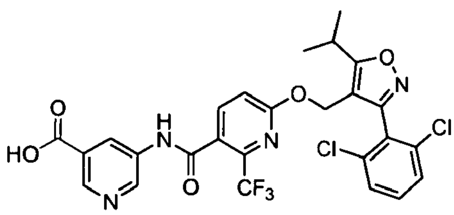

The compounds of the present invention can be in the form of a prodrug compound. "Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods. Other examples of the prodrug are compounds, wherein the carboxylate in a compound of the present invention is, for example, converted into an alkyl-, aryl-, choline-, amino, acyloxymethylester, linolenoyl-ester.

Metabolites of compounds of the present invention are also within the scope of the present invention.

Where tautomerism, like e.g. keto-enol tautomerism, of compounds of the present invention or their prodrugs may occur, the individual forms, like e.g. the keto and enol form, are each within the scope of the invention as well as their mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials.

The compounds of the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be present on these groups and can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Furthermore, the present invention provides pharmaceutical compositions comprising at least one compound of the present invention, or a prodrug compound thereof, or a pharmaceutically acceptable salt or solvate thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing at least one compound of the present invention and a pharmaceutically acceptable carrier.

The pharmaceutical composition of the present invention may additionally comprise one or more other compounds as active ingredients like a prodrug compound or other nuclear receptor modulators.

The compositions are suitable for oral, rectal, topical, parenteral (including subcutaneous, intramuscular, and intravenous), ocular (ophthalmic), pulmonary (nasal or buccal inhalation) or nasal administration, although the most suitable route in any given case will depend on the nature and severity of the conditions being treated and on the nature of the active ingredient. They may be conveniently presented in unit dosage form and prepared by any of the methods well-known in the art of pharmacy.

The compounds of the present invention bind to the NR1H4 receptor (FXR) and act as agonists or partial agonists of

the NR1H4 receptor (FXR).

FXR is proposed to be a nuclear bile acid sensor. As a result, it modulates both, the synthetic output of bile acids in the liver and their recycling in the intestine (by regulating bile acid binding proteins). But beyond bile acid physiology, FXR seems to be involved in the regulation of many diverse physiological processes which are relevant in the etiology and for the treatment of diseases as diverse as cholesterol gallstones, metabolic disorders such as Type II Diabetes, dyslipidemias or obesity, chronic inflammatory diseases such as Inflammatory Bowel Diseases or chronic intrahepatic forms of cholestasis and many others diseases (T. Claudel et al. "The Farnesoid X receptor: a molecular link between bile acid and lipid and glucose metabolism" Arterioscler. Thromb. Vasc. Biol. 2005, 25(10), 2020-2030; S. Westin et al. "FXR, a therapeutic target for bile acid and lipid disorders" Mini Rev. Med. Chem. 2005, 5(8), 719-727).

FXR regulates a complex pattern of response genes in the liver. The gene products have impact on diverse physiological processes. In the course of functional analysis of FXR, the first regulatory network that was analyzed was the regulation of bile acid synthesis. While the LXRs induce the key enzyme of the conversion of cholesterol into bile acids, Cyp7A1, via the induction of the regulatory nuclear receptor LRH-1, FXR represses the induction of Cyp7A1 via the upregulation of mRNA encoding SHP, a further nuclear receptor that is dominant repressive over LRH-1. Since FXR binds the end products of this pathway, primary bile acids such as cholic acid (CA) or chenodeoxycholic acid (CDCA), this can be regarded as an example of feedback inhibition on the gene expression level (B. Goodwin et al. "A regulatory cascade of the nuclear receptors FXR, SHP-1, and LRH-1 represses bile acid biosynthesis" Mol. Cell 2000, 6(3), 517-526; T. Lu et al. "Molecular basis for feedback regulation of bile acid synthesis by nuclear receptors" Mol. Cell 2000, 6(3), 507-515).

Parallel to the repression of bile acid synthesis via SHP, FXR induces a range of so-called ABC (for ATP-binding cassette) transporters that are responsible for the export of toxic bile acids from the hepatocyte cytosol into the canaliculi, the small bile duct ramifications where the bile originates. This hepatoprotective function of FXR became first apparent with the analysis of FXR knockout mice (C. Sinal et al. "Targeted disruption of the nuclear receptor FXR/BAR impairs bile acid and lipid homeostasis" Cell 2000, 102(6), 731-744). where under- or overexpression of several ABC-transporters in the liver was shown. Further detailed analysis revealed that the major bile salt excretory pump BSEP or ABCB11 (M. Ananthanarayananet al. "Human bile salt export pump promoter is transactivated by the farnesoid X receptor/bile acid receptor" J. Biol. Chem. 2001, 276(31), 28857-28865; J. Plass et al. "Farnesoid X receptor and bile salts are involved in transcriptional regulation of the gene encoding the human bile salt export pump" Hepatology 2002, 35(3), 589-96) as well as the key enzyme which mediates lipid transfer from lipoproteins to phospholipids, PLTP (N. Urizar et al. "The farnesoid X-activated receptor mediates bile acid activation of phospholipid transfer protein gene expression" J. Biol. Chem. 2000, 275(50), 39313-39317), and the two key canalicular membrane transporters for phospholipids, MRP-2 (ABCC4) (H. Kast et al. "Regulation of multidrug resistance-associated protein 2 (ABCC2) by the nuclear receptors pregnane X receptor, farnesoid X-activated receptor, and constitutive androstane receptor" J. Biol. Chem. 2002, 277 (4), 2908-2915) and MDR-3 (ABCB4); L. Huang et al. "Farnesoid X receptor activates transcription of the phospholipid pump MDR3" J. Biol. Chem. 2003, 278(51), 51085-51090) are direct targets for ligand-directed transcriptional activation by FXR (summarized in: M. Miyata "Role of farnesoid X receptor in the enhancement of canalicular bile acid output and excretion of unconjugated bile acids: a mechanism for protection against cholic acid-induced liver toxicity", J. Pharmacol. Exp. Ther. 2005, 312(2), 759-766; G. Rizzo et al. "Role of FXR in regulating bile acid homeostasis and relevance for human diseases" Curr. Drug Targets immune Endocr. Metabol. Disord. 2005, 5(3), 289-303.)

The fact that FXR seems to be the major metabolite sensor and regulator for the synthesis, export and re-circulation of bile acids suggested the use of FXR ligands to induce bile flow and change bile acid composition towards more hydrophilic composition. With the development of the first synthetic FXR ligand GW4064 (P. Maloney et al. "Identification of a chemical tool for the orphan nuclear receptor FXR" J. Med. Chem. 2000, 43(16), 2971-2974; T. Willson et al. "Chemical genomics: functional analysis of orphan nuclear receptors in the regulation of bile acid metabolism" Med. Res. Rev. 2001, 21(6) 513-22) as a tool compound and of the semi-synthetic artificial bile acid ligand 6-alpha-ethyl-CDCA, the effects of superstimulation of FXR by potent agonists could be analyzed. It was shown that both ligands induce bile flow in bile duct ligated animals. Moreover, in addition to choleretic effects, also hepatoprotective effects could be demonstrated (R. Pellicciari et al. "6alpha-ethyl-chenodeoxycholic acid (6-ECDCA), a potent and selective FXR agonist endowed with anticholestatic activity" J. Med. Chem. 2002, 45(17), 3569-3572; Y. Liu et al. "Hepatoprotection by the farnesoid X receptor agonist GW4064 in rat models of intra- and extrahepatic cholestasis" J. Clin. Invest. 2003, 112(11), 1678-1687). This hepatoprotective effect was further narrowed down to an anti-fibrotic effect that results from the repression of Tissue Inhibitors of Matrix-Metalloproteinases, TIMP-1 and 2, the induction of collagen-deposit resolving Matrix-Metalloproteinase 2 (MMP-2) in hepatic stellate cells and the subsequent reduction of alpha-collagen mRNA and Transforming growth factor beta (TGF-beta) mRNA which are both pro-fibrotic factors by FXR agonists (S. Fiorucci et al. "The nuclear receptor SHP mediates inhibition of hepatic stellate cells by FXR and protects against liver fibrosis", Gastroenterology 2004, 127 (5), 1497-1512; S. Fiorucci et al. "A farnesoid x receptor-small heterodimer partner regulatory cascade modulates tissue metalloproteinase inhibitor-1 and matrix metalloprotease expression in hepatic stellate cells and promotes resolution of liver fibrosis"J. Pharmacol. Exp. Ther. 2005, 314(2), 584-595). The anti-fibrotic activity of FXR is at least partially mediated by the induction of PPARgamma, a further nuclear receptor, with which anti-fibrotic activity is associated (S. Fiorucci et

al. "Cross-talk between farnesoid-X-receptor (FXR) and peroxisome proliferator-activated receptor gamma contributes to the antifibrotic activity of FXR ligands in rodent models of liver cirrhosis" J. Pharmacol. Exp. Ther. 2005, 315(1), 58-68; A. Galli et al. "Antidiabetic thiazolidinediones inhibit collagen synthesis and hepatic stellate cell activation in vivo and in vitro" Gastroenterology 2002, 122(7), 1924-1940; I. Pineda Torra et al., "Bile acids induce the expression of the human peroxisome proliferator-activated receptor alpha gene via activation of the farnesoid X receptor" Mol. Endocrinol. 2003, 17(2), 259-272). Furthermore, anti-cholestatic activity was demonstrated in bile-duct ligated animal models as well as in animal models of estrogen-induced cholestasis (S. Fiorucci et al. "Protective effects of 6-ethyl chenodeoxycholic acid, a farnesoid X receptor ligand, in estrogen-induced cholestasis" J. Pharmacol. Exp. Ther. 2005, 313(2), 604-612).
Genetic studies demonstrate that in hereditary forms of cholestasis (Progressive Familiar Intrahepatic Cholestasis = PFIC, Type I - IV) either nuclear localization of FXR itself is reduced as a consequence of a mutation in the FIC1 gene (in PFIC Type I, also called Byler's Disease) (F. Chen et al. "Progressive familial intrahepatic cholestasis, type 1, is associated with decreased farnesoid X receptor activity" Gastroenterology. 2004, 126(3), 756-64; L. Alvarez et al. "Reduced hepatic expression of farnesoid X receptor in hereditary cholestasis associated to mutation in ATP8B1" Hum. Mol. Genet. 2004; 13(20), 2451-60) or levels of the FXR target gene encoding MDR-3 phospholipid export pump are reduced (in PFIC Type III). Taken together there is a growing body of evidence that FXR binding compounds will demonstrate substantial clinical utility in the therapeutic regimen of chronic cholestatic conditions such as Primary Biliary Cirrhosis (PBC) or Primary Sclerosing Cholangitis (PSC) (reviewed in: G. Rizzo et al. Curr. Drug Targets Immune Endocr. Metabol. Disord. 2005, 5(3), 289-303; G. Zollner "Role of nuclear receptors in the adaptive response to bile acids and cholestasis: pathogenetic and therapeutic considerations" Mol. Pharm. 2006, 3(3), 231-51, S. Cai et al. "FXR: a target for cholestatic syndromes?" Expert Opin. Ther. Targets 2006, 10(3), 409-421).
The deep impact that FXR activation has on bile acid metabolism and excretion is not only relevant for cholestatic syndromes but even more directly for a therapy against gallstone formation. Cholesterol gallstones form due to low solubility of cholesterol that is actively pumped out of the liver cell into the lumen of the canaliculi. It is the relative percentage of content of the three major components, bile acids, phospholipids and free cholesterol that determines the formation of mixed micelles and hence apparent solubility of free cholesterol in the bile. FXR polymorphisms map as quantitative trait loci as one factor contributing to gallstone disease (H. Wittenburg "FXR and ABCG5/ABCG8 as determinants of cholesterol gallstone formation from quantitative trait locus mapping in mice", Gastroenterology 2003, 125 (3), 868-881). Using the synthetic FXR tool compound GW4064 it could be demonstrated that activation of FXR leads to an improvement of the Cholesterol Saturation Index (=CSI) and directly to an abolishment of gallstone formation in C57L gallstone susceptible mice whereas drug treatment in FXR knockout mice shows no effect on gallstone formation (A. Moschetta et al. "Prevention of cholesterol gallstone disease by FXR agonists in a mouse model" Nature Medicine 2004, 10(12), 1352-1358).
These results qualify FXR as a good target for the development of small molecule agonists that can be used to prevent cholesterol gallstone formation or to prevent reformation of gallstones after surgical removal or shockwave lithotripsy (discussed in:

S. Doggrell "New targets in and potential treatments for cholesterol gallstone disease" Curr. Opin. Investig. Drugs 2006, 7(4), 344-348).

Since the discovery of the first synthetic FXR agonist and its administration to rodents it became evident that FXR is a key regulator of serum triglycerides (P. Maloney et al. J. Med. Chem. 2000, 43(16), 2971-2974; T. Willson et al. Med. Res. Rev. 2001, 21(6), 513-22). Over the past six years accumulating evidence has been published that activation of FXR by synthetic agonists leads to significant reduction of serum triglycerides, mainly in the form of reduced VLDL, but also to reduced total serum cholesterol (H. Kast et al. "Farnesoid X-activated receptor induces apolipoprotein C-II transcription: a molecular mechanism linking plasma triglyceride levels to bile acids" Mol. Endocrinol. 2001, 15(10), 1720-1728; N.Urizar et al. "A natural product that lowers cholesterol as an antagonist ligand for FXR" Science 2002, 296(5573), 1703-1706; G. Lambert et al. "The farnesoid X-receptor is an essential regulator of cholesterol homeostasis" J. Biol. Chem. 2003, 278, 2563-2570; M. Watanabe et al. "Bile acids lower triglyceride levels via a pathway involving FXR, SHP, and SREBP-1c" J. Clin. Invest. 2004, 113(10), 1408-1418; A. Figge et al. "Hepatic overexpression of murine Abcb11 increases hepatobiliary lipid secretion and reduces hepatic steatosis "J. Biol. Chem. 2004, 279(4), 2790-2799; S. Bilz et al. "Activation of the farnesoid X receptor improves lipid metabolism in combined hyperlipidemic hamsters" Am. J. Physiol. Endocrinol. Metab. 2006, 290(4), E716-22).
But the lowering of serum triglycerides is not a stand alone effect. Treatment of db/db or ob/ob mice with synthetic FXR agonist GW4064 resulted in marked and combined reduction of serum triglycerides, total cholesterol, free fatty acids, ketone bodies such as 3-OH Butyrate. Moreover, FXR activation engages with the intracellular insulin signaling pathway in hepatocytes, resulting in reduced output of glucose from liver gluconeogenesis but concomitant increase in liver glycogen. Insulin sensitivity as well as glucose tolerance were positively impacted by FXR treatment (K. Stayrook et al. "Regulation of carbohydrate metabolism by the farnesoid X receptor" Endocrinology 2005, 146(3), 984-91; Y. Zhang et

al. "Activation of the nuclear receptor FXR improves hyperglycemia and hyperlipidemia in diabetic mice" Proc. Natl. Acad. Sci. USA 2006, 103(4), 1006-1011; B. Cariou et al. "The farnesoid X receptor modulates adiposity and peripheral insulin sensitivity in mice" J. Biol. Chem. 2006, 281, 11039-11049; K. Ma et al. "Farnesoid X receptor is essential for normal glucose homeostasis" J. Clin. Invest. 2006, 116(4), 1102-1109; D. Duran-Sandoval et al. "Potential regulatory role of the farnesoid X receptor in the metabolic syndrome" Biochimie 2005, 87(1), 93-98). An effect on reduction of body weight was also recently observed in mice overfed with a high lipid diet (C. Lihong et al."FXR Agonist, GW4064, Reverses Metabolic Defects in High-Fat Diet Fed Mice" American Diabetes Association (ADA) 66th annual scientific sessions, June 2006, Abstract Number 856-P). This weight loss effect might results from FXR's induction of FGF-19, a fibroblast growth factor that is known to lead to weight loss and athletic phenotype (J. Holt et al. Genes Dev. 2003, 17 (13), 1581-1591; E. Tomlinson et al. "Transgenic mice expressing human fibroblast growth factor-19 display increased metabolic rate and decreased adiposity" Endocrinology 2002, 143(5), 1741-1747). In recent patent applications, the effect of FXR agonist on reduction of body weight was demonstrated (Stoffel W. et al. "Methods for inhibiting Adipogenesis and for treating Type 2 Diabetes" International Patent Application WO 2004/087076; S. Jones et al "Methods of using FXR Agonists" International Patent Application WO 2003/080803).

Taken together, these pharmacological effects of FXR agonists can be exploited in different therapeutic ways: FXR binding compounds are thought to be good candidates for the treatment of Type II Diabetes because of their insulin sensitization, glycogenogenic, and lipid lowering effects.

In one embodiment, the compounds according to the invention and pharmaceutical compositions comprising said compounds are used in the treatment of Type II Diabetes which can be overcome by FXR-mediated upregulation of systemic insulin sensitivity and intracellular insulin signalling in liver, increased peripheral glucose uptake and metabolisation, increased glycogen storage in liver, decreased output of glucose into serum from liver-borne gluconeogenesis.

In a further embodiment, said compounds and pharmaceutical compositions are used for the preparation of a medicament for the treatment of chronic intrahepatic and some forms of extrahepatic cholestatic conditions, such as primary biliary cirrhosis (PBC), primary sclerosing cholangitis (PSC), progressive familiar cholestasis (PFIC), alcohol-induced cirrhosis and associated cholestasis, or liver fibrosis resulting from chronic cholestatic conditions or acute intraheptic cholestatic conditions such as estrogen or drug induced cholestasis.

The invention also relates to a compound of formula (I) or to a pharmaceutical composition comprising said compound for the treatment of gastrointestinal conditions with a reduced uptake of dietary fat and fat-soluble dietary vitamins which can be overcome by increased intestinal levels of bile acids and phospholipids.

In a further embodiment, said compound or pharmaceutical composition is used for treating a disease selected from the group consisting of lipid and lipoprotein disorders such as hypercholesterolemia, hypertriglyceridemia, and atherosclerosis as a clinically manifest condition which can be ameliorated by FXR's beneficial effect on raising HDL cholesterol, lowering serum triglycerides, increasing conversion of liver cholesterol into bile acids and increased clearance and metabolic conversion of VLDL and other lipoproteins in the liver.

In one further embodiment, said compound and pharmaceutical composition are used for the preparation of a medicament where the combined lipid lowering, anti-cholestatic and anti-fibrotic effects of FXR-targeted medicaments can be exploited for the treatment of liver steatosis and associated syndromes such as non-alcoholic steatohepatitis ("NASH"), or for the treatment of cholestatic and fibrotic effects that are associated with alcohol-induced cirrhosis, or with viral-borne forms of hepatitis.

In conjunction with the hypolipidemic effects it was also shown that loss of functional FXR leads to increased atherosclerosis in ApoE knockout mice (E. Hanniman et al. "Loss of functional farnesoid X receptor increases atherosclerotic lesions in apolipoprotein E-deficient mice" J. Lipid Res. 2005, 46(12), 2595-2604). Therefore, FXR agonists might have clinical utility as anti-atherosclerotic and cardioprotective drugs. The downregulation of Endothelin-1 in Vascular Smooth Muscle Cells might also contribute to such beneficial therapeutic effects (F. He et al. "Downregulation of endothelin-1 by farnesoid X receptor in vascular endothelial cells" Circ. Res. 2006, 98(2), 192-9).

The invention also relates to a compound according to formula (I) or a pharmaceutical composition comprising said compound for preventive and posttraumatic treatment of cardiovascular disorders such as acute myocardial infarction, acute stroke, or thrombosis which occur as an endpoint of chronic obstructive atherosclerosis.

In a few selected publications the effects of FXR and FXR agonists on proliferation of cancer and non-malignant cells and apoptosis have been assessed. From these preliminary results it seems as if FXR agonists might also influence apoptosis in cancer cell lines (E. Niesor et al. "The nuclear receptors FXR and LXRalpha: potential targets for the development of drugs affecting lipid metabolism and neoplastic diseases" Curr. Pharm. Des. 2001, 7(4), 231-59) and in Vascular Smooth Muscle Cells (VSMCs) (D. Bishop-Bailey et al. "Expression and activation of the farnesoid X receptor in the vasculature" Proc. Natl. Acad. Sci. USA. 2004, 101(10), 3668-3673). Furthermore, FXR seems to be expressed in metastasizing breast cancer cells and in colon cancer (J. Silva "Lipids isolated from bone induce the migration of human breast cancer cells" J. Lipid Res. 2006, 47(4), 724-733; G. De Gottardi et al. "The bile acid nuclear receptor FXR and the bile acid binding protein IBABP are differently expressed in colon cancer" Dig. Dis. Sci. 2004, 49(6), 982-989). Other publications that focus primarily on FXR's effect on metabolism draw a line to intracellular signaling from FXR via

the Forkhead /Wingless (FOXO) family of transcriptional modulators to the Phosphatidylinositol-trisphosphat ($PI_3$)- Kinase / Akt signal transduction pathway (D. Duran-Sandoval et al. J. Biol. Chem. 2005, 280(33), 29971-29979; Y. Zhang et al. Proc. Natl. Acad. Sci. USA. 2006, 103(4), 1006-1011) that is similarily employed by insulin intracellular signaling as well as neoplastically transformed cells.
This would allow to regard FXR also as a potential target for the treatment of proliferative diseases, especially metastasizing cancer forms that overexpress FXR or those where the FOXO /$PI_3$- Kinase / Akt Pathway is responsible for driving proliferation.
Therefore, the compounds according to formula (I) or pharmaceutical composition comprising said compounds are suitable for treating Non-malignant hyperproliferative disorders such as increased neointima formation after balloon vessel dilatation and stent application due to increased proliferation of vascular smooth muscle cells (VSMCs) or Bening Prostate Hyperplasia (BPH), a pre-neoplastic form of hyperproliferation, other forms of scar tissue formation and fibrotisation which can be overcome by e.g. FXR-mediated intervention into the PI-3Kinase / AKT / mTOR intracellular signalling pathway, reduction in Matrix-Metalloproteinase activity and alpha-Collagen deposition.
In a further embodiment, said compounds and pharmaceutical compositions are used for the treatment of malignant hyperproliferative disorders such as all forms of cancer (e.g. certain forms of breast or prostate cancer) where interference with PI-3-Kinase/AKT/mTOR signalling and / or induction of $p27^{kip}$ and / or induction of apoptosis will have a beneficial impact.
Finally, FXR seems also to be involved in the control of antibacterial defense in the intestine (T. Inagaki et al. "Regulation of antibacterial defense in the small intestine by the nuclear bile acid receptor" Proc. Natl. Acad. Sci. USA. 2006, 103 (10), 3920-3905) although an exact mechanism is not provided. From these published data, however, one can conclude that treatment with FXR agonists might have a beneficial impact in the therapy of Inflammatory Bowel Disorders (IBD), in particular those forms where the upper (ileal) part of the intestine is affected (e.g. ileal Crohn's disease) because this seems to be the site of action of FXR's control on bacterial growth. In IBD the desensitization of the adaptive immune response is somehow impaired in the intestinal immune system. Bacterial overgrowth might then be the causative trigger towards establishment of a chronic inflammatory response. Hence, dampening of bacterial growth by FXR-borne mechanisms might be a key mechanism to prevent acute inflammatory episodes.
Thus, the invention also relates to a compound according to formula (I) or a pharmaceutical composition comprising said compound for treating a disease related to Inflammatory Bowel Diseases such as Crohn's disease or Colitis ulcerosa. FXR-mediated restoration of intestinal barrier function and reduction in non-commensal bacterial load is believed to be helpful in reducing the exposure of bacterial antigens to the intestinal immune system and can therefore reduce inflammatory responses.
The invention further relates to a compound or pharmaceutical composition for the treatment of obesity and associated disorders such as metabolic syndrome (combined conditions of dyslipidemias, diabetes and abnormally high body-mass index) which can be overcome by FXR-mediated lowering of serum triglycerides, blood glucose and increased insulin sensitivity and FXR-mediated weight loss.
In one embodiment, said compound or pharmaceutical composition is for treating persistent infections by intracellular bacteria or parasitic protozoae such as *Mycobacterium spec.* (Treatment of Tuberculosis or Lepra), *Listeria monocytogenes* (Treatment of Listeriosis), *Leishmania spec.* (Leishmaniosis), *Trypanosoma spec.* (Chagas Disease; Trypanosomiasis; Sleeping Sickness).
In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.
The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form

is a capsule, it may contain, in addition to materials of the above type, a liquid carrier such as a fatty oil.
Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.
The compounds of the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.
The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.
Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of the present invention are administered orally.
The effective dosage of active ingredient employed may vary depending on the particular compound employed, the mode of administration, the condition being treated and the severity of the condition being treated. Such dosage may be ascertained readily by a person skilled in the art.
When treating or preventing FXR mediated conditions for which compounds of the present invention are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligram to about 50 milligrams. In the case of a 70 kg adult human, the total daily dose will generally be from about 7 milligrams to about 350 milligrams. This dosage regimen may be adjusted to provide the optimal therapeutic response.
Some abbreviations that appear in this application are as follows.

***Abbreviations***

Table 1

| Abbreviation | Designation |
|---|---|
| d | Doublet |
| DCC | Dicyclohexylcarbodiimid |
| DMF | N,N-Dimethyl formamide |
| DMFA | Dimethyl formamide |
| EDC | 1-[3-(Dimethylamino)propyl]-3-ethylcarbodiimide |
| LC | Liquid Chromatography |
| m | Multiplett |
| MS | Mass Spectrometry |
| NMR | Nuclear Magnetic Resonance |
| PyBop | (Benzotriazol-1-yloxy)tripyrrolidinophosphonium hexafluorophosphate |
| q | Quartett |
| rt | Retention Time |
| s | Singlett |
| t | Triplett |
| THF | Tetrahydrofurane |
| TLC | Thin Layer Chromatography |

The compounds of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described above.

The amine-free bases corresponding to the isolated salts can be generated by neutralization with a suitable base, such as aqueous sodium hydrogen carbonate, sodium carbonate, sodium hydroxide and potassium hydroxide, and extraction of the liberated amine-free base into an organic solvent, followed by evaporation. The amine-free base, isolated in this manner, can be further converted into another pharmaceutically acceptable salt by dissolution in an organic solvent, followed by addition of the appropriate acid and subsequent evaporation, precipitation or crystallization. The amine-free base, isolated in this manner, can be further converted into another pharmaceutically acceptable salt by dissolution in an organic solvent, followed by addition of the appropriate acid and subsequent evaporation, precipitation or crystallization. The carboxylic free acids corresponding to the isolated salts can be generated by neutralization with a suitable acid, such as aqueous hydrochloric acid, sodium hydrogen sulfate, sodium dihydrogen phosphate, and extraction of the liberated carboxylic-free acid into an organic solvent, followed by evaporation. The carboxylic acid, isolated in this manner, can be further converted into another pharmaceutically acceptable salt by dissolution in an organic solvent, followed by addition of the appropriate base and subsequent evaporation, precipitation or crystallization.

An illustration of the preparation of compounds of the present invention is shown below. Unless otherwise indicated in the schemes, the variables have the same meaning as described above. The examples presented below are intended to illustrate particular embodiments of the invention. Suitable starting materials, building blocks and reagents employed in the synthesis as described below are commercially available from Sigma-Aldrich Chemie GmbH, Munich, Germany, from Acros Organics, Belgium or from Fisher Scientific GmbH, 58239 Schwerte, Germany, for example, or can be routinely prepared by procedures described in the literature, for example in "March's Advanced Organic Chemistry: Reactions, Mechanisms, and Structure", 5th Edition; John Wiley & Sons or Theophil Eicher, Siegfried Hauptmann "The Chemistry of Heterocycles; Structures, Reactions, Synthesis and Application", 2nd edition, Wiley-VCH 2003; Fieser et al. "Fiesers' Reagents for organic Synthesis" John Wiley & Sons 2000.

In formulas of general synthesis schemes depicted below

$E_L$ is halogen, OH, OC(O)alkyl, OC(O)aryl, O-aryl, O-pentafluorophenyl, O-sulfonylalkyl, O-sulfonylaryl, O-succinylimido, O-benzotriazolyl, nitro, azido, S-alkyl, $SO_2$alkyl, $SO_2$aryl, SC(O)alkyl, SC(O)aryl or cyano;

$E_N$-H is a group acting as a nucleophile; such as OH, SH, $NH_2$, $N(R^{14})H$, NH(CO)O-alkyl, NH(CO)O-aryl, $NH(SO)_2$aryl, $NH(SO)_2$alkyl, $CH_3$ or $CF_2H$;

$L_A$ is halogen, $NH_2$, $N(R^{10})H$, nitro, azido, CN, $CF_3$, COCl, COF, CHO, $CH_2OH$, COOH, $C(O)NHNH_2$, C(O)O-alkyl, C(O)O-aryl, C(O)O-hetaryl, SH, SMe, $SO_2Cl$, $SO_3H$, G-$NH_2$, G-$N(R^{10})H$, OH, O-alkyl, G-SH, G-OH, G-halogen, $B(OMe)_2$, $B(OH)_2$, $BF_3^-$, 4,4,5,5-tetramethyl-1,3,2-dioxaborolan-2-yl or ethinyl;

$L_B$ is halogen, $NH_2$, $N(R^{10})H$, COCl, COF, CHO, $CH_2OH$, COOH, $C(O)NHNH_2$, C(O)O-alkyl, C(O)O-aryl, C(O)O-hetaryl, SH, $SO_2Cl$, $SO_3H$, G-$NH_2$, G-$N(R^{10})H$, OH, G-SH, G-OH, G-halogen, $B(OH)_2$, $B(OMe)_2$, $BF_3^-$, 4,4,5,5-tetramethyl-1,3,2-dioxaborolan-2-yl or ethinyl;

$L_C$ is halogen, $NH_2$, $N(R^{10})H$, COCl, COF, CHO, $CH_2OH$, COOH, $C(O)NHNH_2$, C(O)O-alkyl, C(O)O-aryl, C(O)O-hetaryl, SH, $SO_2Cl$, $SO_3H$, G-$NH_2$, G-$N(R^{10})H$, OH, G-SH, G-OH, G-halogen, $B(OMe)_2$, $B(OH)_2$, $BF_3^-$, 4,4,5,5-tetramethyl-1,3,2-dioxaborolan-2-yl or ethinyl.

$R^{10}$, $R^{14}$ and G are as defined in the claims.

In preferred embodiments of the synthesis method

$E_L$ is halogen, OH, $OSO_2$alkyl or $OSO_2$aryl;

$E_N$-H is OH, SH, $NH_2$, or $CH_3$;

$L_A$ is halogen, nitro, azido, CN, $CF_3$, C(O)O-alkyl, C(O)O-aryl, C(O)O-hetaryl, SH, SMe, $SO_3H$, G-$NH_2$, G-$N(R^{10})$

H, OH, O-alkyl, G-SH, G-OH, G-halogen, $B(OH)_2$, $B(OMe)_2$, $BF_3^-$, 4,4,5,5-tetramethyl-1,3,2-dioxaborolan-2-yl or ethinyl;

$L_B$ is $NH_2$, COCl, COOH, $C(O)NHNH_2$, $SO_2Cl$, $B(OMe)_2$, $B(OH)_2$, $BF_3^-$, 4,4,5,5-tetramethyl-1,3,2-dioxaborolan-2-yl or ethinyl;

$L_C$ is $NH_2$, CHO, COCl, COOH, $C(O)NHNH_2$, $SO_2Cl$, $B(OH)_2$, $B(OMe)_2$, $BF_3^-$, 4,4,5,5-tetramethyl-1,3,2-dioxaborolan-2-yl or ethinyl;

In an even more preferred synthesis method

$E_L$ is chlorine or OH;

$E_N$-H is OH or $NH_2$;

$L_A$ is halogen, nitro, CN, C(O)O-alkyl, SH, $SO_3H$, $B(OMe)_2$, $B(OH)_2$, $BF_3$ or 4,4,5,5-tetramethyl-1,3,2-dioxaborolan-2-yl;

$L_B$ is $NH_2$, COCl, COOH or $SO_2Cl$;

$L_C$ is $NH_2$, CHO, COCl, COOH or $SO_2Cl$.

Pyrazole compounds of general formula IVa are known in the art and, to the extent not commercially available, are readily synthesized by standard procedures commonly employed in the art as illustrated in Scheme 1. In case where $R^1$ and $R^2$ are together carbonyl, pyrazole compounds of general formula IVa may be prepared by combining an acetylene compound of general formula VIIIa with a hydrazone of general formula IIa in acetic acid and in the presence of air to get *t*-butylatet pyrazole compound of general fomula IIIa. Subsequent de-butylation finally leads to a compound of general formula IVa as described in the literature (Kamitori et al., Heterocycles 1994, 38, 21-25; Kamitori et al. J. Heterocycl. Chem. 1993, 30, 389-391). Another method for preparing compounds of formula IVa involves treating an aldehyde of formula Va with trichloroacetylhydrazine to get trichloroacetylhydrazone VIa which is subsequently combined with 1,3 diketone compounds of general formula VIIa to give products of general formula IVa, as exemplified in the literature (Kaim et al., Synlett 2000, 353-355).

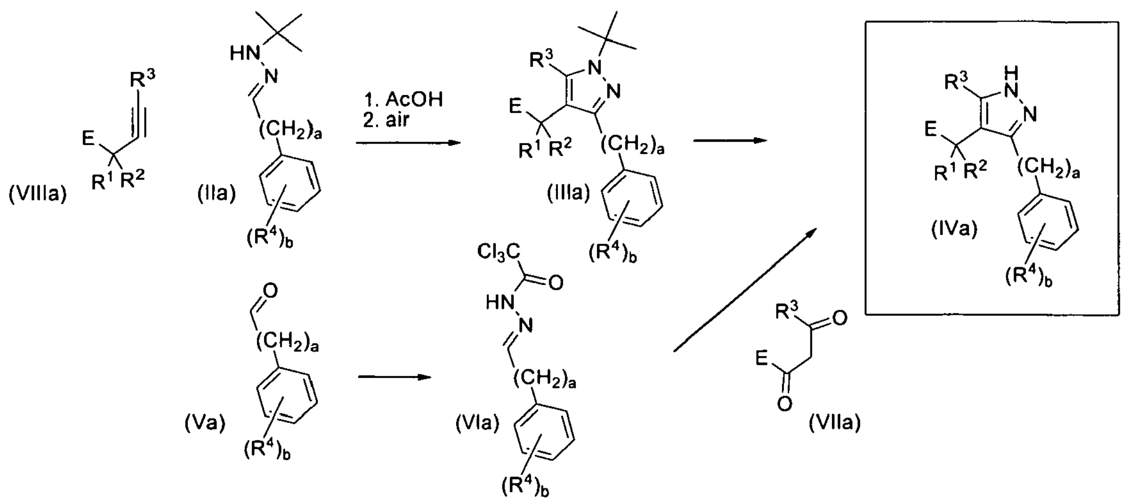


Scheme 1

In Scheme 1 shown above, the variants $R^1$ to $R^3$, a and b are as defined in the claims. The variant E comprises the variants $E_N$-H and $E_L$ having the meaning defined above.

Isoxazole compounds of the general formula IVb are known in the art and, to the extent not commercially available, are readily synthesized by standard procedures commonly employed in the art (Scheme 2), for example by combining acetylene compound of general formula VIIIa with an alpha-chlorooxime compound of formula IIb as described by Quilio et al., Gazz. Chim. Ital. 1937, 67, 589. Alternatively, if $R^1$ and $R^2$ are together carbonyl, compounds IVb are accessible by reacting alpha-chlorooxime of formula IIb with 1,3-dicarbonyl compound Vb as described, for example, by Maloney et al., J. Med. Chem. 2000, 43(16), 2971-2974 and by Doley et al, J. Chem. Soc. 1963, 5838-5845. Another method for preparing compounds of formula IVb is especially suitable if $R^3$ is alkylamino and involves combining an acetylene compound of formula VIIIa with nitrile oxides of general formula VIb as exemplified by Himbert et al., Liebigs Ann. Chem, 1990, 4, 403-407 and in Beyer et al., Justus Liebigs Ann Chem 1970, 45-54.

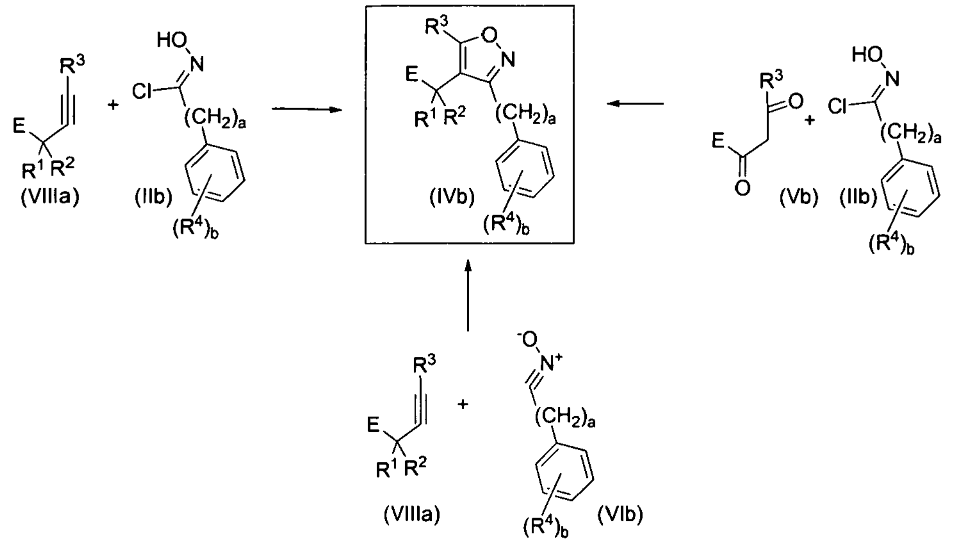
(VIIIa) (IIb) (IVb) (Vb) (IIb) (VIIIa) (VIb)

Scheme 2

Compounds of formula IVc (Scheme 3) are known in the art and, to the extent not commercially available, readily synthesized by standard procedures commonly employed in the art, for example by the procedures described by Y. Chen et al, Heterocycles 1995, 41, 175 and B. Chantegral et al., J. Org. Chem. 1984, 49, 4419-4424. Compounds of formula IVd are known in the art and, to the extent not commercially available, readily synthesized by standard procedures commonly employed in the art, for example by the procedures described by J. Piet et al., Bull. Soc. Chim. Belg., 1996, 105(1), 33-44 and by A. Cwiklicki et al., Arch. Pharm. 2004, 337(3), 156-163. Compounds of formula IVe are known in the art and, to the extent not commercially available, are readily synthesized by standard procedures commonly employed in the art, for example by the procedures described by G. Mitchell et al, J. Chem. Soc. Perkin Trans 1, 1987, 413-422 and Y. Piterskaya et al., Russ. J. Gen. Chem. 1996, 66(7), 1150-1157.

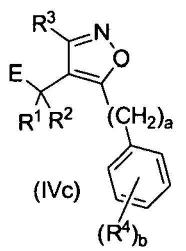
(IVc)

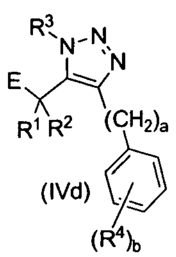
(IVd)

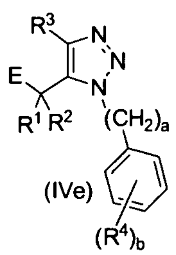
(IVe)

Scheme 3

The variants of compounds of formula IVa-IVe may optionally be further transformed into other variants of compounds of formula IVa-IVe by using derivatisation reactions known to the person skilled in the art, which are described in the literature, for example in: T. Eicher, S. Hauptmann "The Chemistry of Heterocycles; Structures, Reactions, Synthesis and Application", 2nd edition, Wiley-VCH 2003 (hereafter referred to as *Eicher);* "March's Advanced Organic Chemistry: Reactions, Mechanisms, and Structure", 5th Edition; John Wiley & Sons (hereafter referred to as *March);* Larock "Comprehensive Organic Transformations", VCH Publishers, New York, N.Y. 1989 (hereafter referred to as *Larock);* Fieser et al. "Fiesers' Reagents for organic Synthesis" John Wiley & Sons 2000 (hereafter referred to as *Fieser).*

Compounds of general formula IXa and IXb as depicted in Scheme 4 are known in the art and, to the extent not commercially available, readily synthesized by standard procedures commonly employed in the art, for example by the procedures described by the literature cited above. For specific preparations see examples 1-21.

Compounds of general formula IVa-IVe, IXa, IXb, XII, XIII and XIV may optionally be equipped with a temporarily attached protecting group remaining in the compound after its conversion. In later course of the synthesis sequence the protecting group is removed as taught in: T. Greene, P. Wuts "Protective groups in organic synthesis" 3rd ed. Wiley & Sons 1999 (hereafter referred to as *Greene).*

A typical synthesis for the compounds of general formula (I) involves a multistep synthesis sequence as depicted in Scheme 4 and in Scheme 5. Starting from heterocyclic intermediates of general formula IVa-IVe, two synthetic routes are envisaged for the first step resulting in an intermediate of general formula XII.

In step A1, a suitable compound of general formula IXa, equipped with an nucleophilic group chosen from $E_N$-H, is dissolved or suspended in a suitable solvent, preferably but not limited to dimethylformamide, tetrahydrofurane, benzene, toluene, dichloromethane or ether and, if advantageous, a suitable base is optionally added, including but not limited to sodium methoxide, potassium methoxide, potassium carbonate, sodium hexamethyldisilazane, lithium diisopropylamide, *n*-butyllithium or an amine base such as diisopropylethylamine, followed by the addition of a compound of general formula IVa-IVe, equipped with a suitable leaving group $E_L$. If a base is required, it is typically employed in a one to one ratio. However, as the skilled artisan would appreciate, a molar excess, usually in about 1-3 fold molar excess is acceptable. The reactants are typically combined at a temperature from about 0°C to about 100°C, preferably at room temperature and the resulting mixture is typically agitated for from about 5 minutes to about 48 hours. In case where $E_L$ is a poor leaving group (OH for example), it needs to be activated by adding activating reagents to the reaction mixture such as $MeSO_2Cl$, $CF_3(SO_2)_2O$ or Mitsunobu reagents diisopropyldiazenedicarboxylate and triphenylphosphine, for example, as shown in example 1.

Preferably, in step A1 the leaving group $E_L$ is chlorine or OH. Most preferably, the nucleophilic group $E_N$-H in compounds of general formula IXa is OH. In case where $E_N$-H is OH and $E_L$ is chlorine, the reactants of general formula IXa are dissolved or suspended in a suitable solvent, preferably tetrahydrofurane, DMF or methanol and typically 1-2 equivalent of a suitable base, such as sodium hydride or sodium methanolate are added. Subsequently a compound of general formula IVa-IVe is added and the resulting mixture is typically agitated for from about 5 minutes to about 48 hours. Reaction temperatures may range from -10°C to +60°C, typically from -10°C to +10°C. In case where $E_N$-H is OH and $E_L$ is also OH, the reactants of general formula IVa-IVe and IXa are dissolved or suspended in a suitable solvent, preferably benzene or toluene, and 1 to 2 equivalents triphenylphosphine and diisopropyldiazenedicarboxylate (DEAD) are added without addition of a base. The reactants are typically combined at a temperature from about 0°C to about 50°C, preferably at room temperature. The reaction times are typically 1 h to 12h. The solvents are usually removed by distillation at temperatures typically ranging from 10 to 50°C. The crude product is optionally purified by column chromatography and other purification methods known in the art.

In step A2, a suitable compound of general formula IXb, equipped with a suitable leaving group $E_L$, is combined with a compound of general formula IVa-IVe, equipped with an nucleophilic group chosen from $E_N$-H under similar conditions as applied in step A1.

Most preferably, in step A2 the leaving group $E_L$ in compound of general formula IXb is chlorine and the nucleophilic group $E_N$-H in compounds of general formula IVa-IVe is OH or $NH_2$. In such cases the reactants of general formula IVa-IVe are dissolved or suspended in a suitable solvent, preferably tetrahydrofurane, DMF or methanol and typically 1-2 equivalents of a suitable base, such as sodium hydride or sodium methanolate are added. Subsequently a compound of general formula IXb is added and the resulting mixture is typically agitated for about 1 h to 12h. Reaction temperatures may range from -10°C to +60°C, typically from -10°C to +10°C.

The starting materials and products of step A1 and step A2 may optionally be equipped with a protecting group remaining in the compound which needs to be removed in an additional step as taught in *Greene.*

In an optional step B the variants of compounds of formula XII may optionally be further transformed into other variants of compounds of formula XIV by using derivatisation reactions known to the person skilled in the art, which are described in *Greene, Eicher* and *Larock.* Such derivatisation reactions are thought to turn a functional group $L_A$ in formula XII into a functional group moiety $L_B$ in formula XIV, which is able to undergo a reaction with moiety $L_C$ in compound of formula XIII as depicted in step C (Scheme 5). General methods for functional group interconversions are described in *Larock.* In one example of step B, $L_A$ is a nitro group, which is reduced into an amino group $L_B$. In another example, $L_A$ is a nitro

group, which is converted into a bromine by reduction, subsequent diazotation and subsequent substitution by a bromide. In yet another example, $L_A$ is a SH group, which is interconverted by oxidation into a $SO_3H$ group and treated with $POCl_3$ to yielding a $SO_2Cl$ group $L_B$. In another example, $L_A$ is an COOalkyl ester group, which is saponified into a COOH group $L_B$. The starting materials and products of step B may optionally be equipped with a protecting group remaining in the compound which needs to be removed in an additional step as taught in *Greene.*

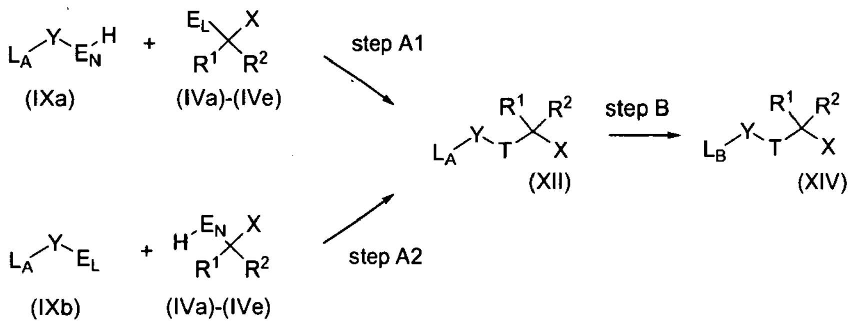

(IXa) (IVa)-(IVe) (XII) (XIV) (IXb) (IVa)-(IVe)

Scheme 4

In step C, a suitable compound of general formula XIII bearing a functional group $L_c$ is dissolved or suspended in a suitable solvent, preferably but not limited to dimethylformamide, acetonitrile, tetrahydrofurane, benzene, toluene, dichloromethane or ether and, if advantageous, a suitable base is optionally added, including but not limited to sodium methoxide, potassium methoxide, potassium carbonate, sodium hexamethyldisilazane, lithium diisopropylamide, *n*-butyllithium or an amine base such as diisopropylethylamine, triethylamine or N-methylmorpholine. A compound of general formula XIV, equipped with a functional group $L_B$ is added. It is contemplated that variants of compounds of general formula XIII and variants of compounds of general formula XIV are selected in a way that the synthetic combination of functional group $L_C$ with functional group $L_B$ results in a moiety L as defined in the description above. If a base is required, it is typically employed in a one to one ratio. However, as the skilled artisan would appreciate, a molar excess, usually in about 1-3 fold molar excess is acceptable. The reactants are typically combined at a temperature from about 0°C to about 100°C, preferably at room temperature and the resulting mixture is typically agitated for from about 5 minutes to about 48 hours. In case where $L_B$ and $L_C$ are groups of low reactivity that do not combine under the conditions described above, the use of an activating agent may be necessary. In one embodiment $L_B$ is COOH and $L_A$ is $NH_2$ or vice versa and coupling agents such as PyBOP; EDC or DCC might be required to ease the reaction. Alternatively the COOH group might be converted into an activated COCl group as described in *Larock.* In another embodiment where $L_B$ is ethinyl and $L_A$ is azido or vice versa, a catalyst such as $CuSO_4$/ascorbic acid might be required as described in K. B. Sharpless et al., Angew. Chem. 2002, 114, 2708-2711. In yet another embodiment where $L_B$ is $B(OH)_2$ and $L_A$ is halogen or vice versa, a Pd catalyst such as $PdCl_2(PPh_3)_2$ is required as described in A. Suzuki "palladium-Catalyzed Cross-Coupling Reactions of Organoboron Compounds" Chem. Rev. 1995, 95, 2457-2483.

The products of step C may optionally be equipped with a protecting group remaining in the compound which needs to be removed in an additional step as taught in *Greene.* The variants of compounds of general formula I may optionally be further transformed into other variants of compounds of general formula I by using derivatisation reactions known to the person skilled in the art, which are described in *Greene, Eicher* and *Larock.* Specific examples of such functional group interconversions can be found in the examples section.

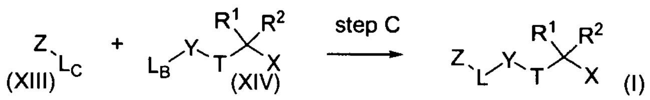

(XIII) (XIV) (I)

Scheme 5

Optionally, the variants of compounds of formula I may be further transformed into other variants of compounds

of formula I by using general methods for single- or multistep functional group interconversions as described in *Larock.* The skilled artisan will appreciate, that the synthesis steps described above may be optionally carried out in an alternative synthesis sequence, i.e. a compound of general formula XIII may be combined with a compound of general formula IX by the techniques mentioned above and the resulting intermediate is subsequently combined with a compound of general formula IVa-IVe to give products of general formula (I). Such a reversed order of synthetic steps is exemplified in example 19 in the examples section. The particular order of steps required to produce the compounds of formula I is dependent upon the particular compound being synthesized, the starting compound, and the relative lability of the substituted moieties.
Most preferred examples of the synthesis procedures are outlined in Scheme 6 where

$R^3$ is alkyl or dialkylamino;

$R^4$ is halogen;

b = 2;

$E_L$ is chlorine or OH;

$L_A$ is nitro;

$L_B$ is $NH_2$:

$L_C$ is COCl or $SO_2Cl$;

Y is $Y^1$, $Y^3$, or $Y^5$ ;

$R^8$ is OMe, $CH_3$ or $CF_3$;

c =1;

L is $-C(O)N(R^{10})-$, $-S(O)_mN(R^{10})-$, $-G-N(R^{10})-$;

$R^{10}$ is hydrogen or methyl;

m =2;

G is $-CH_2-$;

Z is phenyl-A-$R^9$ or pyridyl-A-$R^9$;

A is a bond or $-CH_2-$;

$R^9$ is COOH or COOMe.

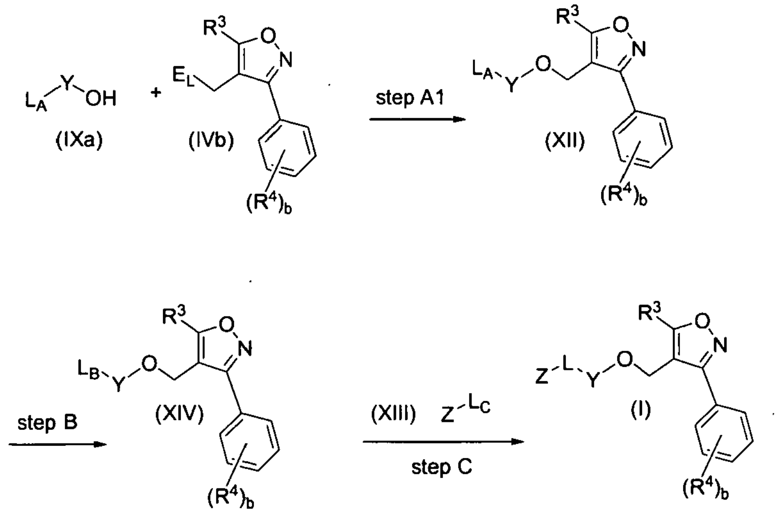


Scheme 6

In step A1 of most preferred embodiments of the invention, a suitable compound of general formula (IVb) is dissolved or suspended in a suitable solvent, preferably tetrahydrofurane, methanol, benzene or toluene. If $E_L$ is chlorine, a base is added, for example sodium hydride or sodium methanolate or the like. In case where $E_L$ is OH, 1 to 2 equivalents triphenylphosphine and diisopropyldiazenedicarboxylate (DEAD) are added instead of a base. A compound of general formula IXa is added and the reactants are typically combined at a temperature ranging from about 0°C to about 50°C, preferably at room temperature. The reaction times are typically 1 to 24h. The solvents are usually removed by distillation at temperatures typically ranging from 10°C to 50°C. The crude product of general formula XII is optionally purified by extraction methods and/or column chromatography and other purification methods known in the art.
In step B of most preferred embodiments of the invention, a nitro group in a compound of general formula XII is reduced to give an amino group being part of a compound of general formula XIV. Several reduction methods are suitable as described in *Larock,* for instance hydrogenation in presence of a hydrogenation catalyst such as palladium on charcoal or the like, or reduction using sodium borohydride in methanol and nickel chloride as catalyst. A reduction using zinc may be carried out as follows: a suitable compound of general formula XII is dissolved or suspended together with zinc powder in a suitable solvent, preferably an alcohol such as methanol and an acid, preferably acetic acid is added. The reactants are typically combined at a temperature from about 0°C to about 50°C, preferably at room temperature, until sufficient conversion is detected by methods known in the art, such as TLC or HPLC. The reaction times typically range from 1 to 24h. Optionally, solid byproducts may then be removed by filtration. Volatiles are removed by methods known in the art, such as distillation, for example. The crude product of general formula XIV is optionally purified by extraction methods and/or column chromatography and other purification methods known in the art.
In step C of most preferred embodiments of the invention, the amino group in the compound of general formula XIV is combined with a sulfonic acid or carboxylic acid moiety or the corresponding sulfonyl chloride or carboxylic acid chloride moiety in compounds of general formula XIII. Several methods are suitable for this transformation as described in *Larock,* including activating a carboxylic acid group of compound of formula XIII using DCC, EDC, PyBroP or another suitable coupling agent known in the art and combining the activated acid with the amine function of compound of general formula XIV. In the case where carboxylic acid chlorides or sulfonic acid chlorides of formula XIII are employed, a typical procedure is as follows:

> Amine compound of formula XIV is dissolved or suspended in an appropriate solvent such as dichloromethane, acetonitrile or tetrahydrofurane or the like, typically dichloromethane or acetonitrile, together with a base, preferably an amine base, such as DIPEA, triethylamine or N-methylmorpholine, and the acid chloride compound of formula XIII. The reactants are typically combined at a temperature from about 0°C to about 50°C, preferably at room temperature, until sufficient conversion is detected by methods known in the art, such as TLC or HPLC. The reaction times typically range from 1 to 24h. The crude product of general formula I is optionally purified by extraction methods and/or column chromatography and other purification methods known in the art.

As mentioned above, compounds of general structure I may further be converted into other variants of the same general structure by single or multistep functional group interconversions such as described in *Larock.*

In most preferred embodiments, an amide or sulfonamide group L is N-alkylated by an alkyl halogen, preferably methyl iodide. In a typical procedure, the compound of generally formula I is dissolved or suspended in a suitable solvent, for example THF at a temperature ranging from -10 to + 30°C, typically at 0°C and a base, typically NaH is added and the mixture is optionally agitated until deprotonation has progressed sufficiently. An alkyl halogen is added and agitation is continued at a temperature ranging from -10°C to +80°C, typically room temperature until conversion into the alkylation product is sufficient. The volatiles are removed by methods known in the art and the crude product is optionally further purified by the generally accepted methods, such as extraction and/or chromatography.

In other most preferred embodiments, a carboxylic ester group in compound of formula I is saponified to give the corresponding carboxylic acid of formula I. In a typical procedure, compound of formula I is dissolved in an suitable solvent, such as an alcohol or an ether, preferably methanol, optionally containing 0-50% of water, together with 1 to 10 equivalents, preferably 1 to 2 equivalents of a base, preferably NaOH or LiOH. The reactants are typically combined at a temperature from about 0°C to about 80°C, preferably from room temperature to 60°C, until sufficient conversion is detected by methods known in the art, such as TLC or HPLC. The reaction times typically range from 1 to 24h. The crude product of general formula I is optionally purified by extraction methods and/or column chromatography and other purification methods known in the art.

Unless otherwise noted, all non-aqueous reactions were carried out either under an argon or nitrogen atmosphere using commercial dry solvents. Compounds were purified by flash column chromatography using silica gel 60 (230-400 mesh), or by reverse phase preparative HPLC using conditions as described in the synthesis procedure. LC/MS analysis was done using a Surveyor MSQ (Thermo Finnigan, USA) with APCI ionization, column: Waters XTerra MS C18 3.5μm 2.1x30 mm, injection volume 1 μl, flow rate 1.0ml/min, mobile phase: A - 0.1 % formic acid; B - acetonitrile.

Gradient table:

| time, min. | A% | B% |
|---|---|---|
| 0,0 | 100 | 0 |
| 0,1 | 100 | 0 |
| 3,0 | 5 | 95 |
| 3,8 | 5 | 95 |
| 3,9 | 100 | 0 |
| 6,5 | 100 | 0 |

Detection: diode array (PDA), 190-800 nm; masspec (APCI + or -). $^1$H NMR spectra were recorded on a *Varian MERCURY plus 400 MHz* spectrometer. Chemical shift values are given in ppm relative to tetramethylsilane (TMS), with the residual solvent proton resonance as internal standard. Melting points were taken on a Sanyo Gallenkamp melting point apparatus (MPD350.BM3.5).

Example 1

***N-{6-[3-(2,6-dichloro-phenyl)-5-isopropyl-isoxazol-4-ylmethoxy]-2-methyl-pyridin-3-yl}-terephthalamic acid***

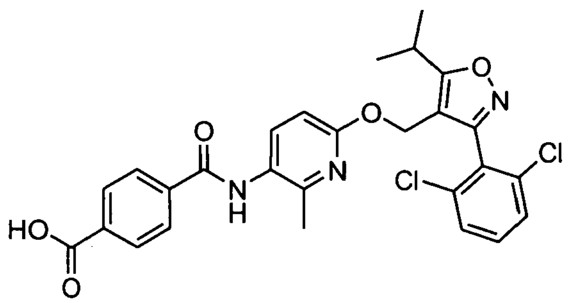

Step 1

Sodium metal (0.119g, 5.1mmol) was dissolved in methanol (6ml) at 0°C (ice bath), 6-chloro-2-methyl-3-nitro-pyridine (0.30g, 1.7mmol) was added and the mixture was stirred at 0°C until the complete consumption of 6-chloro-2-methyl-3-nitro-pyridine (4h). Acetic acid (0.306g, 5.1mmol) was added and the solution was concentrated under reduced pressure. The residue was dissolved in ethyl acetate (20 ml), washed with water (10 ml), dried over anhydrous $Na_2SO_4$, filtered and the solvent was removed under reduced pressure to give 0.28g (97%) 6-methoxy-2-methyl-3-nitropyridine as colourless powder.

Step 2

The product derived from step 1 (0.796g, 4.7mmol) was added to a of solution HBr (33%) in acetic acid (5ml), the mixture was stirred at 60°C for 2h and concentrated under reduced pressure. The residue was dissolved in diethyl ether (5ml), washed with 5% aqueous ammonia (3ml) and water (3ml) and the organic phase was evaporated to give 0.63g (87%) of 6-methyl-5-nitro-pyridin-2-ol as colourless powder.

Step 3

To a suspension of 6-methyl-5-nitro-pyridin-2-ol (0.625 g, 4.1 mmol), [3-(2,6-dichlorophenyl)-5-isopropyl-isoxazol-4-yl]-methanol (1.51g, 5.3mmol) (for preparation refer to:

P. Maloney et al. "Identification of a chemical tool for the orphan nuclear receptor FXR" J. Med. Chem. 2000, 43 (16), 2971-2974) and triphenylphosphine (1.91g, 7.4mmol) in benzene (12ml) was added dropwise diisopropyldiazenedicarboxylate (1.57g, 7.8mmol) and the reaction mixture was stirred at room temperature for 12h. The mixture was concentrated under reduced pressure and purified by reversed phase HPLC (column Reprosil-Pur C18-A9, 250×20 mm, gradient elution acetonitrile:water (2:1) - pure acetonitrile) to give 1.53 g (88%) of 6-[3-(2,6-dichlorophenyl)-5-isopropyl-isoxazol-4-ylmethoxy]-2-methyl-3-nitro-pyridine as colourless powder.

Step 4

Zinc powder (0.156g, 2.4mmol) was added to a vigorously stirred suspension of the product derived from step 3 (0.100g, 0.240mmol) in methanol (4ml), followed by the dropwise addition of acetic acid (0.065g, 1.1mmol). The reaction mixture was stirred for 2h at room temperature and passed through a 2cm layer of silica which was subsequently rinsed with methanol. The eluent was evaporated and the residue was dissolved in ethyl acetate (20ml) and filtered. The filtrate was washed with 10% aqueous $K_2CO_3$ (5ml), water (5ml), dried over anhydrous $Na_2SO_4$ and the solvent was removed under reduced pressure to give 0.088g (93%) of 6-[3-(2,6-dichloro-phenyl)-5-isopropyl-isoxazol-4-ylmethoxy]-2-methyl-pyridin-3-ylamine as yellowish oil.

Step 6

To a solution of 6-[3-(2,6-dichlorophenyl)-5-isopropyl-isoxazol-4-ylmethoxy]-2-methyl-pyridin-3-ylamine (0.137g, 0.350mmol) in $CH_2Cl_2$ (5ml) were added triethylamine (0.106g, 1.05mmol) and 4-chlorocarbonylbenzoic acid methyl ester (0.104g, 0.530mmol). The reaction mixture was stirred for 2h at room temperature, washed with 10% aqueous $K_2CO_3$ (5 ml), water (5 ml) and dried over anhydrous $Na_2SO_4$. The volatiles were removed under reduced pressure to afford a residue which was further purified by reversed phase HPLC (column Reprosil-Pur C18-A9, 250×20 mm, gradient elution acetonitrile:water (2:1) - pure acetonitrile) to give 0.126 g (65%) of N-{6-[3-(2,6-dichloro-phenyl)-5-isopropyl-isoxazol-4-ylmethoxy]-2-methyl-pyridin-3-yl}-terephthalamic acid methyl ester as colourless oil.

Step 7

To a solution of N-{6-[3-(2,6-dichloro-phenyl)-5-isopropyl-isoxazol-4-ylmethoxy]-2-methyl-pyridin-3-yl}-terephthalamic acid methyl ester (0.055g, 0.1mmol) in methanol (5ml) were added NaOH (0.076g, 1.9mmol) in water (0.15ml) and the reaction mixture was stirred at room temperature for 22 h. The solvent was evaporated and the residue was taken up with water (1.0ml). The resulting mixture was acidified to pH 6 with acetic acid, leading to the formation of a precipitate. The precipitate was filtered, washed with water (3ml) and dried on air to give N-{6-[3-(2,6-dichloro-phenyl)-5-isopropyl-isoxazol-4-ylmethoxy]-2-methyl-pyridin-3-yl}-terephthalamic acid as colourless powder. Yield 0.033g (61%). $^1$H-NMR (400 MHz, DMSO-$d_6$); δ (ppm) 1.37 (6H, d), 2.26 (3H, s), 3.40 (1H, br. s), 3.59 (1 H, sept), 5.13 (2H, s), 6.43 (1 H, d), 7.50-7.62 (4H, m), 7.96 (4H, q), 9.85 (1 H, s).

LC-MS: rt 3.41 min; m/z $[M+H]^+$ 539.8 (calculated: 539.1).

Example 2

***4-((6-((3-(2,6-Dichlorophenyl)-5-isopropylisoxazol-4-yl)methoxy)-2-methylpyridin-3-yl)(methyl)carbamoyl) benzoic acid***

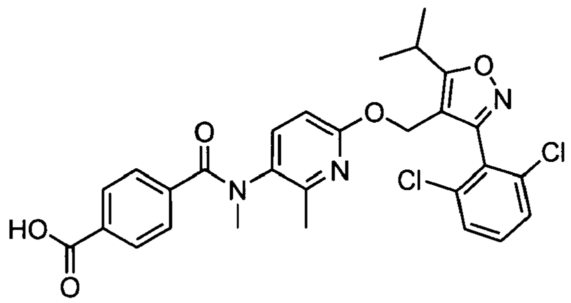

NaH (60% in mineral oil, 0.011g, 0.28mmol) was added to a 0°C solution of N-{6-[3-(2,6-dichloro-phenyl)-5-isopropyl-isoxazol-4-ylmethoxy]-2-methyl-pyridin-3-yl}-terephthalamic acid methyl ester from example 1, step 6 (0.126g, 0.23mmol) in anhydrous THF (5ml, freshly distilled) and the reaction mixture was stirred for 1 h at 0°C. Methyl iodide (0.039g, 0.28mmol) was added and stirring was continued at room temperature for 17 h. The solvent was removed under reduced pressure and the residue was dissolved in methanol (5ml), followed by the addition of NaOH (0.012g, 0.3mmol) and water (0.15ml). The resulting mixture was stirred at 50°C for 3h and the solvent removed under reduced pressure. Water (1ml) was added and the mixture was acidified to pH 6 with acetic acid leading to the formation of a precipitate. The solids were filtered, washed with water (3ml) and dried to give N-{6-[3-(2,6-dichlorophenyl)-5-isopropyl-isoxazol-4-ylmethoxy]-2-methyl-pyridin-3-yl}-N-methylterephthalamic acid as colourless powder. Yield 0.072g (56%).
$^1H$-NMR (400 MHz, DMSO-$d_6$); δ (ppm) 1.30 (6H, d), 2.08 (3H, s), 3.20 (3H, s), 3.25 (1 H, br. s), 3.44 (1 H, sept), 5.06 (2H, s), 6.30 (1 H, d), 7.22 (2H, d), 7.44-7.65 (4H, m), 7.70 (2H, d), 9.85 (1 H, s).
LC-MS: rt 3.38min; m/z $[M+H]^+$ 553.9 (calculated: 554.1).

Example 3

***4-{6-[3-(2,6-Dichloro-phenyl)-5-isopropyl-isoxazol-4-ylmethoxy]-2-methylpyridin-3-ylsulfamoyl}-benzoic acid***

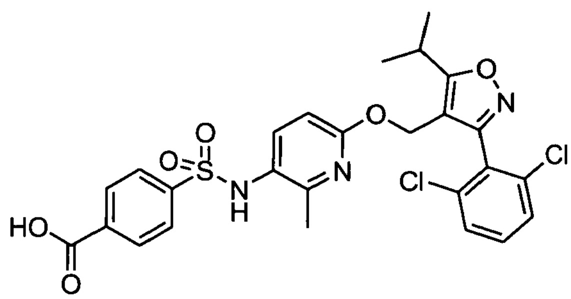

Step 1

To a solution of 6-[3-(2,6-dichloro-phenyl)-5-isopropyl-isoxazol-4-ylmethoxy]-2-methylpyridin-3-ylamine (from example 3 step 5, 0.130g, 0.330 mmol) in $CH_2Cl_2$ (5ml) was added 4-chlorosulfonyl-benzoic acid methyl ester (0.113g,

0.48mmol) and pyridine (0.026g, 0.330mmol). The reaction mixture was stirred at room temperature for 16h and concentrated under reduced pressure. The residue was further purified by preparative TLC on silica (eluent hexanes:ethyl acetate 3:1) to give 0.096g (43%) of 4-{6-[3-(2,6-dichloro-phenyl)-5-isopropyl-isoxazol-4-ylmethoxy]-2-methyl-pyridin-3-ylsulfamoyl}-benzoic acid methyl ester as colourless oil.

Step 2

4-{6-[3-(2,6-Dichloro-phenyl)-5-isopropyl-isoxazol-4-ylmethoxy]-2-methyl-pyridin-3-ylsulfamoyl}-benzoic acid methyl ester (0.059g, 0.10mmol) was dissolved in methanol (5ml) followed by the addition of NaOH (0.076g, 1.9mmol) and water (0.15ml) and the mixture was stirred at 50°C for 3h. The solvent was removed under reduced pressure and the residue dissolved in water (1.0 ml) and acidified to pH 6 with acetic acid. The precipitate was filtered, washed with water (3 ml) and dried to give 4-{6-[3-(2,6-dichloro-phenyl)-5-isopropyl-isoxazol-4-ylmethoxy]-2-methyl-pyridin-3-ylsulfamoyl}-benzoic acid as colourless powder. Yield 0.045g (78%).
$^1$H-NMR (400 MHz, DMSO-$d_6$); δ (ppm) 1.32 (6H, d), 1.95 (3H, s), 3.30 (1H, br. s), 3.50 (1 H, sept), 5.08 (2H, s), 6.31 (1 H, d), 7.12 (1 H, d), 7.45-7.58 (3H, m), 7.64 (2H, d), 8.20 (2H, d).
LC-MS: rt 3.45min; m/z $[M+H]^+$ 575.8 (calculated: 575.1).

Example 4

***4-(N-(6-((3-(2,6-dichlorophenyl)-5-isopropylisoxazol-4-yl)methoxy)-2-methylpyridin-3-yl)-N-methylsulfamoyl) benzoic acid***

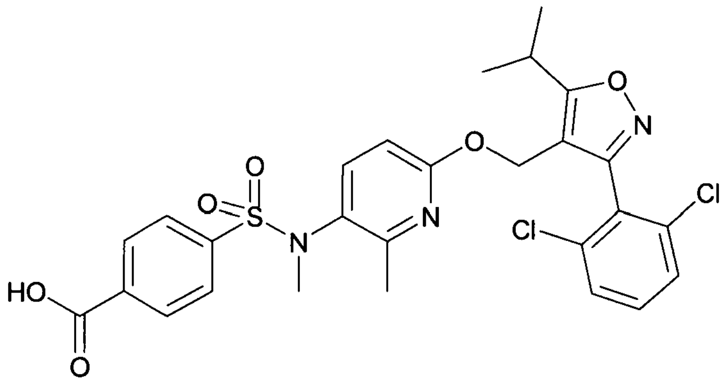

4-{6-[3-(2,6-Dichloro-phenyl)-5-isopropyl-isoxazol-4-ylmethoxy]-2-methyl-pyridin-3-ylsulfamoyl}-benzoic acid methyl ester from example 3 step 1 (0.096g, 0.16mmol) was dissolved in anhydrous THF (3ml) at 0°C, NaH (60% dispersion in mineral oil, 0.008g, 0.192mmol) was added and stirring was continued for 1h at 0°C. Methyl iodide (0.030g, 0.192mmol) was added and the reaction was stirred at room temperature for 17 h. The volatiles were removed under reduced pressure and the residue was dissolved in methanol (5ml). Solid NaOH (0.010g, 0.25mmol) and water (0.15ml) were added and the mixture was stirred at 50°C for 3 h. The solvent was removed under reduced pressure and the resulting residue was dissolved in water (1.0 ml) and the pH was adjusted to 6 by adding acetic acid. The resulting precipitate was filtered, washed with water (3ml) and dried to give 4-({6-[3-(2,6-dichloro-phenyl)-5-isopropyl-isoxazol-4-ylmethoxy]-2-methyl-pyridin-3-yl}-methyl-sulfamoyl)-benzoic acid as colourless powder. Yield 0.068 g (72%).
$^1$H-NMR (400 MHz, DMSO-$d_6$); δ (ppm) 1.35 (6H, d), 2.21 (3H, s), 3.08 (3H, s), 3.30 (1H, br. s), 3.54 (1 H, sept), 5.16 (2H, s), 6.31 (1 H, d), 6.90 (1 H, d), 7.47-7.59 (3H, m), 7.72 (2H, d), 8.12 (2H, d).
LC-MS: rt 3.65min; m/z $[M+H]^+$ 589.8 (calculated: 590.1).

Example 5

***N-{6-[3-(2,-6-dichloro-phenyl)-5-isopropyl-isoxazol-4-ylmethoxy]-2 trifluoromethyl-pyridin-3-yl}-terephthalamic acid methyl ester***

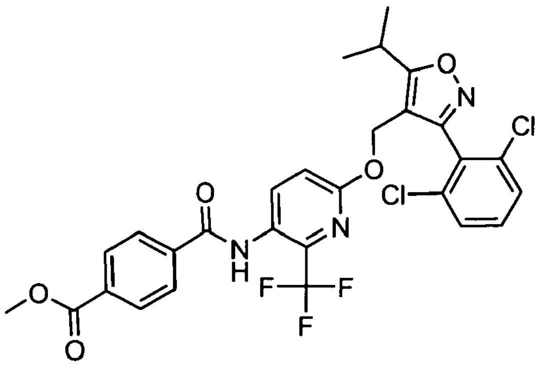

Step 1

2-Chloro-6-methoxy-3-nitro-pyridine (3g, 15.9mmol), CuI (3.63g, 36.7mmol), anhydrous KF (1.8g, 31mmol) and dry DMF (20ml) were placed in a reaction vessel followed by the addition of chloro-difluoro-acetic acid methyl ester (5.61g, 38.8mmol). The mixture was stirred at 127-130° C for 8 h under an atmosphere of nitrogen. The mixture was allowed cool to room temperature, poured into a mixture of aqueous $NH_4OH$ (25ml) and $NH_4Cl$ (40g), stirred for 0.5h and extracted with ethyl acetate. The extract was dried over anhydrous $Na_2SO_4$, filtered and the solvent was removed under reduced pressure. The residue was purified by column chromatography on silica (eluent hexanes : chloroform 10:9) to give 1.5g (42.5%) of 6-methoxy-2-trifluoromethyl-3-nitro-pyridine as colourless oil.

Step 2

6-methoxy-2-trifluoromethyl-3-nitro-pyridine (0.50g, 2.3mmol) was added to a solution of HBr in acetic acid (33% HBr w/w, 5ml) and the mixture was to stirred at 80°C for 16h. The reaction mixture was concentrated and the residue was purified by column chromatography on silica (eluent hexanes : ethyl acetate 10:1) to give 0.396g (83%) of 6-trifluoromethyl-5-nitro-pyridin-2-ol as yellowish oil.

Step 3

6-Trifluoromethyl-5-nitro-pyridin-2-ol (0.396g, 1.9mmol), [3-(2,6-dichloro-phenyl)-5-isopropyl-isoxazol-4-yl]-methanol (0.653g, 2.3mmol), triphenylphosphine (0.898g, 3.4mmol) and benzene (15ml) were placed in an reaction vessel followed by the dropwise addition of diisopropyldiazenedicarboxylate (0.74g, 3.7mmol). The reaction mixture was stirred at room temperature for 3h and the solvent was removed under reduced pressure affording a crude product which was further purified by column chromatography on silica (eluent hexanes : ethyl acetate 10:1) to give 0.66g (73%) of 6-[3-(2,6-dichloro-phenyl)-5-isopropyl-isoxazol-4-ylmethoxy]-2-trifluoromethyl-3-nitropyridine as light yellow powder.

Step 4

Zinc powder (0.21 g, 3.1mmol) was added to a vigorously stirred suspension of 6-[3-(2,6-dichloro-phenyl)-5-isopropyl-isoxazol-4-ylmethoxy]-2-trifluoromethyl-3-nitropyridine (0.150g, 0.310mmol) in methanol (4ml), followed by the dropwise addition of acetic acid (0.084g, 1.4mmol). The resulting mixture was stirred for 2h at 50°C and filtered through a 2cm layer of silica (eluent methanol). The eluent was concentrated under reduced pressure and the residue dissolved in ethyl acetate (20ml). This solution was filtered by paper filter, washed with 10% aqueous $K_2CO_3$ (5ml), water (5ml), dried over anhydrous $Na_2SO_4$ and evaporated to give 0.124g (yield 90%) of 6-[3-(2,6-dichloro-phenyl)-5-isopropyl-isoxazol-4-ylmethoxy]-2-trifluoromethyl-pyridin-3-ylamine as yellowish oil.

Step 5

6-[3-(2,6-Dichloro-phenyl)-5-isopropyl-isoxazol-4-ylmethoxy]-2-trifluoromethyl-pyridin-3-ylamine from step 4 (0.125g, 0.280mmol) was dissolved in $CH_2Cl_2$ (5ml) followed by the addition of triethylamine (0.085g, 0.84mmol) and 4-chlorocarbonyl-benzoic acid methyl ester (0.139g, 0.70mmol). The reaction mixture was stirred for 12h at room temperature, washed with 10% aqueous $K_2CO_3$ (5ml), water (5 ml), dried over anhydrous $Na_2SO_4$ and the volatiles were evaporated to afford a crude product which was further purified by column chromatography on silica (eluent hexanes: ethyl acetate 3:1) to give 0.053g (31%) of N-{6-[3-(2,6-dichloro-phenyl)-5-isopropyl-isoxazol-4-ylmethoxy]-2-trifluoromethyl-pyridin-3-yl}-terephthalamic acid methyl ester as colourless powder.
$^1$H-NMR (400 MHz, DMSO-$d_6$); δ (ppm) 1.42 (6H, d), 3.44 (1H, sept), 3.92 (3H, s), 5.20 (2H, s), 6.81 (1H, d), 7.24-7.40 (6H, m), 8.03 (1H, bs), 8.18 (1H, d), 8.43 (1H, d).
LC-MS: rt 3.66min; m/z $[M+H]^+$ 608.0 (calculated: 608.1).

Example 6

***N-{6-[3-(2,-6-dichloro-phenyl)-5-isopropyl-isoxazol-4-ylmethoxy]-2 trifluoromethyl-pyridin-3-yl}-terephthalamic acid***

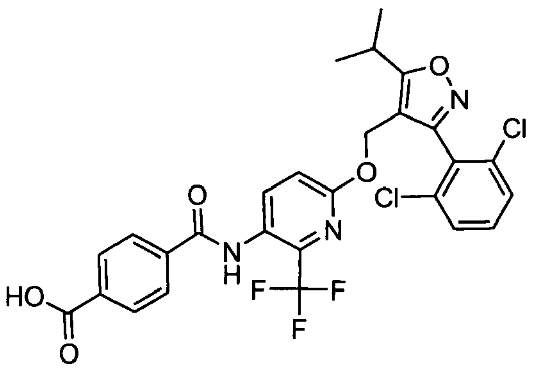

N-{6-[3-(2,6-dichloro-phenyl)-5-isopropyl-isoxazol-4-ylmethoxy]-2-trifluoromethylpyridin-3-yl}-terephthalamic acid methyl ester from example 5 (0.035g, 0.058mmol) was dissolved in methanol (3ml), a solution of NaOH (0.046g, 1.2mmol) in water (0.15ml) was added and the reaction mixture was stirred at room temperature for 50h. The solvent was removed under reduced pressure and the residue dissolved in water (1.5 ml). The resulting solution was acidified with acetic acid to pH6 leading to the formation of a precipitate which was filtered, washed with water (3 ml) and dried to give N-{6-[3-(2,6-dichloro-phenyl)-5-isopropyl-isoxazol-4-ylmethoxy]-2-trifluoromethylpyridin-3-yl}-terephthalamic acid as colourless powder. Yield 0.030g (87%).
$^1$H-NMR (400 MHz, DMSO-$d_6$); δ (ppm) 1.37 (6H, d), 3.40 (1 H, br. s), 3.53 (1 H, sept), 5.23 (2H, s), 6.90 (1 H, d), 7.48-7.61 (3H, m), 7.83 (1 H, d), 7.96 (4H, q), 10.09 (1 H, s).
LC-MS: rt 3.56min; m/z $[M+H]^+$ 594.1 (calculated: 594.1).

Example 7

***N-{6-[3-(2,6-dichloro-phenyl)-5-isopropyl-isoxazol-4-ylmethoxy]-2-trifluoromethyl-pyridin-3-yl}-N-methyl-terephthalamic acid***

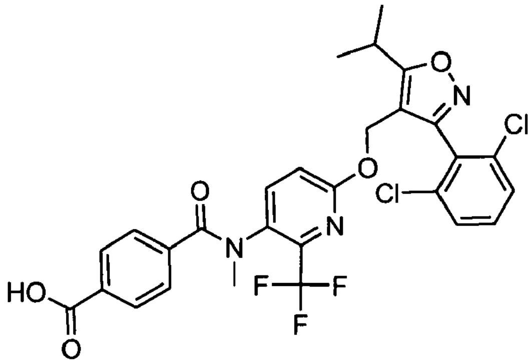

N-{6-[3-(2,6-dichloro-phenyl)-5-isopropyl-isoxazol-4-ylmethoxy]-2-trifluoromethylpyridin-3-yl}-terephthalamic acid methyl ester from example 5 (0.046g, 0.080mmol) was dissolved in anhydrous THF (6ml) at 0°C (ice bath), NaH (60% dispersion in mineral oil, 0.004 g, 0.10 mmol) was added and the reaction mixture was stirred for 30min at 0°C. Methyl iodide (0.0142g, 0.10mmol) was added and the reaction mixture was stirred at room temperature for 15h. The solvent was removed under reduced pressure, the resulting residue was dissolved in methanol (5ml), NaOH (0.066g, 1.7mmol) in water (0.2ml) was added and the mixture was stirred at room temperature for 8h. The solvent was removed under reduced pressure, dissolved in water (1.0ml) and acidified with acetic acid to pH6. The precipitate was filtered, washed with water (3ml) and dried to give N-{6-[3-(2,6-dichloro-phenyl)-5-isopropyl-isoxazol-4-ylmethoxy]-2-trifluoromethyl-pyridin-3-yl}-N-methyl-terephthalamic acid as colourless powder. Yield 0.036g (74%).
$^1$H-NMR (400 MHz, DMSO-$d_6$); δ (ppm) 1.30 (6H, d), 3.20 (4H, m), 3.43 (1H, m), 5.15 (2H, s), 6.82 (1 H, m), 7.05 (1H, m), 7.30-7.60 (4H, m), 7.65 (1 H, m), 7.94 (2H, d).
LC-MS: rt 3.52min; m/z $[M+H]^+$ 607.8 (calculated: 608.1).

Example 8

***4-{6-[3-(2,6-dichloro-phenyl)-5-isopropyl-isoxazol-4-ylmethoxy]-2-trifluoromethyl-pyridin-3-ylsulfamoyl}-benzoic acid***

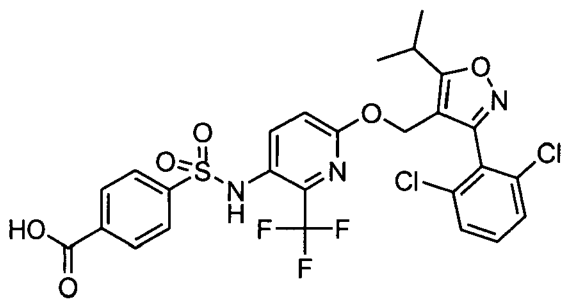

Step 1

6-[3-(2,6-dichloro-phenyl)-5-isopropyl-isoxazol-4-ylmethoxy]-2-trifluoromethyl-pyridin-3-ylamine from example 5, step 4 (0.132g, 0.30mmol) was dissolved in dry acetonitrile (5ml) followed by the addition of 4-chlorosulfonyl-benzoic acid methyl ester (0.070g, 0.33mmol) and pyridine (0.071g, 0.90mmol). The reaction mixture was stirred for 12h at 50°C, N-methylmorpholine (0.03g, 0.3mmol) was added and stirring was continued for 8h. The reaction mixture was concentrated under reduced pressure and purified by reversed phase HPLC (column Reprosil-Pur C18-A9, 250x20 mm, gradient elution acetonitrile:water (2:1) - pure acetonitrile) followed by column chromatography on silica (eluent hexanes:ethyl acetate 3:1) to give 0.060 g (31%) of 4-{6-[3-(2,6-dichlorophenyl)-5-isopropyl-isoxazol-4-ylmethoxy]-2-trifluoromethyl-pyridin-3-ylsulfamoyl}-benzoic acid methyl ester as colourless oil.

Step 2

4-{6-[3-(2,6-dichloro-phenyl)-5-isopropyl-isoxazol-4-ylmethoxy]-2-trifluoromethylpyridin-3-ylsulfamoyl}-benzoic acid methyl ester (0.060g, 0.10mmol) was dissolved in methanol (5ml), NaOH (0.076g, 1.9mmol) in water (0.15ml) was added and the reaction mixture was stirred at 50°C for 3h. The volatiles were evaporated and the residue was dissolved in water (1.0ml), acidified with acetic acid to pH 6 and extracted with ethyl acetate (2×10ml). The combined extracts were washed with water (3ml), dried over anhydrous $Na_2SO_4$, filtered and concentrated under reduced pressure to afford 4-{6-[3-(2,6-dichloro-phenyl)-5-isopropyl-isoxazol-4-ylmethoxy]-2-trifluoromethyl-pyridin-3-ylsulfamoyl}-benzoic acid as colourless oil. Yield 0.020g (32%).
$^1$H-NMR (400 MHz, DMSO-$d_6$); δ (ppm) 1.38 (6H, d), 3.48 (1H, sept), 5.23 (2H, s), 6.74 (1 H, d), 7.35-7.46 (3H, m), 7.62 (1 H, d), 7.77 (2H, d), 8.12 (2H, d).
LC-MS: rt 3.30min; m/z $[M+H]^+$ 630.2 (calculated: 629.0).

Example 9

***4-({6-[3-(2,6-Dichloro-phenyl)-5-isopropyl-isoxazol-4-ylmethoxy]-2-trifluoromethyl-pyridin-3-yl}-methyl-sulfamoyl)-benzoic** acid*

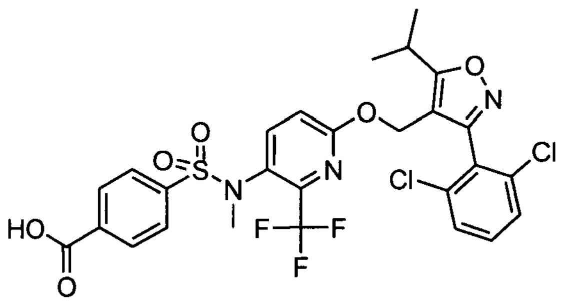

4-{6-[3-(2,6-dichloro-phenyl)-5-isopropyl-isoxazol-4-ylmethoxy]-2-trifluoromethylpyridin-3-ylsulfamoyl}-benzoic acid methyl ester from example 8, step 1 (0.035g, 0.050mmol) was dissolved in anhydrous THF (3ml) at 0°C (ice bath), NaH (60% dispersion in mineral oil, 0.0026g, 0.065mmol) was added and the reaction mixture was stirred for 1 h at 0°C. Methyl iodide (0.028g, 0.20mmol) was added and stirring was continued for 20h. The volatiles were evaporated and the residue was dissolved in methanol (5ml). NaOH (0.010g, 0.25 mmol) and water (0.15 ml) were added and to the resulting solution was stirred at 50°C for 3h. The solvent was evaporated, water (1.0ml) was added and acetic acid until pH 6. The volatiles were evaporated yielding a crude product which was purified by reversed phase HPLC (column Reprosil-Pur C18-A9, 250x20 mm, gradient elution acetonitrile: water (2:1) - pure acetonitrile) to give 4-({6-[3-(2,6-dichloro-phenyl)-5-isopropyl-isoxazol-4-ylmethoxy]-2-trifluoromethylpyridin-3-yl}-methyl-sulfamoyl)-benzoic acid as colourless oil. Yield 0.010 g (31 %). $^1$H-NMR (400 MHz, DMSO-$d_6$); δ (ppm) 1.42 (6H, d), 3.12 (3H, s), 3.53 (1H, sept), 5.32 (2H, s), 6.73 (1 H, d), 7.22 (1 H, d), 7.38-7.48 (3H, m), 7.86 (2H, d), 8.24 (2H, d).
LC-MS: rt 2.34min; m/z $[M+H]^+$ 644.2 (calculated: 644.1).

Example 10

***Synthesis of N-{6-[3-(2,6-dichloro-phenyl)-5-isopropyl-isoxazol-4-ylmethoxy]-2-methoxy-pyridin-3-yl}-terephthalamic acid***

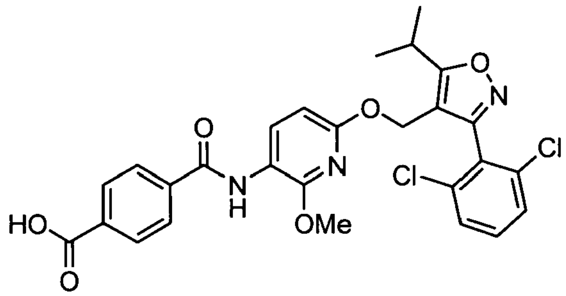

Step 1

2,6-Dichloro-3-nitro-pyridine (2.0g, 10mmol) was dissolved in dry THF (10ml) at 0°C followed by the addition of methanol (0.30g, 9.0mmol) and NaH (60% in mineral oil, 0.40g, 10mmol) in portions. The mixture was stirred for 1h and poured on ice (50g). The precipitating yellow crystals of 6-chloro-2-methoxy-3-nitro-pyridine were filtered and dried on air. Yield 1.8 g (92%).

Step 2

6-Chloro-2-methoxy-3-nitro-pyridine (0.30g, 1.0mmol) was dissolved in anhydrous THF (5 ml) at 0°C, NaH (60% in mineral oil, 0.050 g, 1.2 mmol) was added and the mixture was stirred at this temperature for 1 h, followed by the addition of [3-(2,6-dichloro-phenyl)-5-isopropyl-isoxazol-4-yl]-methanol (0.20g, 1.0mmol). The reaction was stirred at room temperature for 16h. The volatiles were removed under reduced pressure, water (10ml) was added and the mixture was extracted with ethyl acetate (2×10ml). The combined extracts were dried over anhydrous $Na_2SO_4$, filtered and the solvent was removed under reduced pressure. The crude product was subjected to reversed phase HPLC (column Reprosil-Pur C18-A9, 250×20 mm, gradient elution acetonitrile:water (2:1) - pure acetonitrile) to give 0.15g (33%) of 6-[3-(2,6-dichlorophenyl)-5-isopropyl-isoxazol-4-ylmethoxy]-2-methoxy-3-nitro-pyridine.

Step 3

The product synthesised in step 2 (0.23g, 0.50mmol) was dissolved in methanol (5 m), zinc powder (0.32 g, 5.0 mmol) was added followed by acetic acid (0.12g, 2.0mmol) and the reaction mixture was stirred for 30 min at 50°C. The mixture was filtered, washed with methanol (2×10ml) and the combined filtrates were evaporated. The residue was dissolved in $CH_2Cl_2$ (20ml), washed with 10% aqueous $K_2CO_3$ (10ml) and water (10ml), dried over anhydrous $Na_2SO_4$ and filtered. The solvent removed under reduced pressure yielding 0.20g (96%) of 6-[3-(2,6-dichloro-phenyl)-5-isopropyl-isoxazol-4-ylmethoxy]-2-methoxy-pyridin-3-ylamine.

Step 4

6-[3-(2,6-dichloro-phenyl)-5-isopropyl-isoxazol-4-ylmethoxy]-2-methoxy-pyridin-3-ylamine (0.150 g, 0.360 mmol) from step 3 was dissolved $CH_2Cl_2$ (5ml) followed by the addition of diisopropylethylamine (0.14g, 1.1mmol) and 4-chlorocarbonyl-benzoic acid methyl ester (0.090g, 0.44mmol). The mixture was allowed to react for 6h and concentrated under reduced pressure. Column chromatography of the residue on silica (eluent hexanes:ethyl acetate 1:2) gave 0.12g (57%) of N-{6-[3-(2,6-dichlorophenyl)-5-isopropyl-isoxazol-4-ylmethoxy]-2-methoxy-pyridin-3-yl}-terephthalamic acid methyl ester.

Step 5

The product derived from step 4 (0.030g, 0.05mmol) in methanol (2ml) was treated with NaOH (0.002g, 0.05mmol) and water (0.2ml) and the reaction mixture was stirred at 50°C for 2 h. The solvent was evaporated, water (10mll) was added and the mixture was acidified with acetic acid to pH 6, leading to formation of a precipitate which was filtered, washed with water (3ml) and dried on air to give N-{6-[3-(2,6-dichloro-phenyl)-5-isopropyl-isoxazol-4-ylmethoxy]-2-methoxy-pyridin-3-yl}-terephthalamic acid. Yield 0.020 g (67%).
$^1$H-NMR (400 MHz, DMSO-$d_6$); δ (ppm) 1.37 (6H, d), 3.49 (1H, sept), 3.80 (3H, s), 5.16 (2H, s), 6.16 (1 H, d), 7.41-7.62 (3H, m), 7.80 (1 H, d), 8.01 (4H, q), 9.42 (1 H, s).

LC-MS: rt 3.40min; m/z $[M+H]^+$ 555.8 (calculated: 555.1).

Example 11

***N-{6-[3-(2,6-dichloro-phenyl)-5-isopropyl-isoxazol-4-ylmethoxy]-2-methoxypyridin-3-yl}-N-methyl-terephthalamic acid***

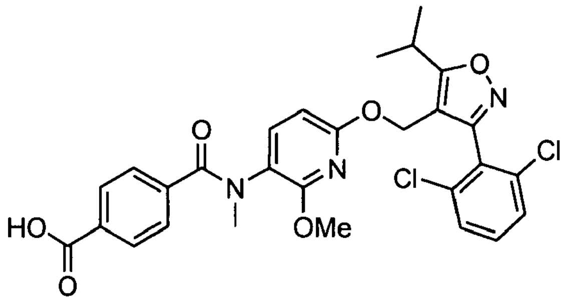

Step 1

N-{6-[3-(2,6-dichloro-phenyl)-5-isopropyl-isoxazol-4-ylmethoxy]-2-methoxy-pyridin-3-yl}-terephthalamic acid methyl ester from example 9, step 4 (0.070g, 0.12mmol) was dissolved in anhydrous THF (5ml) and cooled to 0°C. NaH (60% dispersion in mineral oil, 0.006g, 0.15mmol) was added and the reaction mixture was stirred for 30min. Methyl iodide (0.021 g, 0.15mmol) was added and stirring was continued at room temperature for 12h. The solvents were removed under reduced pressure, the residue was dissolved in water (10ml) and neutralized with acetic acid. The resulting precipitate was filtered and dried to give 0.050g (71%) N-{6-[3-(2,6-dichloro-phenyl)-5-isopropyl-isoxazol-4-ylmethoxy]-2-methoxy-pyridin-3-yl}-N-methyl-terephthalamic acid methyl ester.

Step 2

The product derived from step 1 (0.030g, 0.05mmol) in methanol (2ml) was treated with water (0.2ml) and NaOH (0.002g, 0.05mmol) and the mixture was stirred at 50°C for 2h. The volatiles were evaporated, dissolved in water (10ml) and neutralized with acidic acid. The resulting precipitate was filtered, washed with water and dried to give the title compound. Yield 0.025 g (85%).
$^1H$-NMR (400 MHz, DMSO-$d_6$); δ (ppm) 1.30 (6H, d), 3.10 (3H, s), 3.40 (1H, sept), 3.61 (3H, s), 5.10 (2H, s), 5.96 (1 H, d), 7.04 (2H, d), 7.39 (1 H, d), 7.41-7.49 (3H, m), 7.62 (2H, d).
LC-MS: rt 3.29min; m/z $[M+H]^+$ 569.9 (calculated: 570.1).

Example 12

***Synthesis of 4-{6-[3-(2,6-dichloro-phenyl)-5-isopropyl-isoxazol-4-ylmethoxy]-2-methoxy-pyridin-3-ylsulfamoyl}-benzoic acid***

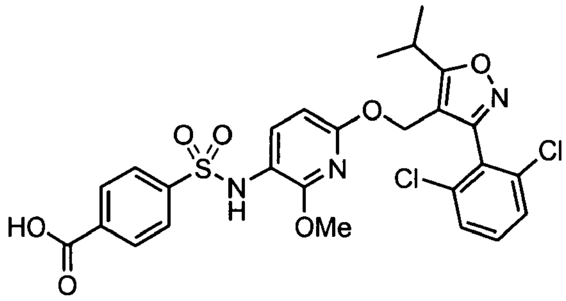

Step 1

6-[3-(2,6-Dichloro-phenyl)-5-isopropyl-isoxazol-4-ylmethoxy]-2-methoxy-pyridin-3-ylamine (example 12, step 2, 0.190g, 0.46mmol) in $CH_2Cl_2$ (5ml) was treated with 4-chlorosulfonyl-benzoic acid methyl ester (0.130g, 0.55mmol) and diisopropylethylamine (0.12g, 0.92mmol). The reaction mixture was stirred for 10h and concentrated in vacuo. The crude product was purified by column chromatography on silica (eluent hexanes:ethyl acetate 1:2) to give 0.070g (25%) of 4-{6-[3-(2,6-dichlorophenyl)-5-isopropyl-isoxazol-4-ylmethoxy]-2-methoxy-pyridin-3-ylsulfamoyl}-benzoic acid methyl ester.

Step 2

The product of step 1 (0.030g, 0.05mmol) in methanol (2ml) was treated with NaOH (0.002g, 0.05mmol) and water (0.2ml) and the reaction mixture was stirred at 50°C for 2h. The solvent was revaporated, dissolved in water (10ml) and acidified with acetic acid to pH6, leading to the formation of a precipitate which was filtered, washed with water (3ml) and dried yielding the title compound. Yield 0.023g (77%).
$^1$H-NMR (400 MHz, DMSO-$d_6$); δ (ppm) 1.32 (6H, d), 3.31 (3H, s), 3.43 (1H, sept), 5.11 (2H, s), 6.07 (1 H, d), 7.36 (1 H, d), 7.42-7.58 (3H, m), 7.73 (2H, d), 8.05 (2H, d).
LC-MS: rt 3.34min; m/z $[M+H]^+$ 591.9 (calculated: 591.1).

Example 13

***4-({6-[3-(2,6-dichloro-phenyl)-5-isopropyl-isoxazol-4-ylmethoxy]-2-methoxypyridin-3-yl}-methyl-sulfamoyl)-benzoic acid***

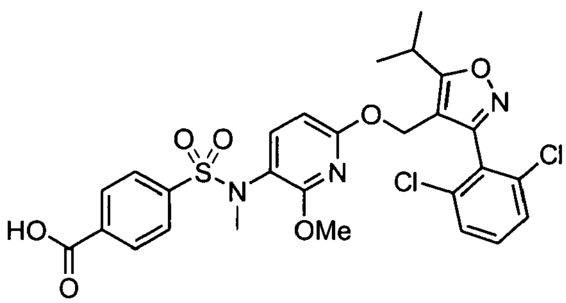

Step 1

The product derived from example 12, step 1 (4-{6-[3-(2,6-dichloro-phenyl)-5-isopropyl-isoxazol-4-ylmethoxy]-2-methoxy-pyridin-3-ylsulfamoyl}-benzoic acid methyl ester, 0.030g, 0.05mmol) in anhydrous THF (3ml) was cooled to

0°C, NaH (60% dispersion in mineral oil, 0.0024g, 0.06mmol) was added and the reaction mixture was stirred for 30min at 0°C. Methyl iodide (0.009g, 0.06mmol) was added and stirring was continued at room temperature for 12h. The volatiles were evaporated, methanol (5ml), NaOH (0.010g, 0.25mmol) and water (0.15ml) were added and the mixture was stirred at 50°C for 3h. The solvent was removed *in vacuo,* water (10ml) was added and the mixture was acidified with acetic acid to pH6. The resulting precipitate was filtered, washed with water (3ml) and dried to give 0.029g (98%) of 4-({6-[3-(2,6-dichloro-phenyl)-5-isopropyl-isoxazol-4-ylmethoxy]-2-methoxy-pyridin-3-yl}-methylsulfamoyl)-benzoic acid methyl ester.

Step 2

The product derived from step1 (0.030g, 0.05mmol) in methanol (2ml) was treated with NaOH (0.002g, 0.05mmol) and water (0.2ml) and heated at 50°C for 2h. The solvent was evaporated, redissolved in water (10ml) and acidified with acetic acid to pH6. The resulting precipitate was filtered, washed with water (3ml) and dried to give the title compound. Yield 0.025 g (83%).
$^1$H-NMR (400 MHz, DMSO-$d_6$); δ (ppm) 1.32 (6H, d), 3.06 (3H, s), 3.31 (3H, s), 3.43 (1H, sept), 5.15 (2H, s), 6.09 (1H, d), 7.38 (1H, d), 7.42-7.61 (3H, m), 7.68 (2H, d), 8.08 (2H, d).
LC-MS: rt 3.37min; m/z $[M+H]^+$ 606.2 (calculated: 606.1).

Example 14

***N-{6-[3-(2,6-dichloro-phenyl)-5-isopropyl-isoxazol-4-ylmethoxy]-4-methylpyridin-3-yl}-terephthalamic acid***

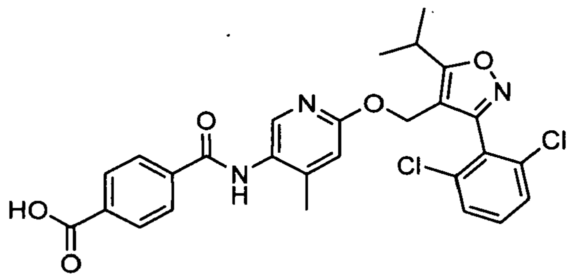

Step 1

Sodium metal (0.106g, 4.6mmol) was dissolved in methanol (5ml) at 0°C, 2-chloro-4-methyl-5-nitro-pyridine (0.2g, 1.0mmol) was added and the reaction mixture stirred at 0°C for 1 h and at room temperature for 1 h. The solvent was removed in vacuo, water (5ml) was added, the crystalline precipitate was filtered, washed with water and dried to give 0.13g (68%) of 2-methoxy-4-methyl-5-nitro-pyridine.

Step 2

The product of step 1 (0.13 g, 0.77 mmol) was dissolved in a 0°C solution of HBr in acetic acid (33% w/w, 5ml) and then stirred at 60°C for 2 h, cooled to room temperature and poured into diethyl ether (10ml). The crystalline precipitate that was filtered, washed with ether and dried 0.155 g (86%) of 4-methyl-5-nitro-pyridin-2-ol.

Step 3

The product derived from step 2 (0.15g, 1.0mmol), [3-(2,6-dichloro-phenyl)-5-isopropyl-isoxazol-4-yl]-methanol (0.28g, 1.0mmol) and triphenylphosphine (0.29g, 1.1mmol) in benzene (10 ml) were treated dropwise with diisopropyldiazenedicarboxylate (0.024g, 1.2mmol) and the reaction mixture was stirred at room temperature for 12h. The volatiles were evaporated and the crude material was purified by column chromatography on silica (eluent hexanes:ethyl acetate 3:1) to give 0.15g (56%) of 2-[3-(2,6-dichloro-phenyl)-5-isopropyl-isoxazol-4-ylmethoxy]-4-methyl-5-nitro-pyridine.

Step 4

The product derived from step 3 (0.15g, 0.35mmol) in methanol (5ml) was treated with zinc powder (0.23g, 3.5mmol) and acetic acid (0.10g, 1.7mmol) and the reaction mixture was stirred at 50°C for 30min. The solids were filtered and washed with methanol (2×10ml) and the filtrate was evaporated. The residue was dissolved in $CH_2Cl_2$ (20ml), washed with 10% aqueous $K_2CO_3$ (10ml) and water (10ml), dried over anhydrous $Na_2SO_4$ and concentrated to afford 0.147g (98%) of 6-[3-(2,6-dichlorophenyl)-5-isopropyl-isoxazol-4-ylmethoxy]-4-methyl-pyridin-3-ylamine.

Step 5

The product derived from step 4 (0.150g, 0.380mmol) was dissolved in $CH_2Cl_2$ (5ml), diisopropylethylamine (0.073g, 0.57mmol) and 4-chlorocarbonyl-benzoic acid methyl ester (0.090g, 0.45mmol) were added and the reaction mixture was stirred for 6h at room temperature. The volatiles were evaporated and the crude material was purified by column chromatography on silica (eluent hexanes:ethyl acetate 1:2) to give 0.085g (40%) of N-{6-[3-(2,6-dichloro-phenyl)-5-isopropyl-isoxazol-4-ylmethoxy]-4-methylpyridin-3-yl}-terephthalamic acid methyl ester.

Step 6

The product derived from the previous step (0.040g, 0.072mmol) was dissolved in methanol (2ml), treated with NaOH (0.003g, 0.072mmol) and water (0.2ml) and the reaction mixture was stirred at 50°C for 2h. The solvent was removed under reduced pressure and the residue redissolved in water (10ml) and acidified with acetic acid to pH6. The resulting precipitate was filtered, washed with water (3ml) and dried to give N-{6-[3-(2,6-dichloro-phenyl)-5-isopropyl-isoxazol-4-ylmethoxy]-4-methyl-pyridin-3-yl}-terephthalamic acid. Yield 0.030 g (75%).
$^1$H-NMR (400 MHz, DMSO-$d_6$); δ (ppm) 1.37 (6H, d), 2.15 (3H, s), 3.53 (1H, sept), 5.07 (2H, s), 6.53 (1 H, s), 7.50-7.65 (3H, m), 7.89 (1 H, s), 8.04 (4H, s), 9.92 (1 H, s).
LC-MS: rt 3.14min; m/z $[M+H]^+$ 540.3 (calculated: 539.1).

Example 15

*3-(6-((3-(2,6-dichlorophenyl)-5-methylisoxazol-4-yl)methoxy)pyridin-3-ylcarbamoyl)benzoic acid*

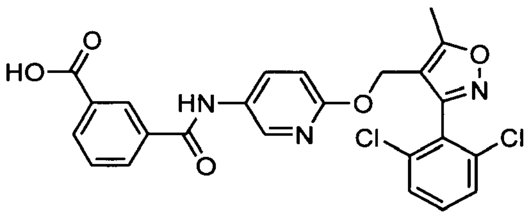

Example 15 was prepared by a procedure similar as employed for preparation of example 1 using the appropriate starting materials.
$^1$H-NMR (400 MHz, MeOH-$d_4$); δ (ppm) 2.57 (3H, s), 5.08 (2H, s), 6.53 (1H, d), 7.33-7.45 (3H, m), 7.54 (1 H, t), 7.84 (1 H, d), 8.05 (1 H, d), 8.14 (1 H, d), 8.26 (1 H, s), 8.50 (1H,s).
LC-MS: rt 3.38min; m/z $[M+H]^+$ 497.8 (calculated: 498.1).

Example 16

***4-(6-((3-(2,6-dichlorophenyl)-5-methylisoxazol-4-yl)methoxy)pyridin-3-ylcarbamoyl)benzoic acid***

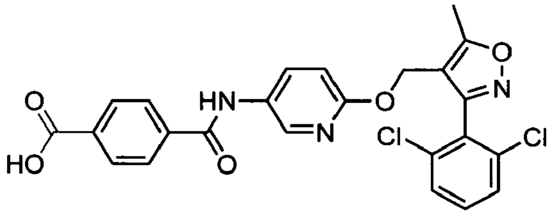

Example 16 was prepared by a procedure similar as employed for preparation of example 1 using the appropriate starting materials.
$^1$H-NMR (400 MHz, MeOH-$d_4$); δ (ppm) 2.56 (3H, s), 5.08 (2H, s), 6.53 (1H, d), 7.32-7.46 (3H, m), 7.84 (1 H, t), 7.93 (2H, d), 8.07 (2H, d), 8.25 (1 H, s).
LC-MS: rt 3.43min; m/z $[M+H]^+$ 497.8 (calculated: 498.1).

Example 17

***3-(N-(6-((3-(2,6-dichlorophenyl)-5-methylisoxazol-4-yl)methoxy)pyridin-3-yl)sulfamoyl)benzoic acid***

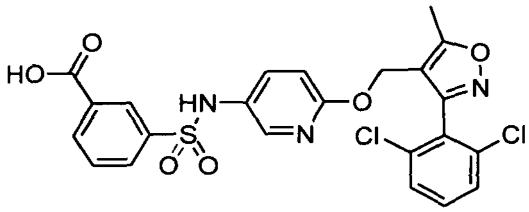

Example 17 was prepared by a procedure similar as employed for preparation of example 3 using the appropriate starting materials.
$^1$H-NMR (400 MHz, MeOH-$d_4$); δ (ppm) 2.72 (3H, s), 5.22 (2H, s), 6.62 (1H, d), 7.50 (1 H, d), 7.55-7.65 (3H, m), 7.76 (2H, t), 8.05 (1 H, d), 8.38 (1 H, d), 8.50 (1 H, s).
LC-MS: rt 3.52min; m/z [M+H]+ 533.8 (calculated: 534.0).

Example 18

***4-(N-(6-((3-(2,6-dichlorophenyl)-5-methylisoxazol-4-yl)methoxy)pyridin-3-yl)sulfamoyl)benzoic acid***

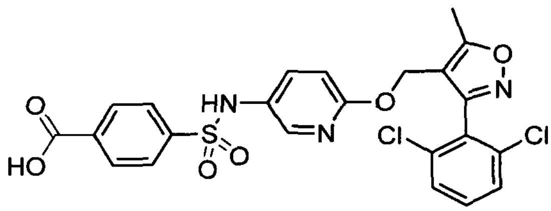

Example 18 was prepared by a procedure similar as employed for preparation of example 3 using the appropriate starting materials.
$^1$H-NMR (400 MHz, MeOH-$d_4$); δ (ppm) 2.49 (3H, s), 5.00 (2H, s), 6.40 (1 H, d), 7.25 (1 H, d), 7.32-7.39 (3H, m), 7.52 (1 H, s), 7.64 (2H, d), 8.02 (2H, d).
LC-MS: rt 3.42min; *m/z* $[M+H]^+$ 533.8 (calculated: 534.0).

Example 19

***N-{5-[3-(2,6-Dichloro-phenyl)-5-isopropyl-isoxazol-4-ylmethoxy]-3-methylpyridin-2-yl}-terephthalamic acid***

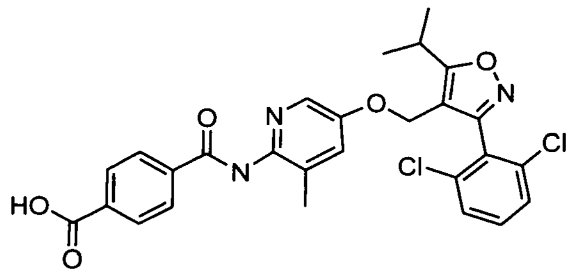

Step 1

3-Methyl-pyridin-2-ylamine (5.0 g, 46.2 mmol) was dissolved in concentrated sulfuric acid (24ml), the mixture was chilled to 0°C and a mixture of fuming nitric acid (d=1.5, 3.5ml) and concentrated sulfuric acid (3.5ml) was added dropwise to the reaction mixture while temperature was kept below 20°C. The stirred mixture was allowed to warm to 20°C and transferred in portions of 3-5ml into a second flask which was heated to 35-40°C (the temperature was not allowed to rise over 40°C - monitoring carefully the temperature after every addition of a new portion). The resulting reaction mixture was subsequently stirred for additional 30min at 50°C, cooled to ambient temperature and neutralized with concentrated aqueous ammonia. This led to the formation of a precipitated which was filtered, washed with water and aqueous DMFA (50%, 6ml), and recrystallized from DMFA yielding 3-methyl-5-nitro-pyridin-2-ylamine (2.52g, 35%).

Step 2

The product of step 1 (0.96g, 6.27mmol), 4-chlorocarbonyl-benzoic acid methyl ester (2.49g, 12.5mmol) and triethylamine (1.27g, 12.5mmol) were dissolved in anhydrous dichloromethane (20ml) and the mixture was stirred for 96h at room temperature. The solvent was removed under reduced pressure and the resulting residue was further purified by column chromatography on silica gel (eluent chloroform, then chloroform-methanol 95:5) yielding the bis acylation product methyl 4-[[4-(methoxycarbonyl)benzoyl](3-methyl-5-nitro-2-pyridinyl)amino]carbonylbenzenecarboxylate (1.1g, 37%).

Step3

The product derived from step 2 (0.5g, 2.1mmol) was dissolved in dry methanol (20ml) and hydrogenated at 4bar $H_2$ pressure and Raney nickel as catalyst (5% w/w). The crude product was purified by column chromatography on silica using chloroform-methanol (40:1) as eluent to give 0.189 g (20%) of N-(5-amino-3-methyl-pyridin-2-yl)-terephthalamic acid methyl ester.

Step 4

The product derived from the previous step (0.189 g, 0.66 mmol) was suspended in 50% aqueous sulfuric acid (2.3 g) and cooled to 0°C. A solution of sodium nitrite (0.047g, 0.68mmol) in water (0.5ml) was added dropwise to the resulting mixture keeping the temperature at 3-5°C and stirred for additional 30min at 3°C and for 2h at 50°C. After cooling to ambient temperature a precipitate was formed which was filtered, dried, washed with dichloromethane and dried to give of N-(5-hydroxy-3-methyl-pyridin-2-yl)-terephthalamic acid methyl ester (0.13g, 58%).

Step 5

A mixture of N-(5-hydroxy-3-methyl-pyridin-2-yl)-terephthalamic acid methyl ester from step 4 (0.135g, 0.47mmol), [3-(2,6-dichloro-phenyl)-5-isopropyl-isoxazol-4-yl]-methanol (0.124g, 0.43mmol) and triphenylphosphine

(0.203g, 0.77mmol) in benzene (6 ml) was treated dropwise with diisopropyldiazenedicarboxylate (0.165 g, 0.82 mmol) and the reaction mixture was stirred at room temperature for 3h. The solvent was removed under reduced pressure and the crude product was purified by reversed phase HPLC (column Reprosil-Pur C18-A9, 250×20 mm, gradient elution acetonitrile:water (2:1) - acetonitrile) to give of N-{5-[3-(2,6-dichloro-phenyl)-5-isopropyl-isoxazol-4-ylmethoxy]-3-methyl-pyridin-2-yl}-terephthalamic acid methyl ester as yellow oil (0.025g, 10%).

Step 6

A solution of the product from step 5 (0.025g, 0.045mmol) in dioxane (1.5ml) was treated with $LiOH \cdot H_2O$ (0.004g, 0.09mmol) in water (0.1ml) and the reaction mixture was stirred at ambient temperature for 3 days. The mixture was treated with acetic acid (until pH6), the solvent was evaporated followed by the addition of water (1ml). The resulting precipitate was filtered, washed with water (2×1ml) and dried to give N-{5-[3-(2,6-dichloro-phenyl)-5-isopropyl-isoxazol-4-ylmethoxy]-3-methyl-pyridin-2-yl}-terephthalamic acid as colourless powder, m.p. 218°C. Yield 0.016 g (66%).
$^1H$-NMR (400 MHz, $CHCl_3$); δ (ppm) 1.44 (6H, d), 2.30 (3H, s), 3.32 (1H, sept), 4.81 (2H, s), 7.15 (1H, s), 7.30-7.45 (4H, m), 7.77 (1H, br. s), 8.18 (4H, q), 10.40 (1H, br. s).
LC-MS: rt 2.02min; m/z $[M+H]^+$ 540.0 (calculated: 539.1).

Example 20

***N-{6-[3-(2,6-Dichloro-phenyl)-5-isopropyl-isoxazol-4-ylmethoxy]-2-trifluoromethyl-pyridin-3-yl}-isophthalamic acid methyl ester***

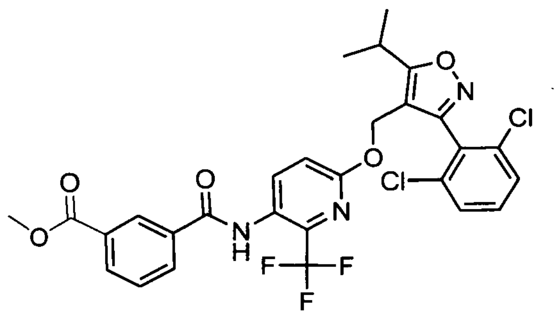

A solution of 6-[3-(2,6-dichloro-phenyl)-5-isopropyl-isoxazol-4-ylmethoxy]-2-trifluoromethyl-pyridin-3-ylamine (0.128g, 0.290mmol) (synthesized as described for example 5, step 4) in $CH_2Cl_2$ (5ml) was treated with triethylamine (0.029g, 0.29mmol) and 3-chlorocarbonyl-benzoic acid methyl ester (0.063g, 0.32mmol). The reaction mixture was stirred for 16h at room temperature, washed with 10% aqueous $K_2CO_3$ (5ml) and water (5ml), dried (anhydrous $Na_2SO_4$) and evaporated. The crude product was purified by column chromatography on silica gel (eluent hexanes:ethyl acetate 3:1) to give N-{6-[3-(2,6-dichloro-phenyl)-5-isopropyl-isoxazol-4-ylmethoxy]-2-trifluoromethyl-pyridin-3-yl}-isophthalamic acid methyl ester (0.13g, 74%) as colourless oil.
$^1H$-NMR (400 MHz, $CHCl_3$); δ (ppm) 1.40 (6H, d), 3.45 (1H, sept), 3.96 (3H, s), 5.19 (2H, s), 6.80 (1H, d), 7.20-7.40 (3H, m), 7.60 (1H, t), 8.03 (1H, br. s), 8.05 (1H, d), 8.25 (1H, d), 8.41 (1H, d), 8.50 (1H, s).
LC-MS: rt 2.38min; m/z $[M+H]^+$ 608.3 (calculated: 608.1).

Example 21

***N-{6-[3-(2,6-Dichloro-phenyl)-5-isopropyl-isoxazol-4-ylmethoxy]-2-trifluoromethyl-pyridin-3-yl}-isophthalamic acid***

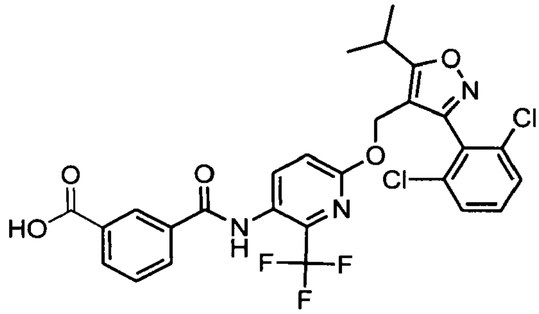

A solution of N-{6-[3-(2,6-dichloro-phenyl)-5-isopropyl-isoxazol-4-ylmethoxy]-2-trifluoromethyl-pyridin-3-yl}-isophthalamic acid methyl ester from example 19 (0.037g, 0.061 mmol) in methanol (5ml) was treated with NaOH (0.044g, 1.1 mmol) in water (0.15m), and the reaction mixture was stirred at room temperature for 50 h. The volatiles were evaporated, dissolved in water (1.0ml) and acidified with acetic acid to pH6, leading to the formation of a white precipitate which was filtered, washed with water (2×1ml) and dried to give N-{6-[3-(2,6-dichloro-phenyl)-5-isopropyl-isoxazol-4-ylmethoxy]-2-trifluoromethyl-pyridin-3-yl}-isophthalamic acid as a colourless powder. Yield 0.032 g (88%). $^1H$-NMR (400 MHz, $CHCl_3$); δ (ppm) 1.38 (6H, d), 3.40 (1 H, m), 5.15 (2H, s), 6.65 (1 H, m), 7.15-7.40 (4H, m), 7.50-8.50 (6H, m).
LC-MS: rt 2.22min; m/z $[M+H]^+$ 594.1 (calculated: 594.1). m.p. 153.8-154.8°C.

## *FRET activity assay*

Determination of a ligand mediated cofactor peptide interaction to quantify ligand binding to the nuclear receptor Farnesoid X Receptor (FXR) was performed as follows:

Preparation of human farnesoid X receptor (FXR) alpha ligand binding domain: The human FXRalpha ligand binding domain (LBD) was expressed in E. coli strain BL21(DE3) as an N-terminally glutathione-S-transferase (GST) tagged fusion protein. The DNA encoding the FXR ligand binding domain was cloned into vector pDEST15 (Invitrogen). Expression was under control of an IPTG inducible T7 promoter. The amino acid boundaries of the ligand binding domain were amino acids 187-472 of Database entry NM_005123 (RefSeq). Expression and purification of the FXR-LBD: An overnight preculture of a transformed E.coli strain was diluted 1:20 in LB-Ampicillin medium and grown at 30 °C to an optical density of OD600=0.4-0.6. Gene expression was then induced by addition of 0.5mM IPTG. Cells were incubated an additional 6h at 30°C, 180 rpm. Cells were collected by centrifugation (7000 x g, 7min, room temperature). Per liter of original cell culture, cells were resuspended in 10ml lysis buffer (50mM Glucose, 50mM Tris pH7.9, 1mM EDTA and 4mg/ml lysozyme) and left on ice for 30min. Cells were then subjected to sonication and cell debris removed via centrifugation (22000 x g, 30min, 4°C). Per 10ml of supernatant 0.5ml prewashed Glutathione 4B sepharose slurry (Qiagen) was added and the suspension kept slowly rotating for 1 h at 4°C. Glutathione 4B sepharose beads were pelleted by centrifugation (2000g, 15 sec, 4°C) and washed twice in wash buffer (25mM Tris, 50mM KCl, 4mM $MgCl_2$ and 1 M NaCl). The pellet was resuspended in 3ml elution buffer per liter of original culture (elution buffer: 20mM Tris, 60mM KCl, 5mM $MgCl_2$ and 80mM glutathione added immediately prior to use as powder). The suspension was left rotating for 15min at 4°C, the beads pelleted and eluted again with half the volume of elution buffer than the first time. The eluates were pooled and dialysed overnight in 20mM Hepes buffer (pH7.5) containing 60mM KCl, 5mM $MgCl_2$ as well as 1 mM dithiothreitol and 10% (v/v) glycerol. The protein was analysed by SDS-Page.

The method measures the ability of putative ligands to modulate the interaction between the purified bacterial expressed FXR ligand binding domain (LBD) and a synthetic biotinylated peptide based on residues 676-700 of SRC-1 (LCD2, 676-700). The sequence of the peptide used was B-CPSSHSSLTERHKILHRLLQEGSPS-COOH where the N-terminus was biotinylated (B). The ligand binding domain (LBD) of FXR was expressed as fusion protein with GST in BL-21 cells using the vector pDEST15. Cells were lysed by sonication, and the fusion proteins purified over glutathione sepharose (Pharmacia) according to the manufacturers instructions. For screening of compounds for their influence on the FXR-peptide interaction, the Perkin Elmer LANCE technology was applied. This method relies on the binding dependent energy transfer from a donor to an acceptor fluorophor attached to the binding partner of interest. For ease of handling and reduction of background from compound fluorescence LANCE technology makes use of generic fluorophore labels

and time resolved detection Assays were done in a final volume of 25μl in a 384well plate, in a Tris-based buffer (20mM Tris-HCl pH7,5; 60mM KCl, 5mM $MgCl_2$; 35ng/μl BSA), containing 20-60ng/well recombinantly expressed FXR-LBD fused to GST, 200-600nM N-terminally biotinylated peptide, representing SRC1 aminoacids 676-700, 200ng/well Streptavidin-xlAPC conjugate(Prozyme) and 6-10ng/well Eu W1024 - antiGST (Perkin Elmer). DMSO content of the samples was kept at 1%. After generation of the assay mix and diluting the potentially FXR modulating ligands, the assay was equilibrated for one hour in the dark at room temperature in FIA-plates black 384well (Greiner). The LANCE signal was detected by a Perkin Elmer VICTOR2VTM Multilabel Counter The results were visualized by plotting the ratio between the emitted light at 665nm and 615nm. A basal level of FXR-peptide formation is observed in the absence of added ligand. Ligands that promote the complex formation induce a concentration-dependent increase in time-resolved fluorescent signal. Compounds which bind equally well to both monomeric FXR and to the FXR-peptide complex would be expected to give no change in signal, whereas ligands which bind preferentially to the monomeric receptor would be expected to induce a concentration-dependent decrease in the observed signal.
To assess the inhibitory potential of the compounds, IC50-values were determined. The following compounds exemplify such activity with "+" meaning 1 μM < IC50 ≤ 10μM and "++" IC50 ≤ 1 μM

| Example No | FRET activity |
|---|---|
| Example 1 | ++ |
| Example 2 | ++ |
| Example 3 | ++ |
| Example 4 | ++ |
| Example 5 | + |
| Example 6 | ++ |
| Example 7 | ++ |
| Example 8 | + |
| Example 9 | ++ |
| Example 10 | ++ |
| Example 11 | + |
| Example 12 | ++ |
| Example 13 | ++ |
| Example 14 | ++ |
| Example 15 | + |
| Example 16 | + |
| Example 17 | + |
| Example 18 | + |
| Example 19 | ++ |
| Example 20 | + |
| Example 21 | ++ |

***Physicochemical & ADME assays***

Physicochemical and ADME parameters of examples of the present invention were determines and compared to those determined for FXR-modulating compounds A-D shown below which are state of the art and not part of the present invention.

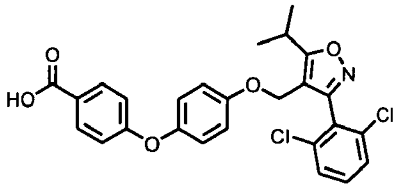

Compound A exemplified in WO03015771 and US7,034,046 B2

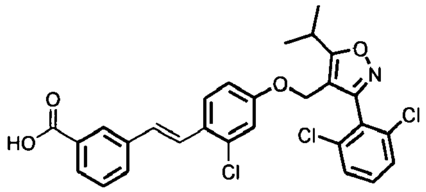

Compound B exemplified in WO0037077

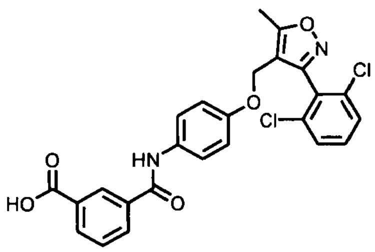

Compound C claimed in WO2004048349

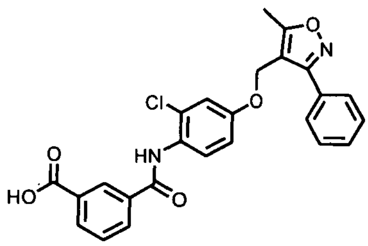

Compound D claimed in WO2004048349

***Aqueous solubility assay***

Aqueous solubility of compounds was determined as follows: Solubility of compounds was measured in PBS (pH7.4), 5% DMSO at 23°C. Nepheloskan Ascent (Thermo Electron Corporation) nephelometer was used for measurement of light scattering. Tested compounds were dissolved in DMSO to 10mM. Prior to measurement the compounds were further diluted with PBS in the wells to final compound concentrations of 100, 70, 50, 35, 25, 17, 12 and <10 μg/ml.

The plates were incubated at RT for 24 hours to reach equilibrium and the scattered light was measured. Assay validation: Aqueous solubility of acetylsalicylic acid was determined to validate the assay. It was found to be >100 μg/ml at the day of experiment, which corresponds to the reported literature value of at least 2,17 mg/ml (The Merck index, 10th edition).

The following compounds exemplify such solubility with "--" meaning solubility < 2μM, "+" meaning 2μM ≤ solubility ≤ 100μM and "++" meaning solubility >100μM.

| Compound | Aqueous solubility pH 7.4 |
|---|---|
| Example 4 | ++ |
| Example 6 | ++ |
| Example 7 | ++ |

(continued)

| Compound | Aqueous solubility pH 7.4 |
|---|---|
| Compound A | + |
| Compound B | -- |

***Determination of log D***

$LogD_{7.4}$ is determined using the octanol/buffer shake flask method for determining lipophilicity. Octanol is added to a solution of the test compound prepared in buffer at pH 7.4. Three different ratios of octanol to buffer are prepared. The system is mixed to allow distribution of the compound between the two phases. After separation, compound is quantified in the aqueous phase by LC-MS/MS and the following equation is used to calculate the logD :

$$LogD = \log [(C_{INI}-C_{FIN})/C_{FIN} \times (V_{aq}/V_{oct})].$$

Where: $C_{INI}$ = Concentration of compound in the initial aqueous solution; $C_{FIN}$ = Concentration of compound in final aqueous phase. $V_{aq}$ is the volume of water and $V_{oct}$ the Volume of octanol. Applying this protocol, log D of examples and reference compounds was determined.

***PAMPA permeability assay***

Artificial membrane permeability was determined as follows: Tested compounds were dissolved to 10mM in 100% DMSO. Permeability of compounds was measured in PBS (pH7.4), 5% DMSO at 23°C. Safire (Tecan) plate reader was used for measurement the UV/Vis absorption. Protocol: Dilute stocks of tested compounds and controls with PBS to 1.67mM and mix well by pipeitting, add 280μl of PBS, 5% DMSO to acceptor plate, add 5μl of 2% L-α-Phosphatidylcholine suspension in dodecane to the membrane of donor plate, Immediately add 98μl of PBS to donor plate and make the sandwich with acceptor plate. Add 42μl of tested compounds and controls dilutions to acceptor plate, cover the plate, place into camera and incubate for 16 hours. Make the equilibrium plate, add 225μl of PBS, 3,7% DMSO and 25μl of tested compounds and controls dilutions to UV plate. After 16 hours pull the donor plate out and transfer 250μl from acceptor plate to UV plate. Scan UV plate on Safire (Tecan) plate reader from 245 to 450nM with step 5nM. Permeability is reported in % of compound found in the receiver compartment after the incubation period. Applying this protocol, PAMPA permeabilities of examples and reference compounds were determined as follows:

| Compound | Permeability |
|---|---|
| Example 4 | 50% |
| Compound A | 32% |
| Compound B | 7% |

***Determination* of *Caco-2 permeability***

Caco-2 cells are widely used as an in vitro model for predicting human drug absorption. The Caco-2 cell line is derived from a human colorectal carcinoma, and when cultured, the cells spontaneously differentiate into monolayers of polarised enterocytes. The cells are seeded on TranswellTM plates and form a confluent monolayer over 20 days prior to the experiment. On day 20, the test compound is added to the apical side of the membrane and the transport of the compound across the monolayer is monitored over a 2 hour time period. To study drug efflux, it is also necessary to investigate transport of the compound from the basolateral compartment to the apical compartment. The permeability coefficient (Papp) is calculated from the following equation:

$$Papp = (dQ/dt)/(C0xA)$$

Where dQ/dt is the rate of permeation of the drug across the cells, C0 is the donor compartment concentration at time zero and A is the area of the cell monolayer.

Applying this protocol, Caco-2 permeability of examples and reference compounds was determined.

***Plasma protein binding assay***

Equilibrium dialysis is used to determine the extent of binding of a compound to plasma proteins. A semi-permeable membrane separates a protein-containing compartment from a protein-free compartment. The system is allowed to equilibrate at 37°C. The test compound present in each compartment is quantified by LC-MS/MS. The extent of binding is reported as a fraction unbound (fu) value which is calculated as fu = 1 - (PC -PF)/PC. PC = Test compound concentration in protein-containing compartment. PF = Test compound concentration in protein-free compartment. In addition to using whole plasma, the plasma protein binding assay can be performed using two other ratios of plasma (10% or 50% plasma in buffer v/v). The following equations are used to convert from a fraction unbound at 10% or 50% to a fraction unbound at 100%:

$$fu_{100\%} = fu_{10\%}/(10-9fu_{10\%})$$

$$fu_{100\%} = fu_{50\%}/(2-fu_{50\%}).$$

Applying this protocol, plasmaprotein binding of examples and reference compounds was determined.

***Microsomal stability assay***

Compound stability towards human or rat liver microsomes was determined as follows: Human or rat liver microsomal suspension (1ml) prepared in reaction buffer at a concentration of 0.5mg microsomal protein/ml was preincubated for 3 min at 37°C with a NADPH-generating system (10mM glucose 6-phosphate, 1mM NADP+, and 1unit/ml yeast glucose-6-phosphate dehydrogenase). The final compounds concentration is 10μM. Boiled microsomes (5min) served as a control. Samples (50μl) were then taken after 0, 5, 15, 30, 45, 120min, into 200μl acetonitrile, centrifuged for 15min at 8000 x g to remove the protein pellet. Samples were analyzed for parent compound by HPLC. Method development: All samples for metabolic stability experiments were analyzed by HPLC. Only parent compounds were analyzed. Data analysis: The percentages of the parent compounds remaining was defined as the ratio of the parent compounds peak area at a specific point and the peak area at the first time point multiplied by 100%. The metabolic stability was evaluated by plotting the natural logarithm of the percentage parent compounds remaining versus time and performing linear regression and finally reported as clearance [μl/min/mg protein] which is reversed proportional to compound stability. Applying this protocol, Microsomal stability of examples and reference compounds was determined.

| Compound | Rat liver microsome clearance [μl/min/mg protein] | Human liver microsome clearance [μl/min/mg protein] |
|---|---|---|
| Example 4 | 90 | 62 |
| Example 7 | 52 | 14 |
| Compound B | 185 | 100 |

***Rat hepatocyte stability assay***

Compound stability towards rat hepatocytes was determined as follows: incubation was performed with rats hepatocytes in suspension. As medium for the hepatocyte incubation, phosphate-buffered saline (PBS) was used with a composition as follows:
$CaCl_2 \times H_2O$ 0.132g/l, KCl 0.2g/l, $KH_2PO_4$ 0.2g/l, $MgCl_2 \times 6H2O$ 0.1 g/l, NaCl 8.0g/l, $NaH_2PO_4$ 1.15g/l, D-Glucose sodium pyruvate 0.0036g/l. Final assay conditions: Incubations were performed in 0.5 ml tubes closed with a cap. A vial was prepared containing 0.5ml of 1,000,000 hepatocytes/ml in incubation buffer. Final concentrations of test substances in the incubation mixtures were 10μM. The Costar tubes were incubated at 37°C in a shaking water bath at 120spm. At

several different time points (T=0, 30, 120, 240min) after addition of the stock solution tubes were taken out of the water bath. For test substances, 250μl acetonitrile was added immediately after sampling and the tubes were mixed and centrifuged at 16000g for 5min. Following centrifugation, samples were placed on ice, and the supernatant was collected in vials with inserts. Samples were analyzed and stored in the freezer at - 20C. Method development: All samples for metabolic stability experiments were analyzed by HPLC (Table 2). Only parent compounds were analyzed. Data analysis: The percentage of the parent compounds remaining was defined as the ratio of the parent compounds peak area at a specific point and the peak area at the first time point multiplied by 100%. The metabolic stability was evaluated by plotting the natural logarithm of the percentage parent compounds remaining versus time and performing linear regression. Applying this protocol, hepatocyte stability of examples and reference compounds was determined.

| Compound | Rat hepatocyte clearance [μl/min/$10^6$cells] |
|---|---|
| Example 7 | 10 |
| Compound B | 16 |

***Determination of Pharmacokinetic parameters in rats***

Information on the rate and extent of absorption as well on the plasma half lives of the different test compounds are generated. In order to achieve the required test dose, respective amounts of the test compounds were added to the respective amounts of a carrier solution, for example an aqueous solution of 0.5 % CMC for the p.o. administration and to respective amounts of a pool sample of plasma of Wistar rats for the i.v. administration. For dosing, about 10 [ml/kg bw.] test substance preparation was dosed orally by gavage to groups of rats. For the i.v. applications, about 2 [ml/kg bw.] test substance preparation was dosed via the tail vein to groups of rats. Samples of each application preparation were taken for the analysis of the homogeneity and correctness of the concentrations of the test substance in the vehicle. Before application, blood samples were taken from each animal to obtain control samples of plasma. In the experiments, animals were treated and blood samples were taken at various time points post the p.o. dose and at various time points post the i.v. dose. After plasma preparation, the test samples were frozen at about - 20 °C prior to analysis. For analysis, the samples of each application group were pooled and the pool samples were mixed with ACN. The mixtures were centrifuged and the supernatants were taken directly for analysis. Analysis was performed by LC/MS. The time point of highest plasma-concentrations, the initial half-life maximum plasma concentrations as well as the area-under-the-data (AUD). The bioavailability is computed by comparison of the dose corrected AUD value of the p.o. dose group in relation to the i.v. dose group. Applying this protocol, pharmacokinetic parameters of examples and reference compounds was determined.

**Claims**

1. A compound of formula (I)

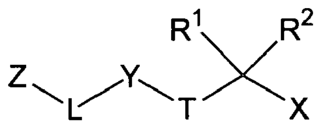

(I)

and an enantiomer, diastereomer, tautomer, solvate or pharmaceutically acceptable salt thereof, wherein

$R^1$ and $R^2$ are independently from each other selected from hydrogen, fluorine, cyano, nitro, azido, $NR^5R^6$, $OR^5$, $SR^5$, $C_1$-$C_6$ alkyl, $C_2$-$C_6$ alkenyl, $C_2$-$C_6$ alkynyl and $C_3$-$C_6$ cycloalkyl; or $R^1$ and $R^2$ are together =O or =S; or $R^1$ and $R^2$ may together form a 3-6-membered carbocyclic or heterocyclic ring which each can be unsaturated or saturated, wherein each alkyl, alkenyl, alkynyl, cycloalkyl group, carbocyclic or heterocyclic ring is unsubstituted or substituted with one to five substituents $R^{11}$;
$R^5$ and $R^6$ are independently from each other selected from hydrogen, $C_1$-$C_6$-alkyl and $C_3$-$C_6$-cycloalkyl; or $R^5$ and $R^6$ together may form a 3-6-membered saturated heterocyclic ring, wherein the alkyl, cycloalkyl and heterocyclic group is unsubstituted or substituted with one to five substituents $R^{11}$;

X is

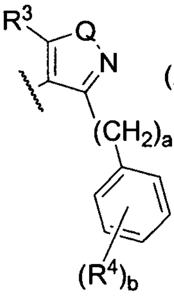 ($X^1$) or 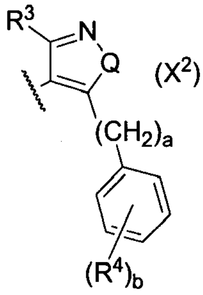 ($X^2$) or 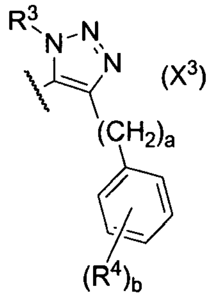 ($X^3$) or 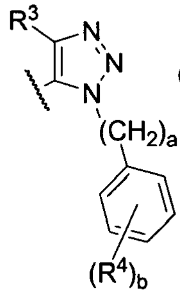 ($X^4$) :

in each formula ($X^1$), ($X^2$), ($X^4$)

$R^3$ is hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, -$NR^{19}R^{20}$, $NR^{19}S(O)_mR^{20}$, $NR^{19}C(O)OR^{20}$, $NR^{19}C(O)R^{20}$, $NR^{19}C(O)NR^{19}R$ , $OR^{19}$, $OC(O)R^{19}$, $S(O)_iR^{19}$, $SO_2NR^{19}C(O)R^{20}$, $S(O)_mNR^{19}R^{20}$, $C(O)R^{19}$, $C(O)OR^{20}$, $C(O)NR^{19}R^{20}$, $C(NR^{19})NR^{19}R^{20}$, wherein each alkyl, alkenyl, alkynyl, cycloalkyl heterocyclyl, aryl or heteroaryl is unsubstituted or substituted with one to five substituents $R^{11}$;

in each formula ($X^3$)

$R^3$ is hydrogen, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $SO_2R^{19}$, $C(O)R^{19}$, $C(O)OR^{19}$, $C(O)NR^{19}R^{20}$,

wherein each alkyl, alkenyl, alkynyl, cycloalkyl, heterocyclyl, aryl or heteroaryl is unsubstituted or substituted with one to five substituents $R^{11}$;

$R^{19}$ and $R^{20}$ are independently from each other selected from hydrogen, $C_1$- $C_6$-alkyl, $C_2$-$C_6$-alkenyl, $C_2$-$C_6$ alkynyl and $C_3$-$C_6$-cycloalkyl, or $R^{19}$ and $R^{20}$ together may form a 3-7-membered heterocyclic or heteroaromatic ring, wherein the $C_1$-$C_6$-alkyl, $C_2$-$C_6$-alkenyl, $C_3$-$C_6$-cycloalkyl, heterocyclyl and heteroaryl group is unsubstituted or substituted with one to five substituents $R^{11}$ ;

$R^4$ is independently selected from hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{15}R^{16}$, $NR^{15}SO_2R^{16}$, $NR^{15}C(O)OR^{16}$ , $NR^{15}C(O)R^{16}$, $NR^{15}C(O)NR^{15}R^{16}$, $NR^{15}C(NCN)NR^{15}R^{16}$, $OR^{15}$, $OC(O)R^{15}$, $S(O)_iR^{15}$, $SO_2NR^{15}C(O)R^{16}$, $S(O)_mNR^{15}R^{16}$, $SC(O)R^{15}$, $C(O)R^{15}$, $C(O)OR^{15}$, $C(O)NR^{15}R^{16}$, $C(O)NHOR^{15}$, $C(O)SR^{15}$ and $C(NR^{15})NR^{15}R^{16}$, wherein each alkyl, alkenyl, alkynyl, cycloalkyl, heterocyclyl, aryl or heteroaryl is unsubstituted or substituted with one to five substituents $R^{11}$;

and further two substituents $R^4$ can be taken together with the atom to which they attach to form a 4-7 membered carbocyclic, aryl, heteroaryl or heterocyclic ring, each of which is substituted or unsubstituted with one to five substituents $R^{11}$;

$R^{15}$ and $R^{16}$ are independently from each other selected from hydrogen, $C_1$- $C_6$-alkyl, $C_2$-$C_6$-alkenyl, $C_2$-$C_6$ alkynyl and $C_3$-$C_6$-cycloalkyl; or $R^{15}$ and $R^{16}$ together may form a 3-7-membered heterocyclic or heteroaromatic ring, wherein the alkyl, alkenyl, cycloalkyl, heterocyclyl and heteroaryl groups are unsubstituted or substituted with one to five substituents $R^{11}$;

$R^{11}$ is independently selected from hydrogen, halogen, cyano, nitro, azido, =O, =S, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{12}R^{13}$, $NR^{12}S(O)_mR^{13}$, $NR^{12}C(O)OR^{13}$, $NR^{12}C(O)R^{13}$, $NR^{12}C(O)NR^{12}R^{13}$, $NR^{12}C(NCN)NR^{12}R^{13}$, $=NOR^{12}$, $-OR^{12}$, $OC(O)R^{12}$, $S(O)_iR^{12}$, $SO_2NR^{12}C(O)R^{13}$, $S(O)_mNR^{12}R^{13}$, $SC(O)R^{12}$, $C(O)R^{12}$, $C(O)OR^{12}$, $C(O)SR^{12}$, $C(O)NR^{12}R^{13}$, $C(O)NOR^{12}$, and $C(NR^{12})NR^{12}R^{13}$;

$R^{12}$ and $R^{13}$ are independently from each other selected from hydrogen, $C_1$-$C_6$ alkyl and $C_3$-$C_6$ cycloalkyl, wherein each alkyl or cycloalkyl may be unsubstituted or substituted with one to five fluorines and/or one or two substituents selected from OH, $OCH_3$, $OCH_2F$, $OCHF_2$, $OCF_3$, =O, $SCF_3$, $NH_2$, $NHCH_3$ and $N(CH_3)_2$; or $R^{12}$ and $R^{13}$ can be taken together with the atom to which they are attached to form a 4 to 6 membered carbocyclic, heteroaryl or heterocyclic ring, each of which may be unsubstituted or substituted with one to five fluorines and/or one or two substituents selected from OH, $OCH_3$, -$OCH_2F$, $OCHF_2$, $OCF_3$, =O, $SCF_3$, $NH_2$, $NHCH_3$ and

$N(CH_3)_2$;
Q is $NR^7$;
$R^7$ is hydrogen, $C_1$-$C_3$-alkyl or $C_3$-$C_5$ cycloalkyl, wherein each alkyl or cycloalkyl is unsubstituted or substituted with 1-5 fluorine atoms;
T is -O-, -S-, -N($R^{14}$)-, $CH_2$ or $CF_2$;
$R^{14}$ is hydrogen, $C_1$-$C_3$-alkyl or $C_3$-$C_5$ cycloalkyl, wherein each alkyl or cycloalkyl is unsubstituted or substituted with 1-5 fluorine atoms;
Y is selected from

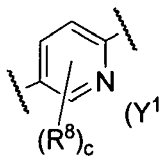
($Y^1$)
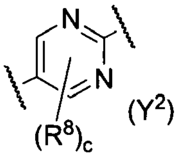
($Y^2$)
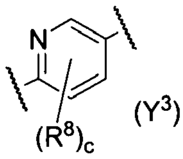
($Y^3$)

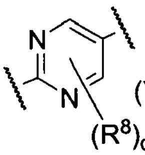
($Y^4$)
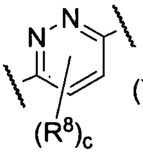
($Y^5$)
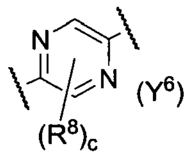
($Y^6$)

$R^8$ is independently selected from hydrogen, halogen, cyano, nitro, azido, $C_1$-$C_6$ alkyl, $C_2$-$C_6$ alkenyl, $C_2$-$C_6$ alkynyl, $C_3$-$C_6$ cycloalkyl, heterocyclyl, aryl, heteroaryl, $NR^{12}R^{13}$, $NR^{12}S(O)_mR^{13}$, $NR^{12}C(O)OR^{13}$, $NR^{12}C(O)R^{13}$, $NR^{12}C(O)NR^{12}R^{13}$, $OR^{12}$, $OC(O)R^{12}$, $S(O)_iR^{12}$, $SO_2NR^{12}C(O)R^{13}$, $S(O)_mNR^{12}R^{13}$, $C(O)R^{12}$, $C(O)OR^{12}$, $C(O)NR^{12}R^{13}$, and $C(NR^{12})NR^{12}R^{13}$, wherein each alkyl, alkenyl, alkynyl, cycloalkyl, heterocyclyl, aryl or heteroaryl is unsubstituted or substituted with one to five substituents $R^{11}$;
L is a bond, -C(O)N($R^{10}$)-, -$S(O)_m$N($R^{10}$)-, -G-N($R^{10}$)-, - N($R^{10}$)C(O)-, -N($R^{10}$)$S(O)_m$-, -N($R^{10}$)-G-, -G-S-, -G-O-, -S-G-, or O-G, or L is

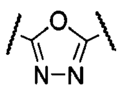
,
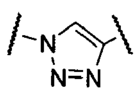
or
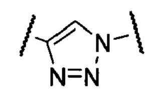

$R^{10}$ is hydrogen, $C_1$-$C_3$-alkyl, or $C_3$-$C_5$ cycloalkyl, wherein each alkyl or cycloalkyl is unsubstituted or substituted with 1-5 fluorine atoms;
G is methylene or ethylene which is unsubstituted or substituted with 1-5 fluorine atoms;
Z is phenyl-A-$R^9$, pyridyl-A-$R^9$, pyrimidyl-A-$R^9$ or pyridazyl-A-$R^9$, wherein phenyl, pyridyl, pyrimidyl or pyridazyl is unsubstituted or substituted with one to three groups selected from halogen, $C_1$-$C_4$ alkyl, $C_3$-$C_5$ cycloalkyl, $C_2$-$C_4$ alkenyl, $C_2$-$C_4$ alkynyl, cyano, OH, $OCH_3$, $OCH_2F$, $OCHF_2$, $OCF_3$, $SCF_3$, $NH_2$, $NHCH_3$ and $N(CH_3)_2$;
A is a bond, $CH_2$, $CHCH_3$, $C(CH_3)_2$ or $CF_2$;
$R^9$ is hydrogen, $COOR^{17}$, $CONR^{17}R^{18}$, $C(O)NHSO_2R^{17}$, $SO_2NHC(O)R^{17}$, $S(O)_mR^{17}$, $C(NR^{17})NR^{17}R^{18}$, or tetrazole which is connected to A via the C-atom;
$R^{17}$ and $R^{18}$ are independently from each other selected from hydrogen, $C_1$-$C_6$-alkyl, $C_2$-$C_6$-alkenyl, $C_2$-$C_6$ alkynyl and $C_3$-$C_6$-cycloalkyl; or $R^{17}$ and $R^{18}$ together may form a 3-7-membered heterocyclic or heteroaromatic ring, wherein the $C_1$-$C_6$-alkyl, $C_2$-$C_6$-alkenyl, $C_3$-$C_6$-cycloalkyl, heterocyclyl and heteroaryl groups are unsubstituted or substituted with one to five substituents $R^{11}$;
a is 0 or 1;
b is 1, 2 or 3;
c is 1 or 2;

i is 0, 1 or 2; and
m is 1 or 2.

**2.** The compound according to claim 1, wherein

$R^1$ and $R^2$ are independently selected from hydrogen, fluorine and $C_{1-6}$ alkyl, wherein the alkyl group is unsubstituted or substituted with one to five substituents $R^{11}$; or $R^1$ and $R^2$ are together =O or =S.

**3.** The compound according to claim 1 or 2, wherein

Q is O or NH.

**4.** The compound according to any of claims 1 to 3, wherein
in each formula ($X^1$), ($X^2$) and ($X^4$)

$R^3$ is hydrogen, $C_1$-$C_6$ alkyl, $NR^{19}R^{20}$ or $C_3$-$C_6$ cycloalkyl, wherein each alkyl or cycloalkyl is unsubstituted or substituted with one to five substituents $R^{11}$; and

in each formula ($X^3$)

$R^3$ is hydrogen, $C_{1-6}$ alkyl or $C_3$-$C_6$ cycloalkyl, wherein each alkyl or cycloalkyl is unsubstituted or substituted with one to five substituents $R^{11}$.

**5.** The compound according to any of claims 1 to 4, wherein

$R^4$ is hydrogen, halogen, $C_{1-6}$ alkyl, O-$C_1$-$C_6$ alkyl or CN, wherein each alkyl group is unsubstituted or substituted by one to five substituents $R^{11}$.

**6.** The compound according to any of claims 1 to 5, wherein

T is O, $CH_2$ or $NR^{14}$.

**7.** The compound according to any of claims 1 to 6, wherein

Y is selected from formula ($Y^1$), ($Y^2$ ) and ($Y^3$).

**8.** The compound according to any of claims 1 to 7, wherein

$R^8$ is hydrogen, halogen, $C_1$-$C_6$-alkyl or O-$C_1$-$C_3$-alkyl, wherein each alkyl group is unsubstituted or substituted with one to five substituents $R^{11}$.

**9.** The compound according to any of claims 1 to 8, wherein

L is a bond, -C(O)N($R^{10}$)-, -S(O)$_i$N($R^{10}$)-, -G-N($R^{10}$)- or -N($R^{10}$)-G;
$R^{10}$ is hydrogen or $C_1$-$C_6$-alkyl; and i is 2.

**10.** The compound according to any of claims 1 to 9, wherein

Z is phenyl-A-$R^9$, wherein phenyl is unsubstituted or substituted with one to three groups selected from halogen, cyano, $C_{1-4}$ alkyl, OH, $OCH_3$, $OCH_2F$, $OCHF_2$, $OCF_3$, $SCF_3$, $NH_2$, $NHCH_3$ and $N(CH_3)_2$.

**11.** The compound according to any of claims 1 to 10 as a medicament.

**12.** A pharmaceutical composition comprising at least one compound according to any of claims 1 to 10 and at least one pharmaceutically acceptable excipient and/or carrier.

**13.** Use of a compound according to any of claims 1 to 10 for the preparation of a medicament for the prophylaxis and/or treatment of diseases mediated by FXR.

**14.** The use according to claim 13 for chronic intrahepatic or some forms of extrahepatic cholestatic conditions, or liver fibrosis resulting from chronic cholestatic conditions or acute intraheptic cholestatic conditions.

**15.** The use according to claim 14, wherein the chronic intraheptic cholestatic conditions are primary biliary cirrhosis (PBC), primary sclerosing cholangitis (PSC), progressive familiar cholestasis (PFIC), alcohol-induced cirrhosis and associated cholestasis, and the liver fibrosis is estrogen or drug induced cholestasis.

**16.** The use according to claim 13 for obstructive or chronic inflammatory disorders that arise out of improper bile composition.

**17.** The use according to claim 16, wherein the obstructive or chronic inflammatory disorders are cholelithiasis (cholesterol gallstones).

**18.** The use according to claim 13 for gastrointestinal conditions with a reduced uptake of dietary fat and fat-soluble dietary vitamins.

**19.** The use according to claim 13 for Inflammatory Bowel Diseases.

**20.** The use according to claim 19, wherein the inflammatory bowel diseases are Crohn's disease or Colitis Ulcerosa.

**21.** The use according to claim 13 for lipid and lipoprotein disorders.

**22.** The use according to claim 21, wherein the lipid and lipoprotein disorders are hypercholesterolemia, hypertriglyceridemia, and atherosclerosis as a clinically manifest condition.

**23.** The use according to claim 13 for Type II Diabetes.

**24.** The use according to claim 13 for obesity and metabolic syndrome (combined conditions of dyslipidemia, diabetes and abnormally high body-mass index).

**25.** The use according to claim 13 for acute myocardial infarction, acute stroke, or thrombosis which occur as an endpoint of chronic obstructive atherosclerosis.

**26.** The use according to claim 13 for persistant infections by intracellular bacteria or parasitic protozoae.

**27.** The use according to claim 26, wherein the bacterial or parasitic protozoae are selected from Mycobacterium spec. (Treatment of Tuberculosis or Lepra), Listeria monocytogenes (Treatment of Listeriosis), Leishmania spec. (Leishmaniosis), Trypanosoma spec. (Chagas Disease; Trypanosomiasis; Sleeping Sickness).

**28.** The use according to claim 13 for non-malignant hyperproliferative disorders.

**29.** The use according to claim 28, wherein the non-malignant hyperproliferative disorders are increased neointima formation after balloon vessel dilatation and stent application due to increased proliferation of vascular smooth muscle cells (VSMCs) Bening Prostate Hyperplasia (BPH), or other forms of scar tissue formation and fibrotisation.

**30.** The use according to claim 13 for malignant hyperproliferative disorders.

**31.** The use according to claim 30, wherein the malignant hyperproliferative disorders are cancer.

**32.** The use according to claim 13 for liver steatosis and associated syndromes, cholestatic and fibrotic effects that are associated with alcohol-induced cirrhosis or with viral-borne forms of hepatitis.

**33.** The use according to claim 32, wherein the liver steatosis associated syndrome is non-alcoholic steatohepatitis ("NASH").

**34.** A method for preparing a compound of formula (I) according to any of claims 1 to 10 comprising the step of reacting a compound of formula (XIII)

$Z\text{-}L_C$ (XIII)

wherein
Z is as defined in claim 1 and
$L_C$ is halogen, $NH_2$, $N(R^{10})H$, COCl, COF, CHO, $CH_2OH$, COOH, $C(O)NHNH_2$, C(O)O-alkyl, C(O)O-aryl, C(O)O-hetaryl, SH, $SO_2Cl$, $SO_3H$, G-$NH_2$, G-$N(R^{10})H$, OH, G-SH, G-OH, G-halogen, $B(OMe)_2$, $B(OH)_2$, $BF_3^-$, 4,4,5,5-tetramethyl-1,3,2-dioxaborolan-2-yl or ethinyl;
with a compound of formula (XIV)

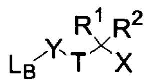 (XIV)

wherein
Y, T, X, $R^1$ and $R^2$ are as defined in claim 1 and
$L_B$ is halogen, $NH_2$, $N(R^{10})H$, COCl, COF, CHO, $CH_2OH$, COOH, $C(O)NHNH_2$, C(O)O-alkyl, C(O)O-aryl, C(O)O-hetaryl, SH, $SO_2Cl$, $SO_3H$ G-$NH_2$, G-$N(R^{10})H$, OH, G-SH, G-OH, G-halogen, $B(OMe)_2$, $B(OH)_2$, $BF_3^-$, 4,4,5,5-tetramethyl-1,3,2-dioxaborolan-2-yl or ethinyl.

**35.** The method according to claim 34, wherein
Z, L and Y are as defined in claim 1,
TisO,
$R^1$ and $R^2$ are hydrogen and
X is $X^1$ with Q being O and a being 0.

**36.** The method according to claim 34 or 35, comprising the further steps of reacting a compound of formula (IXa) or formula (IXb)

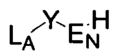 (IXa), 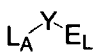 (IXb)

wherein
$L_A$ is halogen, nitro, azido, CN, $CF_3$, C(O)O-alkyl, C(O)O-aryl, C(O)O-hetaryl, SH, SMe, $SO_3H$, G-$NH_2$, G-$N(R^{10})H$, OH, O-alkyl, G-SH, G-OH, G-halogen, $B(OH)_2$, $B(Oalkyl)_2$ or ethinyl;
$E_N$-H is OH, SH, $NH_2$, $N(R^{14})H$, NH(CO)O-alkyl, NH(CO)O-aryl, $NH(SO)_2aryl$, $NH(SO)_2alkyl$, $CH_3$ or $CF_2H$;
$E_L$ is halogen, OH, OC(O)alkyl, OC(O)aryl, O-aryl, O-pentafluorophenyl, O-sulfonylalkyl, O-sulfonylaryl, O-succinylimido, O-benzotriazolyl, nitro, azido, S-alkyl, $SO_2alkyl$, $SO_2aryl$, SC(O)alkyl, SC(O)aryl or cyano;
with a compound of formula (IVa) or (IVb), respectively

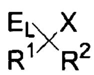 (IVa), 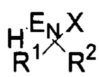 (IVb)

wherein
$R^1$, $R^2$ and X are as defined in claim 1 and
$E_L$ and $E_N$-H are as defined above;
in order to obtain a compound of formula (XII)

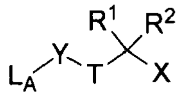 (XII)

wherein
Y, T, $R^1$, $R^2$ and X are as defined in claim 1;
which is further reacted to a compound of formula (XIV) by replacing the $L_A$ moiety with a $L_B$ moiety.

**37.** The method according to claim 36, wherein
Y, $R^4$, b and $R^3$ are as defined in claim 1,
TisO,
$R^1$ and $R^2$ are hydrogen and
X is $X^1$ with Q being O and a being 0.

European Patent Office

# EUROPEAN SEARCH REPORT

Application Number
EP 06 01 8023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 03/015771 A (LION BIOSCIENCE AG [DE]; BAUER ULRIKE [DE]; CHERUVALLATH ZACH [US]; DE) 27 February 2003 (2003-02-27) Claims 4-13. ----- | 1,12,13, 34 | INV. C07D401/06 C07D401/12 C07D413/12 C07D413/14 A61K31/4427 A61K31/501 A61K31/506 A61P35/00 |
| D,A | WO 2004/048349 A (SMITHKLINE BEECHAM CORP [US]; BOGGS SHARON D [US]; COLLINS JON L [US];) 10 June 2004 (2004-06-10) Examples 1-103, pages 47-103. Claims 15 and 26. ----- | 1,12,13, 34 | |
| A | US 5 883 105 A (ANTHONY NEVILLE J [US]) 16 March 1999 (1999-03-16) Claim 9, 14-17. ----- | 1,12,13, 34 | |

TECHNICAL FIELDS SEARCHED (IPC)

C07D
A61K
A61P

The present search report has been drawn up for all claims

3

| Place of search | Date of completion of the search | Examiner |
|---|---|---|
| The Hague | 9 February 2007 | Menchaca, Roberto |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 01 8023

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2007

| Patent document cited in search report | Publication date | Patent family member(s) | Publication date |
|---|---|---|---|
| WO 03015771 A | 27-02-2003 | NONE | |
| WO 2004048349 A | 10-06-2004 | AU 2003290700 A1 | 18-06-2004 |
| | | EP 1562915 A1 | 17-08-2005 |
| | | JP 2006515838 T | 08-06-2006 |
| US 5883105 A | 16-03-1999 | US 6051574 A | 18-04-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004048349 A
- WO 2003015771 A
- WO 2000037077 A
- WO 2004087076 A
- WO 2003080803 A

### Non-patent literature cited in the description


- **D. MANGELSDORF et al.** The nuclear receptor superfamily: the second decade. *Cell,* 1995, vol. 83 (6), 835-839
- **R EVANS.** The nuclear receptor superfamily: a rosetta stone for physiology. *Mol. Endocrinol.,* 2005, vol. 19 (6), 1429-1438
- **M. SCHENA.** Mammalian glucocorticoid receptor derivatives enhance transcription in yeast. *Science,* 1988, vol. 241 (4868), 965-967
- **A. BRZOZOWSKI et al.** Molecular basis of agonism and antagonism in the oestrogen receptor. *Nature,* 1997, vol. 389 (6652), 753-758
- **D. HEERY et al.** A signature motif in transcriptional co-activators mediates binding to nuclear receptors. *Nature,* 1997, vol. 387 (6634), 733-6
- **T. HEINZEL et al.** A complex containing N-CoR, mSin3 and histone deacetylase mediates transcriptional repression. *Nature,* 1997, vol. 387 (6628), 16-17
- **K. NETTLES ; G. GREENE.** Ligand control of coregulator recruitment to nuclear receptors. *Annu. Rev. Physiol.,* 2005, vol. 67, 309-33
- **A. ARANDA ; A. PASCUAL.** Nuclear hormone receptors and gene expression. *Physiol. Rev.,* 2001, vol. 81 (3), 1269-1304
- **B. FORMAN et al.** Identification of a nuclear receptor that is activated by farnesol metabolites. *Cell,* 1995, vol. 81 (5), 687-693
- **D. PARKS et al.** Bile acids: natural ligands for an orphan nuclear receptor. *Science,* 1999, vol. 284 (5418), 1365-1368
- **M. MAKISHIMA et al.** Identification of a nuclear receptor for bile acids. *Science,* 1999, vol. 284 (5418), 1362-1365
- **J. HOLT et al.** Definition of a novel growth factor-dependent signal cascade for the suppression of bile acid biosynthesis. *Genes Dev,* 2003, vol. 17 (13), 1581-91
- **T. INAGAKI et al.** Fibroblast growth factor 15 functions as an enterohepatic signal to regulate bile acid homeostasis. *Cell Metab,* 2005, vol. 2 (4), 217-225
- **P. ANANDET.** Downregulation of TACO gene transcription restricts mycobacterial entry/survival within human macrophages. *FEMS Microbiol. Lett,* 2005, vol. 250 (1), 137-144
- **R. C. BUIJSMAN et al.** Non-Steroidal Steroid Receptor Modulators. *Curr. Med. Chem,* 2005, vol. 12, 1017-1075
- **R. PRENTIS et al.** Pharmaceutical innovation by seven UK-owned pharmaceutical companies (1964-1985. *Br. J. Clin. Pharmacol,* 1988, vol. 25, 387-396
- **J. LIN ; A. LU.** Role of pharmacokinetics and Metabolism in Drug Discovery and Development. *Pharmacol. Rev,* 1997, vol. 49 (4), 404-449
- **C. A. LIPINSKI.** Drug-like properties and the causes of poor solubility and poor permeability. *J. Pharmacol. Toxicol. Methods,* 2000, vol. 44, 235-249
- **L. DI ; E. KERNS.** Profiling drug-like properties in discovery research. *Curr. Opin. Chem. Biol.,* 2003, vol. 7, 402-408
- **A.M. MARINO et al.** Validation of the 96-well Caco-2 cell culture model for high-throughput permeability and assessment of discovery compounds. *Int. J. Pharmaceutics,* 2005, vol. 297, 235-241
- **F. WOHNSLAND ; B. FALLER.** High-throughput Permeability pH Profile and High-throughput Alkane/Water Log P With Artificial Membranes. *J. Med. Chem.,* 2001, vol. 44, 923-930
- **Y. KWON.** Handbook of essential pharmacokinetics, pharmacodynamics and drug metabolism for industrial scientists. Springer Verlag, 2001
- **T. IWATSUBO et al.** Prediction of in vivo drug metabolism in the human liver from in vitro metabolism data. *Pharm. Ther.,* 1997, vol. 73, 147-171
- **T. CLAUDEL et al.** The Farnesoid X receptor: a molecular link between bile acid and lipid and glucose metabolism. *Arterioscler. Thromb. Vasc. Biol,* 2005, vol. 25 (10), 2020-2030
- **S. WESTIN et al.** FXR, a therapeutic target for bile acid and lipid disorders. *Mini Rev. Med. Chem,* 2005, vol. 5 (8), 719-727

- **B. GOODWIN et al.** A regulatory cascade of the nuclear receptors FXR, SHP-1, and LRH-1 represses bile acid biosynthesis. *Mol. Cell,* 2000, vol. 6 (3), 517-526
- **T. LU et al.** Molecular basis for feedback regulation of bile acid synthesis by nuclear receptors. *Mol. Cell,* 2000, vol. 6 (3), 507-515
- **C. SINAL et al.** Targeted disruption of the nuclear receptor FXR/BAR impairs bile acid and lipid homeostasis. *Cell,* 2000, vol. 102 (6), 731-744
- **M. ANANTHANARAYANANET.** Human bile salt export pump promoter is transactivated by the farnesoid X receptor/bile acid receptor. *J. Biol. Chem.,* 2001, vol. 276 (31), 28857-28865
- **J. PLASS et al.** Farnesoid X receptor and bile salts are involved in transcriptional regulation of the gene encoding the human bile salt export pump. *Hepatology,* 2002, vol. 35 (3), 589-96
- **N. URIZAR et al.** The farnesoid X-activated receptor mediates bile acid activation of phospholipid transfer protein gene expression. *J. Biol. Chem.,* 2000, vol. 275 (50), 39313-39317
- **H. KAST et al.** Regulation of multidrug resistance-associated protein 2 (ABCC2) by the nuclear receptors pregnane X receptor, farnesoid X-activated receptor, and constitutive androstane receptor. *J. Biol. Chem.,* 2002, vol. 277 (4), 2908-2915
- **L. HUANG et al.** Farnesoid X receptor activates transcription of the phospholipid pump MDR3. *J. Biol. Chem.,* 2003, vol. 278 (51), 51085-51090
- **M. MIYATA.** Role of farnesoid X receptor in the enhancement of canalicular bile acid output and excretion of unconjugated bile acids: a mechanism for protection against cholic acid-induced liver toxicity. *J. Pharmacol. Exp. Ther.,* 2005, vol. 312 (2), 759-766
- **G. RIZZO et al.** Role of FXR in regulating bile acid homeostasis and relevance for human diseases. *Curr. Drug Targets immune Endocr. Metabol. Disord.,* 2005, vol. 5 (3), 289-303
- **P. MALONEY et al.** Identification of a chemical tool for the orphan nuclear receptor FXR. *J. Med. Chem.,* 2000, vol. 43 (16), 2971-2974
- **T. WILLSON et al.** Chemical genomics: functional analysis of orphan nuclear receptors in the regulation of bile acid metabolism. *Med. Res. Rev.,* 2001, vol. 21 (6), 513-22
- **R. PELLICCIARI et al.** 6alpha-ethyl-chenodeoxycholic acid (6-ECDCA), a potent and selective FXR agonist endowed with anticholestatic activity. *J. Med. Chem.,* 2002, vol. 45 (17), 3569-3572
- **Y. LIU et al.** Hepatoprotection by the farnesoid X receptor agonist GW4064 in rat models of intra- and extrahepatic cholestasis. *J. Clin. Invest,* 2003, vol. 112 (11), 1678-1687
- **S. FIORUCCI et al.** The nuclear receptor SHP mediates inhibition of hepatic stellate cells by FXR and protects against liver fibrosis. *Gastroenterology,* 2004, vol. 127 (5), 1497-1512
- **S. FIORUCCI et al.** A farnesoid x receptor-small heterodimer partner regulatory cascade modulates tissue metalloproteinase inhibitor-1 and matrix metalloprotease expression in hepatic stellate cells and promotes resolution of liver fibrosis. *J. Pharmacol. Exp. Ther.,* 2005, vol. 314 (2), 584-595
- **S. FIORUCCI et al.** Cross-talk between farnesoid-X-receptor (FXR) and peroxisome proliferator-activated receptor gamma contributes to the antifibrotic activity of FXR ligands in rodent models of liver cirrhosis. *J. Pharmacol. Exp. Ther.,* 2005, vol. 315 (1), 58-68
- **A. GALLI et al.** Antidiabetic thiazolidinediones inhibit collagen synthesis and hepatic stellate cell activation in vivo and in vitro. *Gastroenterology,* 2002, vol. 122 (7), 1924-1940
- **I. PINEDA TORRA et al.** Bile acids induce the expression of the human peroxisome proliferator-activated receptor alpha gene via activation of the farnesoid X receptor. *Mol. Endocrinol,* 2003, vol. 17 (2), 259-272
- **S. FIORUCCI et al.** Protective effects of 6-ethyl chenodeoxycholic acid, a farnesoid X receptor ligand, in estrogen-induced cholestasis. *J. Pharmacol. Exp. Ther,* 2005, vol. 313 (2), 604-612
- **F. CHEN et al.** Progressive familial intrahepatic cholestasis, type 1, is associated with decreased farnesoid X receptor activity. *Gastroenterology,* 2004, vol. 126 (3), 756-64
- **L. ALVAREZ et al.** Reduced hepatic expression of farnesoid X receptor in hereditary cholestasis associated to mutation in ATP8B1. *Hum. Mol. Genet.,* 2004, vol. 13 (20), 2451-60
- **G. RIZZO et al.** *Curr. Drug Targets Immune Endocr. Metabol. Disord.,* 2005, vol. 5 (3), 289-303
- **G. ZOLLNER.** Role of nuclear receptors in the adaptive response to bile acids and cholestasis: pathogenetic and therapeutic considerations. *Mol. Pharm.,* 2006, vol. 3 (3), 231-51
- **S. CAI et al.** FXR: a target for cholestatic syndromes. *Expert Opin. Ther. Targets,* 2006, vol. 10 (3), 409-421
- **H. WITTENBURG.** FXR and ABCG5/ABCG8 as determinants of cholesterol gallstone formation from quantitative trait locus mapping in mice. *Gastroenterology,* 2003, vol. 125 (3), 868-881
- **A. MOSCHETTA et al.** Prevention of cholesterol gallstone disease by FXR agonists in a mouse model. *Nature Medicine,* 2004, vol. 10 (12), 1352-1358
- **S. DOGGRELL.** New targets in and potential treatments for cholesterol gallstone disease. *Curr. Opin. Investig. Drugs,* 2006, vol. 7 (4), 344-348

- **P. MALONEY et al.** *J. Med. Chem.,* 2000, vol. 43 (16), 2971-2974
- **T. WILLSON et al.** *Med. Res. Rev.,* 2001, vol. 21 (6), 513-22
- **H. KAST et al.** Farnesoid X-activated receptor induces apolipoprotein C-II transcription: a molecular mechanism linking plasma triglyceride levels to bile acids. *Mol. Endocrinol,* 2001, vol. 15 (10), 1720-1728
- **N.URIZAR et al.** A natural product that lowers cholesterol as an antagonist ligand for FXR. *Science,* 2002, vol. 296 (5573), 1703-1706
- **G. LAMBERT et al.** The farnesoid X-receptor is an essential regulator of cholesterol homeostasis. *J. Biol. Chem.,* 2003, vol. 278, 2563-2570
- **M. WATANABE et al.** Bile acids lower triglyceride levels via a pathway involving FXR, SHP, and SREBP-1c. *J. Clin. Invest,* 2004, vol. 113 (10), 1408-1418
- **A. FIGGE et al.** Hepatic overexpression of murine Abcb11 increases hepatobiliary lipid secretion and reduces hepatic steatosis. *J. Biol. Chem.,* 2004, vol. 279 (4), 2790-2799
- **S. BILZ et al.** Activation of the farnesoid X receptor improves lipid metabolism in combined hyperlipidemic hamsters. *Am. J. Physiol. Endocrinol. Metab.,* 2006, vol. 290 (4), E716-22
- **K. STAYROOK et al.** Regulation of carbohydrate metabolism by the farnesoid X receptor. *Endocrinology,* 2005, vol. 146 (3), 984-91
- **Y. ZHANG et al.** Activation of the nuclear receptor FXR improves hyperglycemia and hyperlipidemia in diabetic mice. *Proc. Natl. Acad. Sci. USA,* 2006, vol. 103 (4), 1006-1011
- **B. CARIOU et al.** The farnesoid X receptor modulates adiposity and peripheral insulin sensitivity in mice. *J. Biol. Chem.,* 2006, vol. 281, 11039-11049
- **K. MA et al.** Farnesoid X receptor is essential for normal glucose homeostasis. *J. Clin. Invest,* 2006, vol. 116 (4), 1102-1109
- **D. DURAN-SANDOVAL et al.** Potential regulatory role of the farnesoid X receptor in the metabolic syndrome. *Biochimie,* 2005, vol. 87 (1), 93-98
- **C. LIHONG et al.** FXR Agonist, GW4064, Reverses Metabolic Defects in High-Fat Diet Fed Mice. *American Diabetes Association (ADA) 66th annual scientific sessions,* June 2006, 856
- **J. HOLT et al.** *Genes Dev.,* 2003, vol. 17 (13), 1581-1591
- **E. TOMLINSON et al.** Transgenic mice expressing human fibroblast growth factor-19 display increased metabolic rate and decreased adiposity. *Endocrinology,* 2002, vol. 143 (5), 1741-1747
- **E. HANNIMAN et al.** Loss of functional farnesoid X receptor increases atherosclerotic lesions in apolipoprotein E-deficient mice. *J. Lipid Res.,* 2005, vol. 46 (12), 2595-2604
- **F. HE et al.** Downregulation of endothelin-1 by farnesoid X receptor in vascular endothelial cells. *Circ. Res.,* 2006, vol. 98 (2), 192-9
- **E. NIESOR et al.** The nuclear receptors FXR and LXRalpha: potential targets for the development of drugs affecting lipid metabolism and neoplastic diseases. *Curr. Pharm. Des.,* 2001, vol. 7 (4), 231-59
- **D. BISHOP-BAILEY et al.** Expression and activation of the farnesoid X receptor in the vasculature. *Proc. Natl. Acad. Sci. USA.,* 2004, vol. 101 (10), 3668-3673
- **J. SILVA.** Lipids isolated from bone induce the migration of human breast cancer cells. *J. Lipid Res.,* 2006, vol. 47 (4), 724-733
- **G. DE GOTTARDI et al.** The bile acid nuclear receptor FXR and the bile acid binding protein IBABP are differently expressed in colon cancer. *Dig. Dis. Sci.,* 2004, vol. 49 (6), 982-989
- **D. DURAN-SANDOVAL et al.** *J. Biol. Chem.,* 2005, vol. 280 (33), 29971-29979
- **Y. ZHANG et al.** *Proc. Natl. Acad. Sci. USA.,* 2006, vol. 103 (4), 1006-1011
- **T. INAGAKI et al.** Regulation of antibacterial defense in the small intestine by the nuclear bile acid receptor. *Proc. Natl. Acad. Sci. USA.,* 2006, vol. 103 (10), 3920-3905
- March's Advanced Organic Chemistry: Reactions, Mechanisms, and Structure. John Wiley & Sons
- **THEOPHIL EICHER ; SIEGFRIED HAUPTMANN.** The Chemistry of Heterocycles; Structures, Reactions, Synthesis and Application. Wiley-VCH, 2003
- **FIESER et al.** Fiesers' Reagents for organic Synthesis. John Wiley & Sons, 2000
- **KAMITORI et al.** *Heterocycles,* 1994, vol. 38, 21-25
- **KAMITORI et al.** *J. Heterocycl. Chem.,* 1993, vol. 30, 389-391
- **KAIM et al.** *Synlett,* 2000, 353-355
- **QUILIO et al.** *Gazz. Chim. Ital.,* 1937, vol. 67, 589
- **MALONEY et al.** *J. Med. Chem.,* 2000, vol. 43 (16), 2971-2974
- **DOLEY et al.** *J. Chem. Soc.,* 1963, 5838-5845
- **HIMBERT et al.** *Liebigs Ann. Chem,* 1990, vol. 4, 403-407
- **BEYER et al.** *Justus Liebigs Ann Chem,* 1970, 45-54
- **Y. CHEN et al.** *Heterocycles,* 1995, vol. 41, 175
- **B. CHANTEGRAL et al.** *J. Org. Chem.,* 1984, vol. 49, 4419-4424
- **J. PIET et al.** *Bull. Soc. Chim. Belg,* 1996, vol. 105 (1), 33-44

- **A. CWIKLICKI et al.** *Arch. Pharm.,* 2004, vol. 337 (3), 156-163
- **G. MITCHELL et al.** *J. Chem. Soc. Perkin Trans,* 1987, vol. 1, 413-422
- **Y. PITERSKAYA et al.** *Russ. J. Gen. Chem.,* 1996, vol. 66 (7), 1150-1157
- **T. EICHER ; S. HAUPTMANN.** The Chemistry of Heterocycles; Structures, Reactions, Synthesis and Application. Wiley-VCH, 2003
- **LAROCK.** Comprehensive Organic Transformations. VCH Publishers, 1989
- **T. GREENE ; P. WUTS.** Protective groups in organic synthesis. Wiley & Sons, 1999
- **K. B. SHARPLESS et al.** *Angew. Chem.,* 2002, vol. 114, 2708-2711
- **A. SUZUKI.** palladium-Catalyzed Cross-Coupling Reactions of Organoboron Compounds. *Chem. Rev.,* 1995, vol. 95, 2457-2483